# EUROPEAN PATENT SPECIFICATION

(11) **EP 2 496 711 B1**
(45) Date of publication and mention of the grant of the patent: **17.02.2016**
(21) Application number: 10776196.7
(22) Date of filing: 02.11.2010
(51) Int. Cl.: C12Q 1/68

(54) **METHODS FOR MONITORING CELLULAR STATES AND FOR IMMORTALIZING MESENCHYMAL STEM CELLS**
VERFAHREN ZUR ÜBERWACHUNG VON ZELLULÄREN ZUSTÄNDEN UND ZUR IMMORTALISIERUNG VON MESENCHYMALEN STAMMZELLEN
PROCEDES DE SURVEILLANCE DES ETATS CELLULAIRES ET DE IMMORTALISER DES CELLULES SOUCHES MÉSENCHYMATEUSES

(30) Priority: 02.11.2009 US 257121 P
(43) Date of publication of application: 12.09.2012
(73) Proprietor: Agency for Science, Technology and Research, Singapore 138632 (SG)
(72) Inventor: LIM, Sai Kiang, Singapore 138648 (SG)
(74) Representative: Cornish, Kristina Victoria Joy
(86) International application number: PCT/SG2010/000422
(87) International publication number: WO 2011/053257

(56) References cited:
- EP-A1- 1 447 443
- WO-A1-2009/070653
- WO-A2-01/78753
- WO-A2-2007/081720
- US-A1- 2003 104 615
- JIANG JINMAI ET AL: "Real-time expression profiling of microRNA precursors in human cancer cell lines", NUCLEIC ACIDS RESEARCH, vol. 33, no. 17, 1 January 2005 (2005-01-01), pages 5394-5403, XP002577637, OXFORD UNIVERSITY PRESS, SURREY, GB ISSN: 0305-1048, DOI: 10.1093/NAR/GKI863 [retrieved on 2005-09-28]
- SCHMITTGEN ET AL: "Real-time PCR quantification of precursor and mature microRNA", METHODS : A COMPANION TO METHODS IN ENZYMOLOGY, vol. 44, no. 1, 23 December 2007 (2007-12-23), pages 31-38, XP022398577, ACADEMIC PRESS INC., NEW YORK, NY, US ISSN: 1046-2023, DOI: 10.1016/J.YMETH.2007.09.006
- O'HARA ANDREA J ET AL: "Gene alteration and precursor and mature microRNA transcription changes contribute to the miRNA signature of primary effusion lymphoma", BLOOD, vol. 111, no. 4, February 2008 (2008-02), pages 2347-2353, XP002626284, ISSN: 0006-4971
- CHEN TIAN SHENG ET AL: "Mesenchymal stem cell secretes microparticles enriched in pre-microRNAs.", NUCLEIC ACIDS RESEARCH, vol. 38, no. 1, 22 October 2009 (2009-10-22), pages 215-224, XP002626285, ISSN: 1362-4962
- VALADI H ET AL: "Exosome-mediated transfer of mRNAs and microRNAs is a novel mechanism of genetic exchange between cells", NATURE CELL BIOLOGY, vol. 9, no. 6, 1 June 2007 (2007-06-01), pages 654-659, XP002546815, NATURE PUBLISHING GROUP, GB ISSN: 1465-7392, DOI: 10.1038/NCB1596 [retrieved on 2007-05-07]
- RABINOWITS, G.GERCEL-TAYLOR, C.DAY, J.M.TAYLOR, D.D.KLOECKER, G.H.: "Exosomal microRNA: a diagnostic marker for lung cancer", CLIN LUNG CANCER, vol. 10, 20 September 2001 (2001-09-20), pages 42-46, XP002595815, cited in the application
- TAYLOR, D.D.GERCEL-TAYLOR, C.: "MicroRNA signatures of tumor-derived exosomes as diagnostic biomarkers of ovarian cancer", GYNECOL ONCOL, vol. 110, 20 August 2001 (2001-08-20), pages 13-21, XP0022795052, cited in the application
- SZE SIU KWAN ET AL: "Elucidating the secretion proteome of human embryonic stem cell-derived mesenchymal stem cells", MOLECULAR & CELLULAR PROTEOMICS, vol. 6, no. 10, 1 October 2007 (2007-10-01), pages 1680-1689, XP002531526, AMERICAN SOCIETY FOR BIOCHEMISTRY AND MOLECULAR BIOLOGY, INC, US ISSN: 1535-9476, DOI: 10.1074/MCP.M600393-MCP200 cited in the application
- NAGAI ATSUSHI ET AL: "Multilineage potential of stable human mesenchymal stem cell line derived from fetal marrow.", PLOS ONE, vol. 2, no. 12, E1272, 2007, pages 1-18, XP002635355, ISSN: 1932-6203 cited in the application
- TIMMERS LEO ET AL: "Reduction of myocardial infarct size by human mesenchymal stem cell conditioned medium.", STEM CELL RESEARCH, vol. 1, no. 2, November 2007 (2007-11), pages 129-137, XP002635356, ISSN: 1876-7753 cited in the application
- CHUNG YOUNG ET AL: "Human embryonic stem cell lines generated without embryo destruction", CELL STEM CELL, ELSEVIER, CELL PRESS, AMSTERDAM, NL, vol. 2, no. 2, 7 February 2008 (2008-02-07), pages 113-117, XP002604696, ISSN: 1934-5909, DOI: 10.1016/J.STEM.2007.12.013

## Description

### FIELD

The present invention relates to the fields of medicine, cell biology, molecular biology and genetics. This invention relates to the field of medicine.

In particular, it relates to methods of monitoring the physiological or pathological state of a cell or organism. The invention also relates to the diagnosis and treatment of diseases such as cancer.

Reference is made to United States Patent Publication Numbers 2008/0199849, 2008/0219957 and 2010/0323027. Reference is also made to International Patent Publication Numbers WO2006/027545, WO2007/027157, WO2007/027156, WO2008/020815 and WO2009/105044.

### SUMMARY

The invention is set out in the claims.

The following also describes the invention. A method of monitoring the state of a cell, the method comprising establishing, for a selected microRNA (miRNA) species secreted by the cell, a ratio of: (a) a precursor form of the miRNA species (pre-miRNA); to (b) a mature form of the miRNA species (mature miRNA); in which the pre- to mature miRNA ratio so established is indicative of the state of the cell.

The cell may be comprised in a cell mass, tissue, organ or organism and the pre- to mature miRNA ratio is indicative of the state of the respective cell mass, tissue, organ or organism. The miRNA species may be comprised in exosomes secreted by the cell and the method may comprise obtaining such exosomes and obtaining precursor and mature forms of the miRNA species therefrom. The exosomes may be obtained from a sample of an organism. The organism may comprise the cell. The sample may comprise a blood sample, a serum sample, a saliva sample or a urine sample.

The pre- to mature miRNA ratio may be established by hybridisation to an array comprising nucleic acid sequences capable of binding to and distinguishing between precursor and mature forms of the miRNA species. The pre- to mature miRNA ratio may be established by real time polymerase chain reaction (RT-PCR).

The method may comprise establishing a profile comprising a plurality of pre- to mature miRNA ratios for a plurality of selected miRNA species, each indicative of the state of the cell. The profile may be established by hybridisation to an array comprising a plurality of probes capable of binding to and distinguishing between precursor and mature forms of the plurality of the selected miRNA species.

The state of the cell may comprise a physiological state, such as a cell cycle state, a differentiation state, a development state or a metabolic state or a pathological state, such as a disease state, a human disease state, a diabetic state, an immune disorder state, a neurodegenerative disorder state, an oncogenic state, a cancerous state or a tumour state.

The invention also relates to a method: (a) for detecting a change in state of a cell, the method comprising detecting a change in a pre- to mature miRNA ratio of the cell (or a profile comprising such a ratio), in which such a change indicates a change in state of the cell. We provide for a method for establishing that a cell is in a particular state, the method comprising comparing a pre- to mature miRNA ratio of the cell (or a profile comprising such a ratio) with a pre- to mature miRNA ratio (or a profile comprising such a ratio) of a cell known to be in that particular state. We provide for a method of monitoring the state of an organism, the method comprising obtaining a sample from or of that organism, performing a method according to any preceding claim on the sample, and establishing the state of the organism from the pre- to mature miRNA ratio thereby obtained. We provide for a method of detecting a disease in an organism, the method comprising obtaining a sample from or of that organism, performing a method according to any preceding claim on the sample, and comparing the pre-to mature miRNA ratio thereby obtained with a pre- to mature miRNA ratio of a sample known to be of or from a diseased organism. We provide for a method of treatment or prevention of a disease in an organism, the method comprising detecting a disease in an organism as set out above, and administering a treatment for that disease to the organism.

The cell may comprise a mesenchymal stem cell (MSC). The states of the cell may comprise an untransformed state and a transformed state. The transformed state may comprise a c-myc transformed state. The microRNA may comprise hsa-let-7b microRNA (miRBase Accession Number: MI0000063). The ratio may be 2.8 higher in a transformed state compared to a normal state. Any combination of the above may be possible.

The invention also relates to an array comprising a plurality of nucleic acid sequences capable of binding to and distinguishing between precursor and mature forms of a plurality of miRNA species.

The invention also relates to a method comprising the steps of: (a) providing a mesenchymal stem cell (MSC); and (b) introducing an oncogene into the mesenchymal stem cell to thereby transform it; in which the transformed mesenchymal stem cell does not secrete a gene product of the oncogene into a medium in which it is grown.

The oncogene may comprise c-myc. The mesenchymal stem cell (MSC) may comprise an established cell line. The mesenchymal stem cell (MSC) may be derived from umbilical cord.

The transformed mesenchymal stem cell may be capable of bypassing senescence. The transformed mesenchymal stem cell may have an increased proliferation rate as compared to a mesenchymal stem cell that has not been transformed. The transformed mesenchymal stem cell may have a decreased population doubling time such as 24 hours, as compared to a mesenchymal stem cell that has not been transformed. The transformed mesenchymal stem cell may have an increased telomerase activity as compared to a mesenchymal stem cell that has not been transformed.

The description describes a method of providing a conditioned medium, the method comprising the steps set out above, followed by culturing the transformed mesenchymal stem cell, or a descendent thereof, in a cell culture medium to condition it, and separating the conditioned from the transformed mesenchymal stem cell.

The invention also relates to a method of providing an exosome, the method comprising: (a) obtaining a mesenchymal stem cell conditioned medium (MSC-CM) by a method according to Claim 12; (b) concentrating the mesenchymal stem cell conditioned medium, for example by ultrafiltration over a >1000 kDa membrane; (c) subjecting the concentrated mesenchymal stem cell conditioned medium to size exclusion chromatography, such as using a a TSK Guard column SWXL, 6 x 40 mm or a TSK gel G4000 SWXL, 7.8 x 300 mm column; and (d) selecting UV absorbant fractions, for example, at 220 nm, that exhibit dynamic light scattering, such as detected by a quasi-elastic light scattering (QELS); in which step (d) for example comprises collecting fractions which elute with a retention time of 11-13 minutes, such as 12 minutes.

The description describes a method of preparing a pharmaceutical composition, the method comprising obtaining a mesenchymal stem cell conditioned medium (MSC-CM) or an exosome by a method set out above and admixing the MSC-CM or exosome, as the case may be, with a pharmaceutically acceptable carrier or diluent.

The transformed mesenchymal stem cell, the conditioned medium, exosome or pharmaceutical composition may comprise at least one biological property of a mesenchymal stem cell. The biological property may comprise cardioprotection. The conditioned medium, exosome or pharmaceutical composition may be capable of reducing infarct size for example as assayed in a mouse or pig model of myocardial ischemia and reperfusion injury. The conditioned medium, exosome or pharmaceutical composition may be capable of reducing oxidative stress for example as assayed in an *in vitro* assay of hydrogen peroxide (H₂O₂)-induced cell death.

The invention also relates to an immortalised mesenchymal stem cell, a conditioned medium, an exosome or a pharmaceutical composition obtainable by methods described above.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principle and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies: A Laboratory Manual: Portable Protocol NO. I by Edward Harlow, David Lane, Ed Harlow (1999, Cold Spring Harbor Laboratory Press, ISBN 0-87969-544-7); Antibodies: A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3.

### BRIEF DESCRIPTION OF THE FIGURES

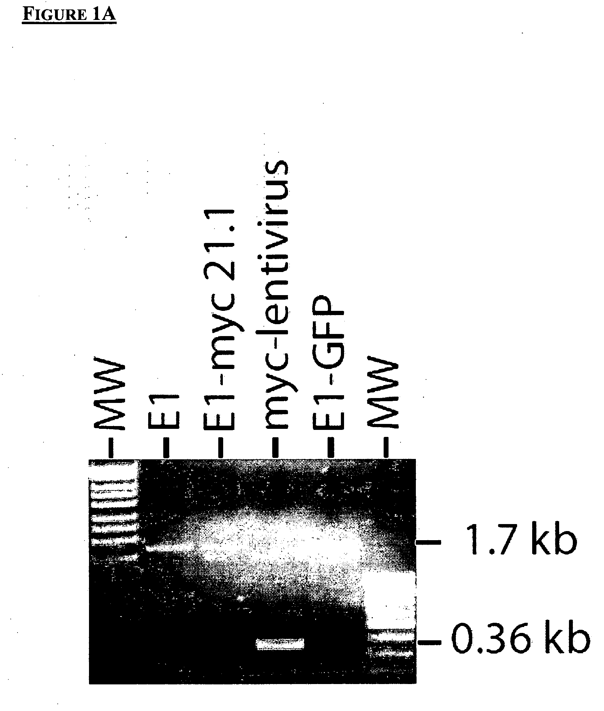

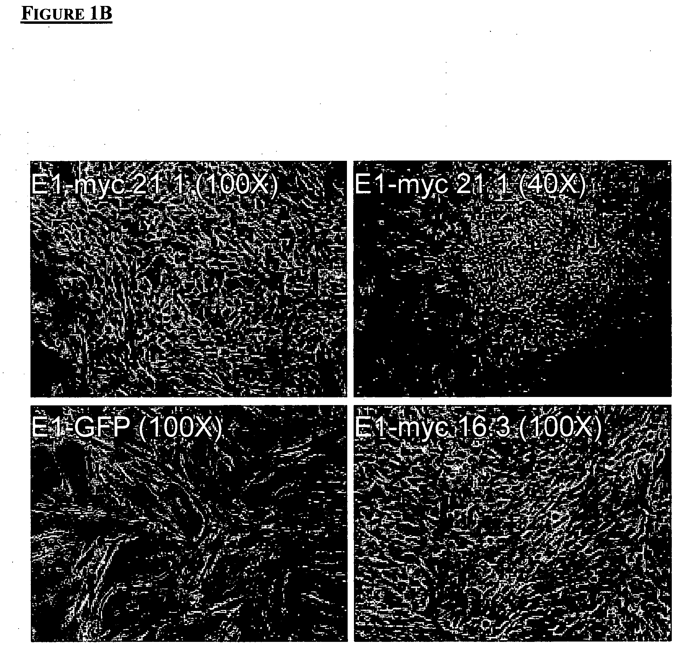

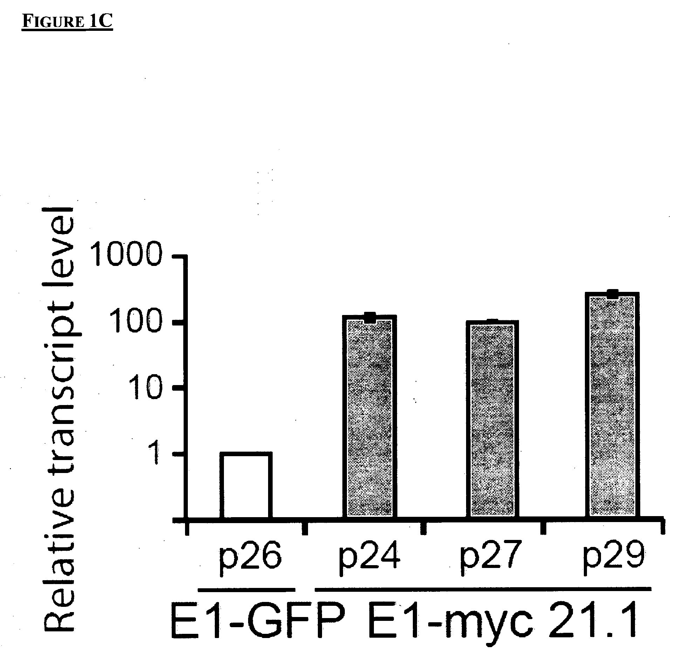

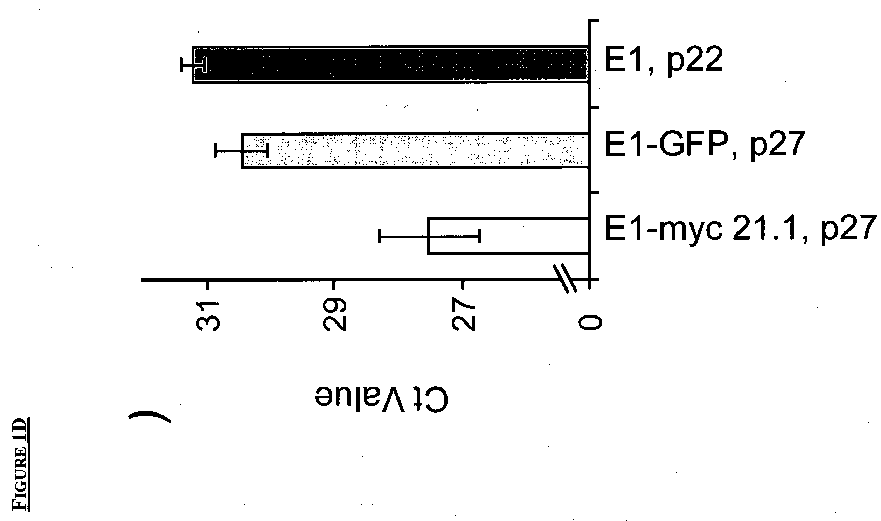

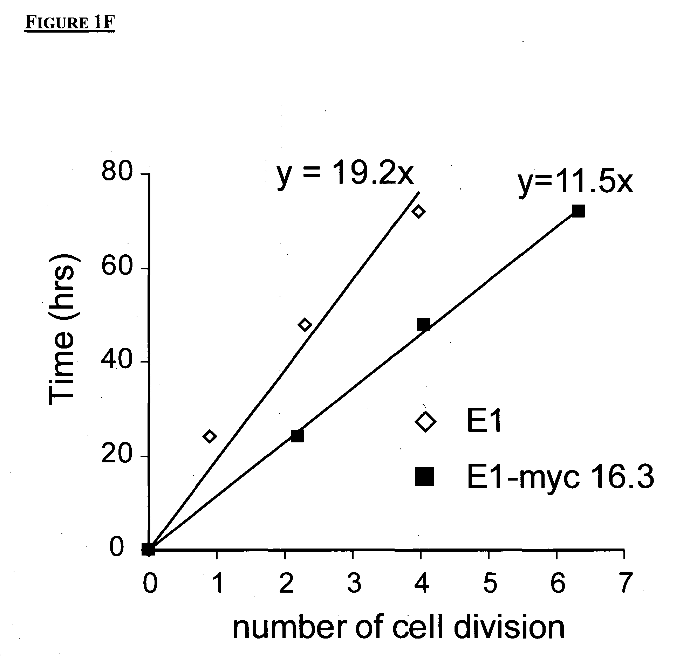

Figure 1A, Figure 1B, Figure 1C, Figure 1D, Figure IE and Figure 1F are diagrams showing transformation of hESC-MSC.
Figure 1A. PCR analysis of cellular DNA from myc-transfected HuES9.E1 MSCs (E1-myc 21.1), GFP transfected HuES9.E1 MSCs (E1-GFP) and the parental MSCs, HuES9.E1 (E1). DNA was amplified using primers specific for exon 2 and exon 3, respectively. The expected PCR fragment size for the endogenous myc gene was 1.7kb and for the transfected myc cDNA was 0.36kb as represented by the amplified fragment from the myc-lentivirus.
Figure 1B. Cell Morphology of transfected MSCs as observed under light microscopy.
Figure 1C. Quantitative RT-PCR was performed on RNA from different passages of E1-myc 21.1 and GFP-MSCs for the level of c-myc mRNA. The relative myc-transcript level was normalized to that in GFP-MSCs.
Figure 1D. Relative telomerase activity. 1 µg of cell lysate protein was first used to extend a TS primer by telomerase activity and the telomerase product was then quantitated by real time PCR. The Ct value represented the amount of telomerase product and was therefore indirectly proportional to telomerase activity in the lysate.

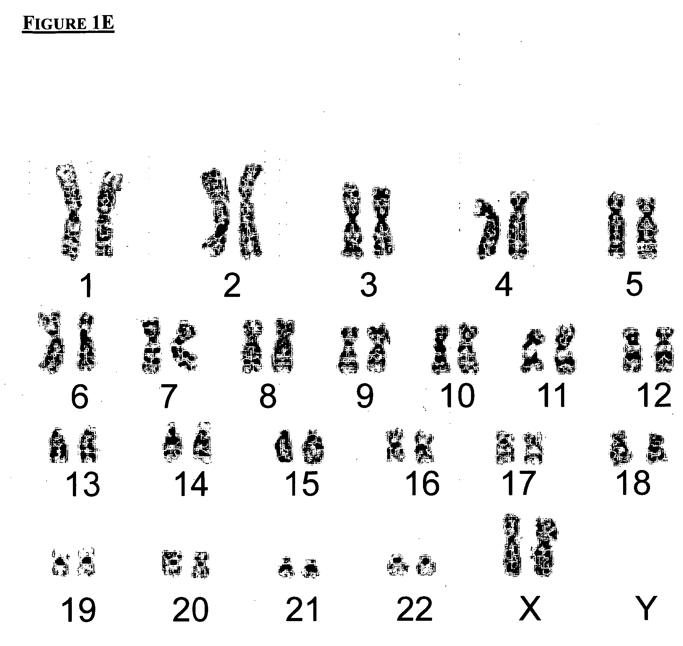

Figure 1E. Karyotpye analysis of E1-myc 16.3 by G-banding.
Figure IF. Rate of cell cycling. Cells were labelled with CFDA and their fluorescence was monitored over time by flow cytometry. The loss of cellular fluorescence at each time point was used to calculate the number of cell division that the cells have undergone as described in Materials and Methods.

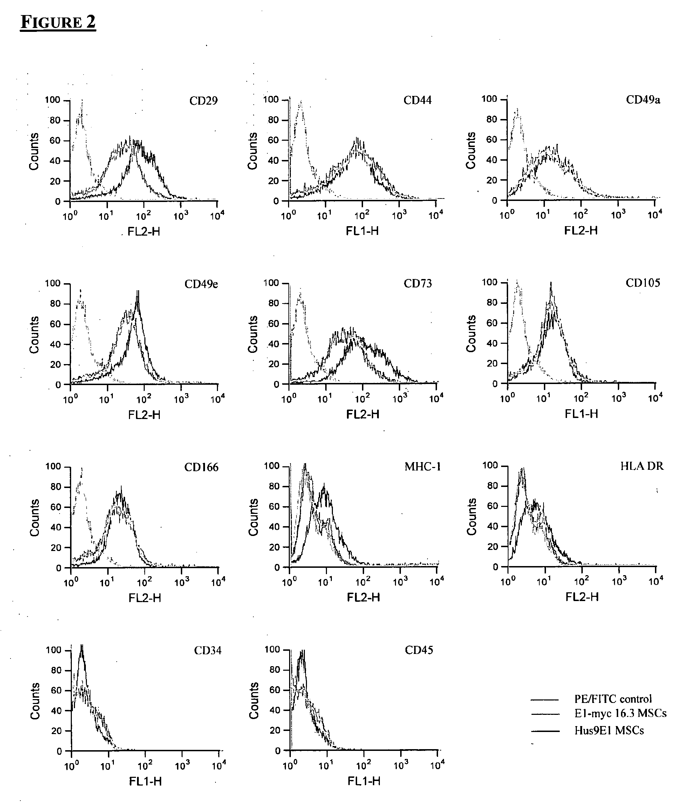

Figure 2 is a diagram showing surface antigen profiling. HuES9.E1 and E1-myc 16.3 MSCs were stained with a specific antibody conjugated to a fluorescent dye and analyzed by FACS. The fluorescence of E1-myc 16.3 was the average cellular fluorescence of cells at p16 or p6. Nonspecific fluorescence was assessed by incubating the cells with isotype-matched mouse monoclonal antibodies

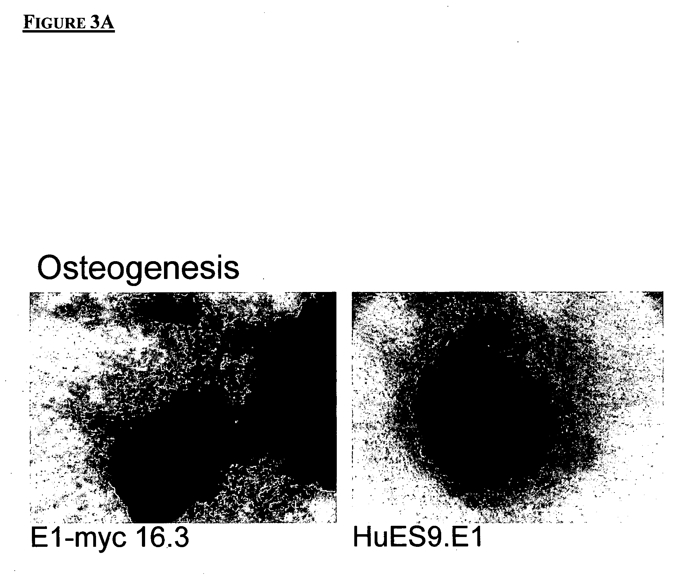

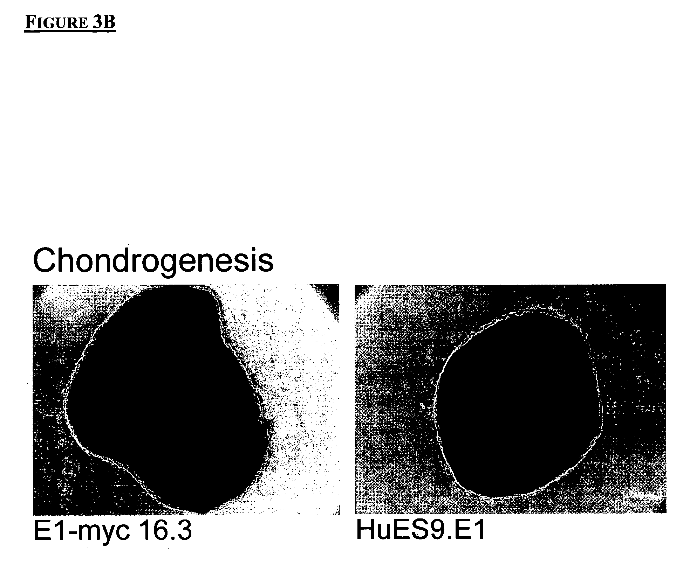

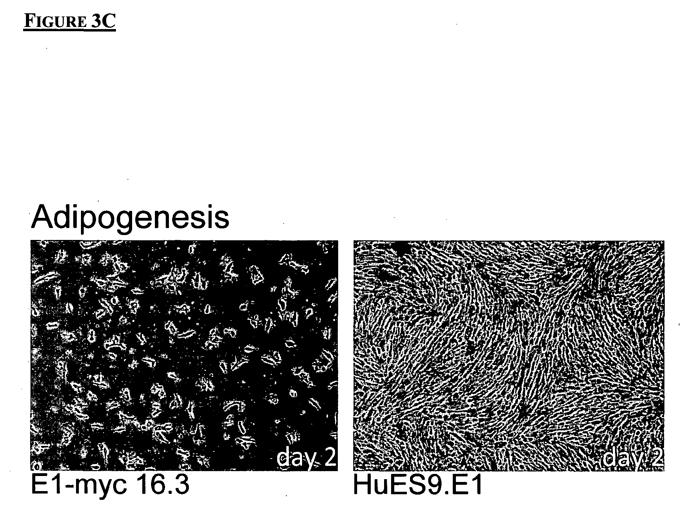

Figure 3A, Figure 3B and Figure 3C are diagrams showing differentiation of Hus9E1 and E1-myc 16.3 MSCs. MSCs were induced to undergo (Figure 3A) osteogenesis and then stained with von Kossa stain; (Figure 3B) chondrogenesis and then stained with Alcian blue; (Figure 3C) adipogenesis where E1-myc 16.3 and Hus9E1 MSCs were exposed to adipogenesis induction medium for two days.

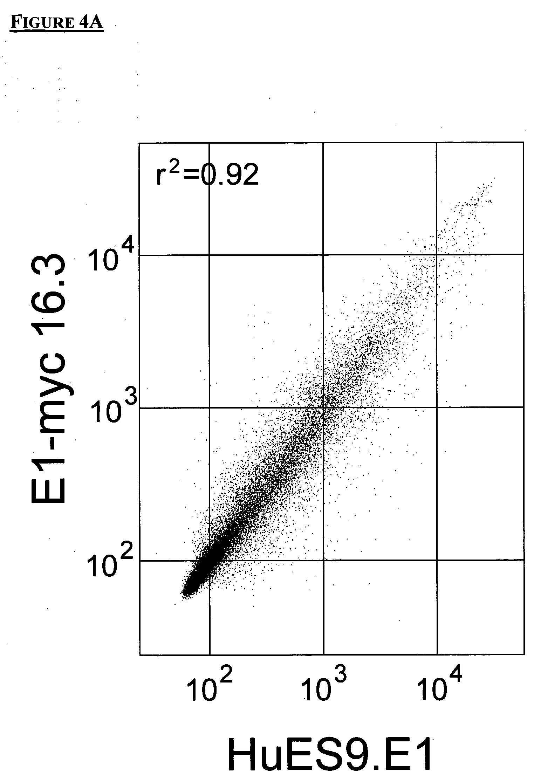

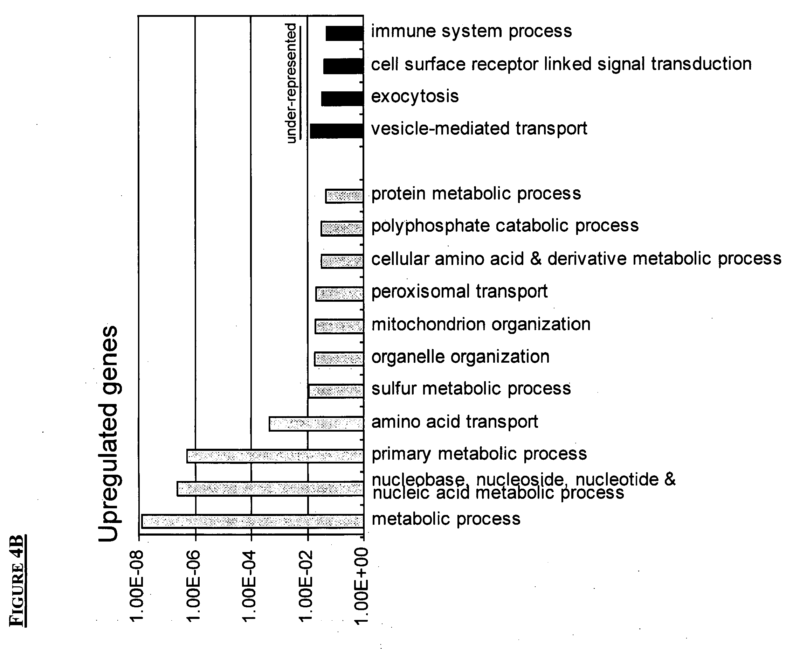

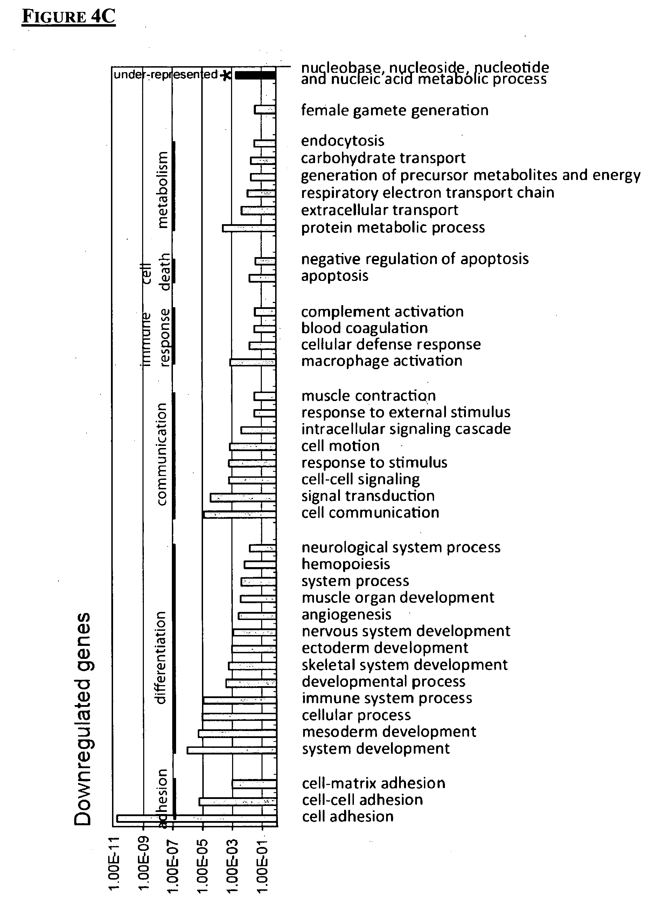

Figure 4A, Figure 4B and Figure 4C are diagrams showing gene expression analysis. RNA from Hus9E1 and E1-myc 16.3 MSCs were hybridized to Sentrix HumanRef-8 Expression BeadChip Version 3 and analysed by Beadstudio and Genespring GX 10.
Figure 4A. Pairwise comparison of gene expression between HuES9.E1 and E1-myc 16.3 MSCs using Beadstudio analysis.
Figure 4B and Figure 4C. PANTHER analysis. 161 genes that were over-expressed by > 2-fold and 226 under-expressed genes that were under-expressed by > 2-fold in E1-myc 16.3 MSCs were analyzed using PANTHER algorithm. The observed frequency of genes for each biological process in each gene set was compared with the reference frequency which, in this case is the frequency of genes for that biological process in the NCBI database. Those biological processes whose observed frequency exceeds the reference frequency with a p < 0.05 are considered significant.
Figure 5A, Figure 5B and Figure 5C are diagrams showing analysis of secretion.

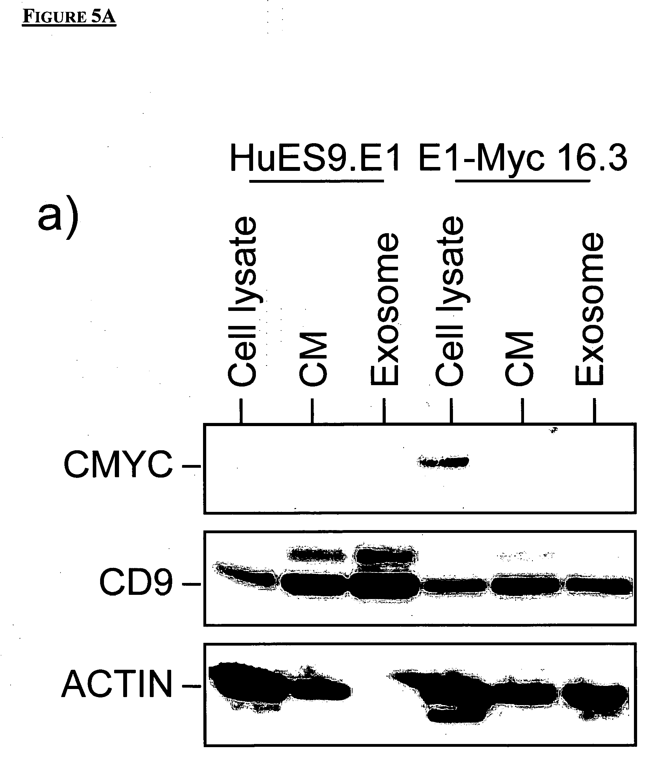

Figure 5A. Western blot analysis. Proteins from cell lysate, conditioned medium (CM), and HPLC-purified exosomes of E1MSCs or E1myc-MSCs were separated on SDS-PAGE and probed with anti-myc, anti-Actin, and anti-CD9 antibodies.

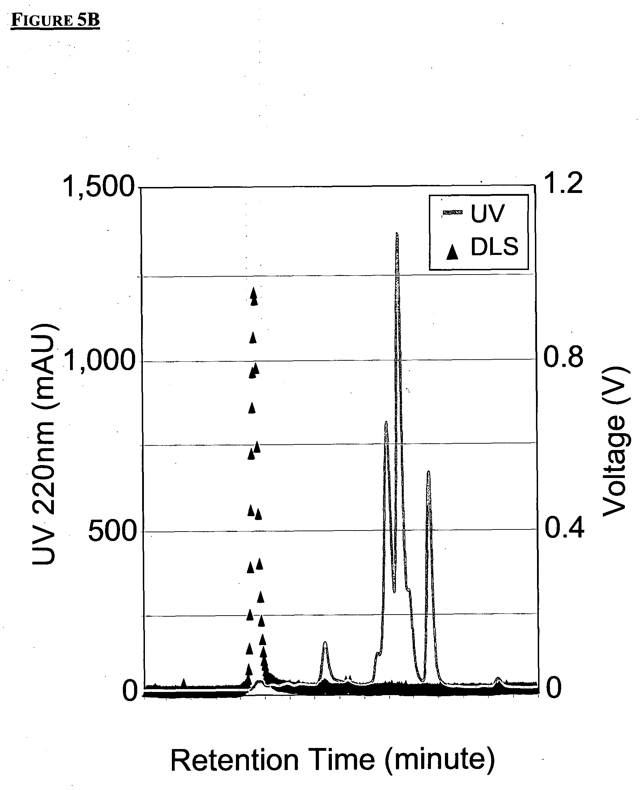

Figure 5B. HPLC fractionation and dynamic light scattering of CM from E1myc-MSC. CM was fractionated on a HPLC using BioSep S4000, 7.8 mm x 30 cm column. The components in CM were eluted with 20 mM phosphate buffer with 150 mM of NaCl at pH 7.2. The elution mode was isocratic and the run time was 40 minutes. The eluent was monitored for UV absorbance at 220 ηm. Each eluting peak was then analyzed by light scattering. The fastest eluting peak (arrow) was collected for testing in a mouse model of ischemia/reperfusion injury.

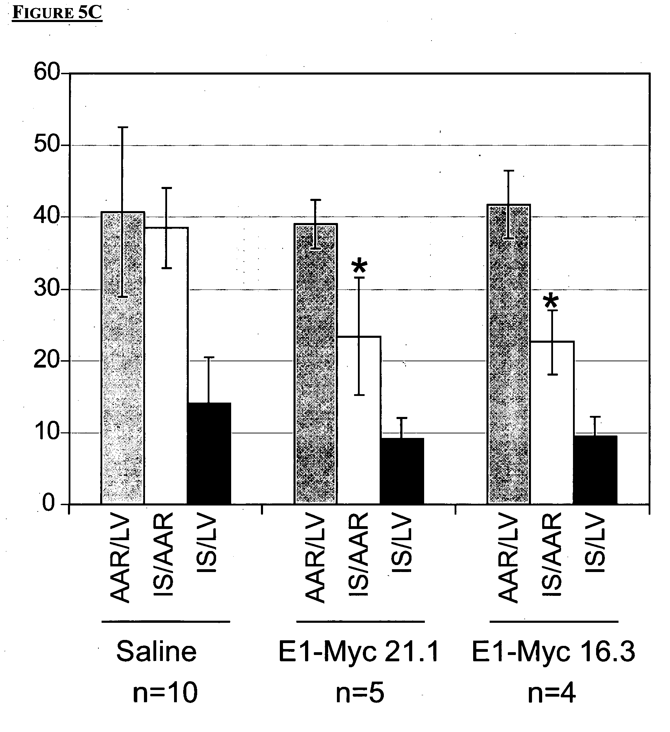

Figure 5C. 0.3 µg HPLC-purified exosomes was administered intravenously to a mouse model of acute myocardial/ischemia reperfusion injury five minutes before reperfusion. Infarct size (IS) as a percentage of the area at risk (AAR) upon treatment with saline (n=10), exosomes from E1myc 21.1 (n=5) and exosomes from E1myc 16.3 (n=4) were measured. The relative infarct size (IS/AAR) in mice treated with E1-myc 21.1 exosome or E1-myc 16.3 exosome was 23.4 ± 8.2%, and 22.6 ± 4.5%, respectively and their relative infarct sizes were significantly lower than the relative infarct size of 38.5 ±5.6% in saline-treated mice (p<0.001 and p<0.002, respectively).

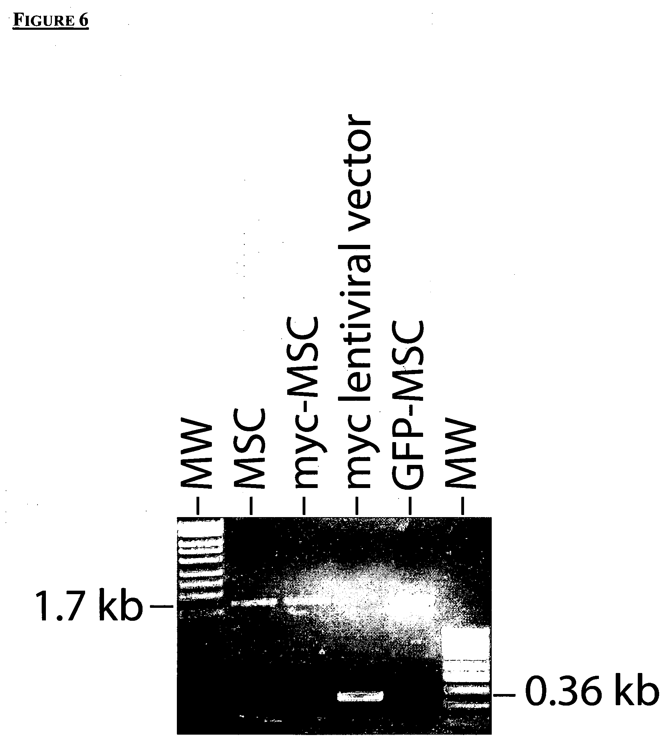

Figure 6 is a diagram showing transformation of hESC-MSC. Figure 6 shows PCR analysis of cellular DNA from myc-transfected MSCs (myc-MSC), GFP transfected MSCs (GFP-MSC) and the parental MSCs, HuES9.E1 (MSC). DNA was amplified using primers specific for exon2 and exon 3, respectively. The expected PCR fragment size for the endogenous myc gene was 1.7kb and for the transfected myc cDNA was 0.36kb.

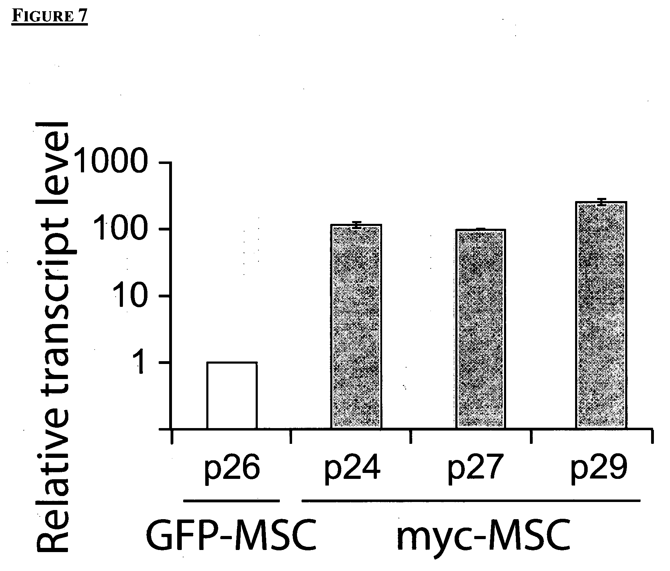

Figure 7 shows quantitative RT-PCR performed on RNA from myc-MSCs or GFP-MSCs for c-myc mRNA.

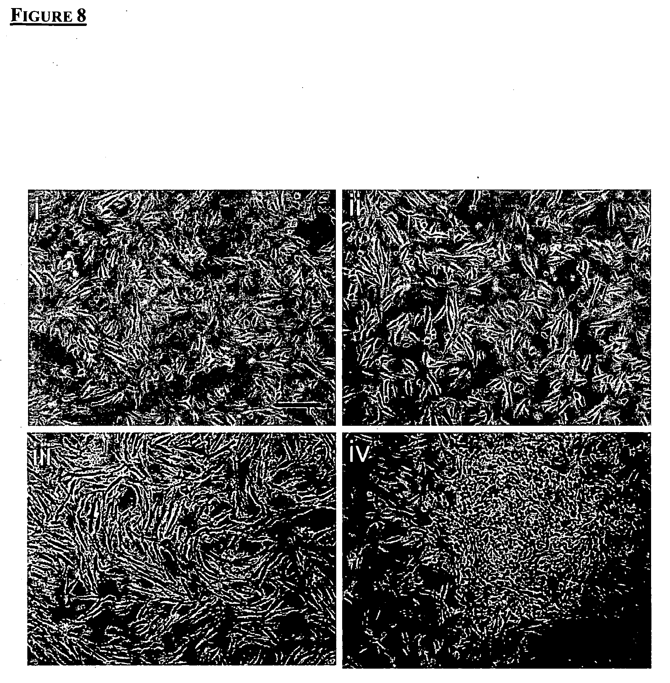

Figure 8 shows cell Morphology of hESC-MSCs after transfection. HuES9.E1 ESC-MSCs at passage 22 were transfected with either c-myc or GFP containing lentiviral vectors. The morphology of c-myc or GFP transfected cells were observed under light microscopy after transfection at passage 22 and then one passage later at passage 23.

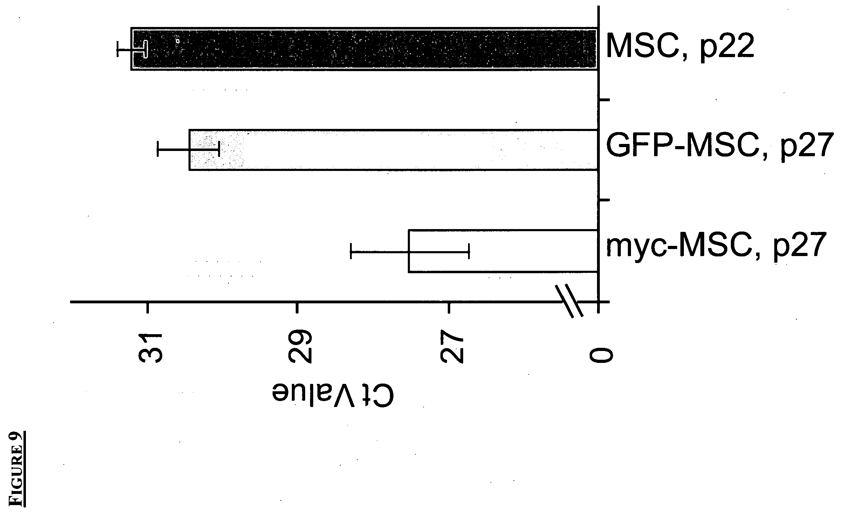

Figure 9 shows relative telomerase activity. 1 µg of cell lysate protein was first used to extend a TS primer by telomerase activity and the telomerase product was then quantitated by real time PCR. The Ct value which represented the amount of telomerase product was therefore indirectly proportional to telomerase activity in the lysate.

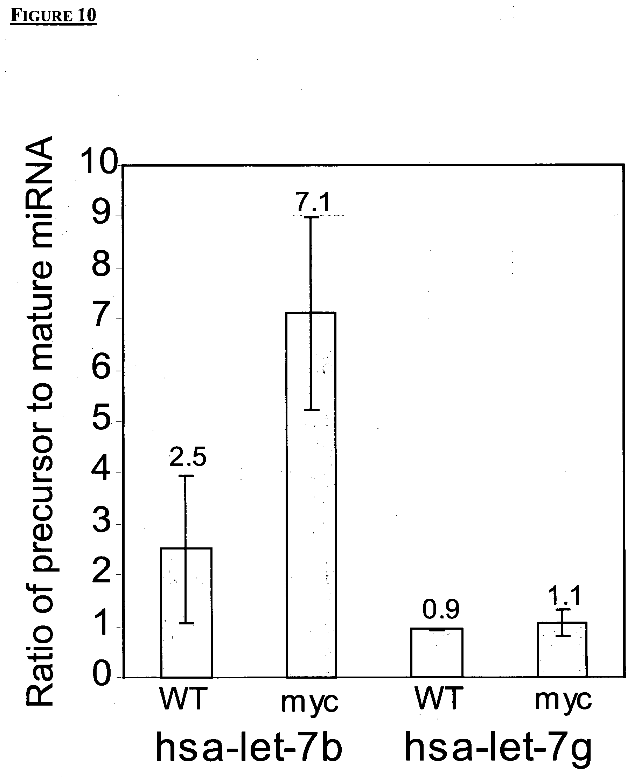

Figure 10 is a diagram showing ratio of precursor to mature miRNA in cell secretion. Culture medium conditioned by either parental MSCs or myc-MSCs were harvested, extracted for RNA and then assayed for mature and precursor miRNA. Quantitative RT-PCR was performed on the RNA using primers specific for the pre- and mature forms of hsa-let-7b (miRBase Accession Number: MI0000063) and hsa-let-7g miRNAs and the ratio of pre- and mature hsa-let-7b (miRBase Accession Number: MI0000063) and hsa-let-7g miRNA calculated.

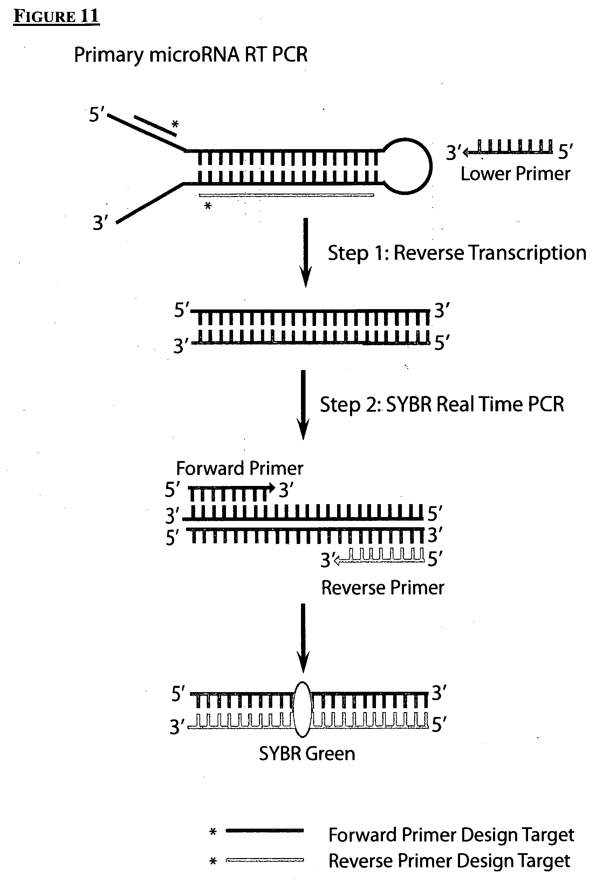

Figure 11 is a diagram showing the design of primers to identify primary forms of miRNA.

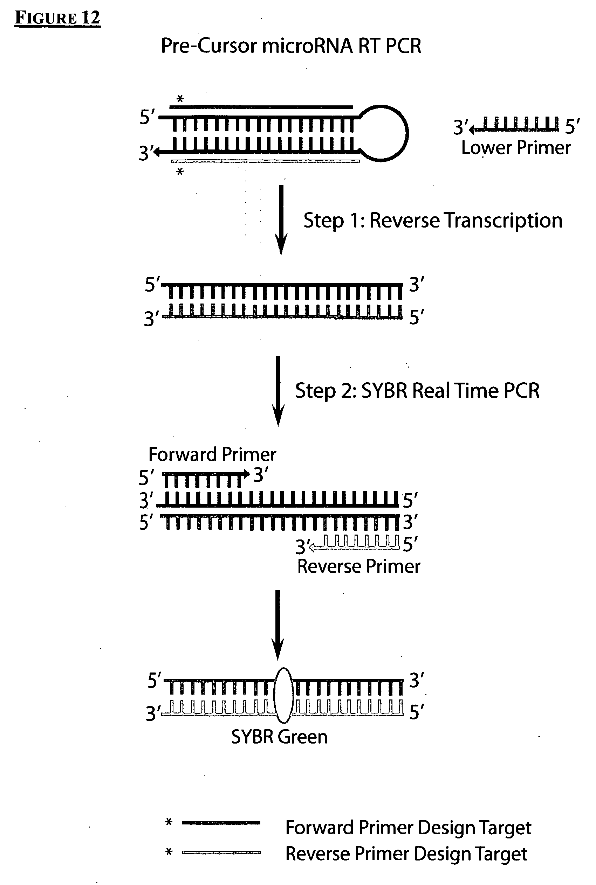

Figure 12 is a diagram showing the design of primers to identify precursor forms of miRNA.

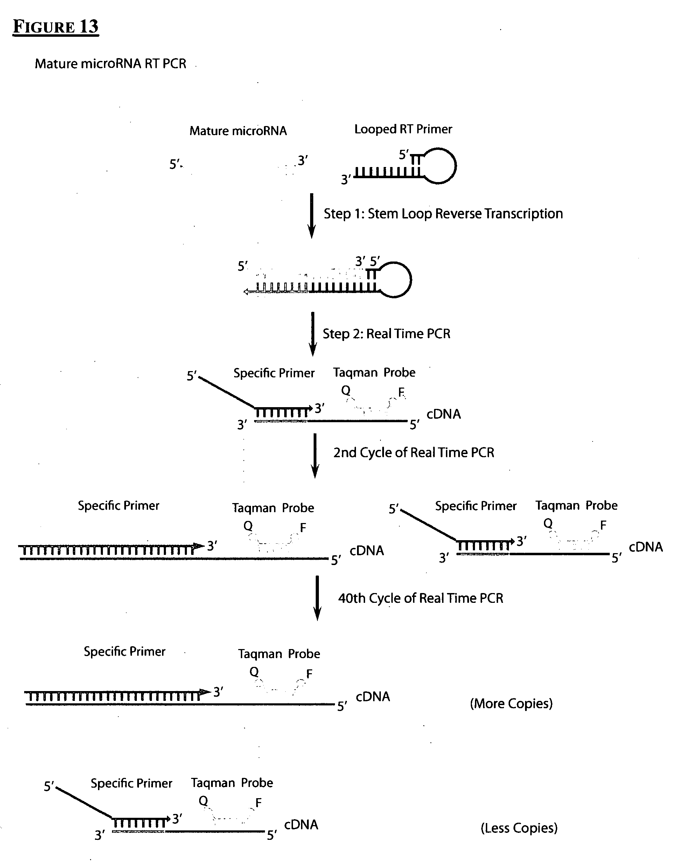

Figure 13 is a diagram showing the design of primers to identify mature forms of miRNA.

### DETAILED DESCRIPTION

Mesenchymal stem cells (MSCs) are multipotent stem cells that have a limited but robust potential to differentiate into mesenchymal cell types, e.g. adipocytes, chondrocytes and osteocytes, with negligible risk of teratoma formation. MSC transplantation has been used to treat musculoskeletal injuries, improve cardiac function in cardiovascular disease and ameliorate the severity of graft-versus-host-disease [1]. In recent years, MSC transplantations have demonstrated therapeutic efficacy in treating different diseases but the underlying mechanism has been controversial [2,3,4,5,6,7,8,9]. Some reports have suggested that factors secreted by MSCs [10] were responsible for the therapeutic effect on arteriogenesis [11], stem cell crypt in the intestine [12], ischemic injury [9,13,14,15,16,17,18], and hematopoiesis [19,20]. In support of this paracrine hypothesis, many studies have observed that MSCs secrete cytokines, chemokines and growth factors that could potentially repair injured cardiac tissue mainly through cardiac and vascular tissue growth and regeneration [21,22]. This paracrine hypothesis could potentially provide for a non-cell based alternative for using MSC in treatment of cardiovascular disease [23]. Non-cell based therapies as opposed to cell-based therapies are generally easier to manufacture and are safer as they are non-viable and do not elicit immune rejection.

We have previously demonstrated that culture medium conditioned by mesenchymal stem cells (MSCs) that were derived from human embryonic stem cells or fetal tissues [24,25,26] could protect the heart from myocardial ischemia/reperfusion injury and reduce infarct size in both pig and mouse models of MI/R injury [25,26,27]. Subsequent studies demonstrated that this cardioprotection was mediated by exosomes or microparticles of about 50-100 ηm in diameter and these microparticles carry both protein and RNA load [24,25,26,27,28]. These exosomes could be purified as a population of homogenously sized particles by size exclusion on HPLC [25,26] and reduced infarct size in a mouse model of MI/R injury at about a tenth of the dosage of the conditioned medium.

The identification of exosomes as the therapeutic agent in the MSC secretion could potentially provides for a biologic- rather than cell-based treatment modality. Unlike cells, exosomes do not elicit acute immune rejection and being non-viable and much smaller. They pose less safety risks such as the formation of tumor or embolism. Unlike cell-based therapies where there is a need to maintain viability, manufacture and storage of non-viable exosomes is less complex and therefore less costly. Besides being therapeutic agents, exosomes have been advocated as 'natural' drug delivery vehicles [29]. These lipid vesicles could be loaded with therapeutic agents and be used to deliver the agents in a cell type specific manner. hESC-MSCs could be the ideal cellular source for the efficient production of exosomes. We have demonstrated that these cells could be grown in a chemically defined during the production and harvest of exosomes and these exosomes could be purified by HPLC to generate a population of homogenously sized particles [25,26,27]. Another advantage is that these cells were derived from hESC, an infinitely expansible cell source.

While hESC-MSCs are also highly expansible in culture, they unlike their parental hESC can undergo only a finite number of cell divisions before their growth is arrested and they senesce. Therefore there will be a need to constantly derive new batches of MSCs from hESCs to replenish the cell source of exosomes with each derivation necessitating recurring cost of derivation, testing and validation. To circumvent this need for re-derivation and ensure an infinite supply of identical MSCs for commercially sustainable production of exosomes as therapeutic agents or delivery vehicle, we explore the use of oncogenic transformation to bypass senescence. Oncogenic transformation could potentially alter the cell biology and affect the production or the properties of the exosomes. It was previously reported that transfection of v-myc gene into fetal MSCs immortalized the cells but did not alter the fundament characteristics of these MSCs [30].. Here we transfected a lentiviral vector with the c-myc gene which encodes for the MYC protein into the previously described huES9.E1 MSCs [24] at passage 22 and 16 to generate a pooled cell line and three independently derived clonal cell lines, respectively. We examined the transformed cells according to the ISCT minimal defining criteria for MSCs [31] namely plastic adherence, surface antigen profile of CD29⁺, CD44⁺, CD49a⁺ CD49e⁺, CD105⁺, CD166⁺, MHCI⁺, CD34⁻, CD45⁻ and HLA-DR⁻, and potential to differentiate into adipocytes, chondrocytes and osteocytes. The secretion of these cells was evaluated for the presence of exosomes and the therapeutic efficacy of these exosomes were tested in a mouse model of myocardial ischemia/reperfusion injury (MI/R) as previously described [27].

### TRANSFORMED MESENCHYMAL STEM CELLS

Exosomes or secreted bi-lipid vesicles from human ESC-derived mesenchymal stem cells (hESC-MSCs) have been shown to reduce myocardial ischemia/reperfusion injury in animal models. However as hESC-MSCs are not infinitely expansible, large scale production of these exosomes would require replenishment of hESC-MSC through derivation from hESCs and incur recurring costs for testing and validation of each new batch.

Here we investigate if c-myc immortalization of hESC-MSC would circumvent this constraint without compromising the production of therapeutically efficacious exosomes. Myc-transformed MSCs largely resembled the parental hESC-MSCs with major differences being reduced plastic adherence, faster growth, failure to senesce, increased myc expression and loss of *in vitro* adipogenic potential that technically rendered the transformed cells as non-MSCs. Nevertheless, exosomes from myc-transformed MSCs were able to reduce relative infarct size in a mouse model of myocardial ischemia/reperfusion injury indicating that the capacity for producing therapeutic exosomes was preserved.

We therefore first provide for a transformed mesenchymal stem cell. The transformed mesenchymal stem cell may be obtainable or derivable from a mesenchymal stem cell, such as by transformation with a suitable transforming agent, such as an oncogene. We further provide a descendent of such a transformed mesenchymal stem cell, a cell culture or a cell line comprising either. Such cells may be referred to in this document as 'transformed mesenchymal stem cells' or a transformed mesenchymal stem cells obtained by the methods described in this document.

We therefore describe a method comprising the steps of: (a) providing a mesenchymal stem cell (MSC); and (b) introducing an oncogene into the mesenchymal stem cell to thereby transform it; in which the transformed mesenchymal stem cell does not secrete a gene product of the oncogene into a medium in which it is grown.

The oncogene may comprise c-myc.

The mesenchymal stem cell (MSC) may comprise an established cell line.

The mesenchymal stem cell (MSC) may be derived from umbilical cord.

The transformed mesenchymal stem cell may be capable of bypassing senescence as compared to a mesenchymal stem cell that has not been transformed. The transformed mesenchymal stem cell have an increased proliferation rate as compared to a mesenchymal stem cell that has not been transformed. The transformed mesenchymal stem cell may have a decreased population doubling time such as 24 hours as compared to a mesenchymal stem cell that has not been transformed. The transformed mesenchymal stem cell may have an increased telomerase activity as compared to a mesenchymal stem cell that has not been transformed.

We describe a method of providing a conditioned medium, the method comprising the steps set out above, followed by culturing the transformed mesenchymal stem cell, or a descendent thereof, in a cell culture medium to condition it, and separating the conditioned from the transformed mesenchymal stem cell.

We further describe a method of providing an exosome, the method comprising: (a) obtaining a mesenchymal stem cell conditioned medium (MSC-CM) by a method as set out above; (b) concentrating the mesenchymal stem cell conditioned medium, for example by ultrafiltration over a >1000 kDa membrane; (c) subjecting the concentrated mesenchymal stem cell conditioned medium to size exclusion chromatography, such as using a a TSK Guard column SWXL, 6 x 40 mm or a TSK gel G4000 SWXL, 7.8 x 300 mm column; and (d) selecting UV absorbant fractions, for example, at 220 nm, that exhibit dynamic light scattering, such as detected by a quasi-elastic light scattering (QELS); in which step (d) for example comprises collecting fractions which elute with a retention time of 11-13 minutes, such as 12 minutes.

We further describe a method of preparing a pharmaceutical composition, the method comprising obtaining a mesenchymal stem cell conditioned medium (MSC-CM) by a method as set out above or an exosome by a method as set out above and admixing the MSC-CM or exosome, as the case may be, with a pharmaceutically acceptable carrier or diluent.

The conditioned medium, exosome or pharmaceutical composition may comprise at least one biological property of a mesenchymal stem cell such as a biological activity of a mesenchymal stem cell conditioned medium (MSC-CM), for example cardioprotection.

The conditioned medium, exosome or pharmaceutical composition may be capable of reducing infarct size for example as assayed in a mouse or pig model of myocardial ischemia and reperfusion injury, or which is capable of reducing oxidative stress for example as assayed in an *in vitro* assay of hydrogen peroxide (H₂O₂)-induced cell death.

The conditioned medium, exosome or pharmaceutical composition may be suitable for treating: a disease selected from the group consisting of: cardiac failure, bone marrow disease, skin disease, burns and degenerative diseases such as diabetes, Alzheimer's disease, Parkinson's disease, cancer, myocardial infarction, a cutaneous wound, a dermatologic disorder, a dermatological lesion, dermatitis, psoriasis, condyloma, verruca, hemangioma, keloid, skin cancer, atopic dermatitis, Behcet disease, chronic granulomatous disease, cutaneous T cell lymphoma, ulceration, a pathological condition characterised by initial injury inducing inflammation and immune dysregulation leading to chronic tissue remodeling including fibrosis and loss of function, renal ischemic injury, cystic fibrosis, sinusitis and rhinitis or an orthopaedic disease or may be used to aid wound healing, scar reduction, bone formation, a bone graft or bone marrow transplantation in an individual, or (i) in the regulation of a pathway selected from any one or more of the following: cytoskeletal regulation by Rho GTPase, cell cycle, integrin signalling pathway, Inflammation mediated by chemokine & cytokine signaling pathway, FGF signaling pathway, EGF receptor signaling pathway, angiogenesis, plasminogen activating cascade, blood coagulation, glycolysis, ubiquitin proteasome pathway, de novo purine biosynthesis, TCA cycle, phenylalanine biosynthesis, heme biosynthesis; (ii) in the regulation of processes including any one or more of the following: cell structure and motility, cell structure, cell communication, cell motility, cell adhesion, endocytosis, mitosis, exocytosis, cytokinesis, cell cycle, immunity and defense, cytokine/chemokine mediated immunity, macrophage-mediated immunity, granulocyte-mediated immunity, ligand-mediated signaling, cytokine and chemokine mediated signaling pathway, signal transduction, extracellular matrix protein-mediated signaling, growth factor homeostasis, receptor protein tyrosine kinase signaling pathway, cell adhesion-mediated signaling, cell surface receptor mediated signal transduction, JAK-STAT cascade, antioxidation and free radical removal, homeostasis, stress response, blood clotting, developmental processes, mesoderm development, skeletal development, angiogenesis, muscle development, muscle contraction, protein metabolism and modification, proteolysis, protein folding, protein complex assembly, amino acid activation, intracellular protein traffic, other protein targeting and localization, amino acid metabolism, protein biosynthesis, protein disulfide-isomerase reaction, carbohydrate metabolism, glycolysis, pentose-phosphate shunt, other polysaccharide metabolism, purine metabolism, regulation of phosphate metabolism, vitamin metabolism, amino acid biosynthesis, pre-mRNA processing, translational regulation, mRNA splicing; or (iii) in the supply of functions including any one or more of the following: signaling molecule, chemokine, growth factor, cytokine, interleukin, other cytokine, extracellular matrix, extracellular matrix structural protein, other extracellular matrix, extracellular matrix glycoprotein, protease, metalloprotease, other proteases, protease inhibitor, metalloprotease inhibitor, serine protease inhibitor, oxidoreductase, dehydrogenase, peroxidase, chaperone, chaperonin, Hsp 70 family chaperone, other chaperones, synthetase, synthase and synthetase, select calcium binding protein, aminoacyl-tRNA synthetase, lyase, isomerase, other isomerase, ATP synthase, hydratase, transaminase, other lyase, other enzyme regulator, select regulatory molecule, actin binding cytoskeletal protein, cytoskeletal protein, non-motor actin binding protein, actin and actin related protein, annexin, tubulin, cell adhesion molecule, actin binding motor protein, intermediate filament, ribonucleoprotein, ribosomal protein, translation factor, other RNA-binding protein, histone, calmodulin related protein, vesicle coat protein.

We further describe an immortalised mesenchymal stem cell obtainable by a method as set out above.

We further describe a conditioned medium obtainable by a method as set out above.

We further describe aexosome obtainable by a method as set out above.

We further describe a pharmaceutical composition obtainable by a method as set out above.

We further describe a delivery system for delivering a conditioned medium, an exosome or a pharmaceutical composition, comprising a source of conditioned medium as set out above, a source of an exosome as set out above, or a source of a pharmaceutical composition as set out above, together with a dispenser operable to deliver the conditioned medium, exosome or pharmaceutical composition to a target.

We further describe the use of a delivery system as set out above in a method of delivering a conditioned medium, exosome or a pharmaceutical composition to a target.

The transformed mesenchymal stem cell, cell culture or cell line may comprise cardioprotective activity. The cardioprotective activity may be assayed in a mouse model of acute myocardial infarction (AMI).

The transformed mesenchymal stem cell, cell culture or cell line may be obtainable by transforming a mesenchymal stem cell obtained by any means. The mesenchymal stem cell cell from which the transformed mesenchymal stem cell is derived may comprise any suitable mesenchymal stem cell, for example derived from an embryonic stem cell, a foetal cell, such as a cadaveric foetal cell or any other suitable tissue source. The mesenchymal stem cell may be obtained or derived from an embryonic stem cell line, or a foetal cell line. Similarly, the transformed mesenchymal stem cell may be established from a mesenchymal stem cell line.

The mesenchymal stem cell may also be established from a pre-natal cell, such as a foetal cell which is a first or second trimester foetal cell. The foetal cell may comprise a cell of any suitable age, for example up to 2, 4, 8, 16 or 24 weeks. The foetal cell may comprise a cell from any tissue such as a limb cell, a kidney cell or a liver cell. The pre-natal cell from which the mesenchymal stem cell is derived may be comprised in a tissue. The tissue may be derived from a cadaveric foetus. The mesenchymal stem cell may comprise a mammalian, primate or human mesenchymal stem cell.

The method may further comprise culturing the mesenchymal stem cell in a serum free medium. The serum free medium may comprise serum-free culture medium such as Knockout DMEM medium. This may be supplemented with 10% serum replacement media. It may be supplemented with non-essential amino acids. It may be supplemented with 10 ng/ml FGF2. It may be supplemented with 10ng/ml Recombinant Human EGF. It may be supplemented with 55µM β-mercaptoethanol.

The transformed mesenchymal stem cell, cell culture or cell line may be such that the transformed mesenchymal stem cell displays one or more of the following characteristics. The transformed mesenchymal stem cell may display one or more morphological characteristics of mesenchymal stem cells. Such a characteristic may include fingerprint whorl at confluency. It may include forming an adherent monolayer with a fibroblastic phenotype.

The transformed mesenchymal stem cell, cell culture or cell line may be capable of adhering to plastic. The transformed mesenchymal stem cell, cell culture or cell line may display an average population doubling time of between 72 to 96 hours. The transformed mesenchymal stem cell, cell culture or cell line may display a surface antigen profile comprising expression of one or more, such as all, of the following: CD29, CD44, CD49a, CD49e, Cod105, CD166, MHC I. It may display a reduced or absent expression of one or more of the following: HLA-DR, CD34 and CD45. The transformed mesenchymal stem cell, cell culture or cell line may be CD29+, CD44+, CD49a+ CD49e+, CD105+, CD166+, MHC I+, CD34⁻ and CD45⁻.

The transformed mesenchymal stem cell, cell culture or cell line may display a reduced expression of one or more, such as all, of HESX1, POUFL5, SOX-2, UTF-1 and ZFP42. The transformed mesenchymal stem cell, cell culture or cell line may display a reduced expression of one or more, such as all, of OCT4, NANOG and SOX2. The transformed mesenchymal stem cell, cell culture or cell line may display no detectable alkaline phosphatase activity.

The transformed mesenchymal stem cell, cell culture or cell line may be maintainable in cell culture for greater than 10, 20, 30, 40 or more generations. The transformed mesenchymal stem cell, cell culture or cell line may have a substantially stable karyotype or chromosome number when maintained in cell culture for at least 10 generations. The transformed mesenchymal stem cell, cell culture or cell line may be such that it does not substantially induce formation of teratoma when transplanted to a recipient animal. The recipient animal may comprise an immune compromised recipient animal. The time period may be after 3 weeks, such as after 2 to 9 months. The transformed mesenchymal stem cell, cell culture or cell line may be such that it not teratogenic when implanted in SCID mice.

The transformed mesenchymal stem cell, cell culture or cell line may be such that it is negative for mouse-specific *c-mos* repeat sequences and positive for human specific *alu* repeat sequences. The transformed mesenchymal stem cell, cell culture or cell line may be capable of undergoing osteogenesis, adipogenesis or chondrogenesis, such as capable of differentiating into osteocytes, adipocytes or chondrocytes. The transformed mesenchymal stem cell, cell culture or cell line may have a substantially stable gene expression pattern from generation to generation.

The transformed mesenchymal stem cell, cell culture or cell line may be such that any two or more, such as all, transformed mesenchymal stem cells obtainable by the method exhibit substantially identical gene expression profiles. It may be such that the gene expression correlation coefficient between any two or more isolates of transformed mesenchymal stem cells obtained by the method is greater than 0.9. It may be such that any two or more, such as all, isolates of transformed mesenchymal stem cells obtainable by the method are substantially similar or identical (such as homogenous) with each other. It may be such that the gene expression correlation coefficient between a transformed mesenchymal stem cell obtainable by the method and cells of a parental culture is greater than 0.8.

The transformed mesenchymal stem cell, cell culture or cell line may comprise a mammalian, such as a mouse or human, transformed mesenchymal stem cell, cell culture or cell line.

We further provide for a conditioned medium comprising medium conditioned by a transformed mesenchymal stem cell, cell culture or cell line as described above. Also provided for is a particle secreted by a transformed mesenchymal stem cell as described above and comprising at least one biological property of a transformed mesenchymal stem cell such as a biological activity of a transformed mesenchymal stem cell conditioned medium (MSC-CM), for example cardioprotection. These are described in further detail below.

We also provide for a transformed mesenchymal stem cell, cell culture, cell line, conditioned medium or particle as set out above for use in a method of treatment of a disease. The disease may be selected from the group consisting of: cardiac failure, bone marrow disease, skin disease, burns and degenerative diseases such as diabetes, Alzheimer's disease, Parkinson's disease, cancer, a disease associated with accumulation of protein aggregates or intracellular or extracellular lesions; Huntingdon's disease and alcoholic liver disease.

The disease may be selected from the group consisting of: myocardial infarction, a cutaneous wound, a dermatologic disorder, a dermatological lesion, dermatitis, psoriasis, condyloma, verruca, hemangioma, keloid, skin cancer, atopic dermatitis, Behcet disease, chronic granulomatous disease, cutaneous T cell lymphoma, ulceration, a pathological condition characterised by initial injury inducing inflammation and immune dysregulation leading to chronic tissue remodeling including fibrosis and loss of function, renal ischemic injury, cystic fibrosis, sinusitis and rhinitis or an orthopaedic disease.

The transformed mesenchymal stem cell, cell culture, cell line, conditioned medium or particle may be used to aid wound healing, scar reduction, bone formation, a bone graft or bone marrow transplantation.

The transformed mesenchymal stem cell, cell culture, cell line, conditioned medium or particle may be used to regulate or assist in the regulation of a pathway selected from any one or more of the following: cytoskeletal regulation by Rho GTPase, cell cycle, integrin signalling pathway, Inflammation mediated by chemokine & cytokine signaling pathway, FGF signaling pathway, EGF receptor signaling pathway, angiogenesis, plasminogen activating cascade, blood coagulation, glycolysis, ubiquitin proteasome pathway, de novo purine biosynthesis, TCA cycle, phenylalanine biosynthesis, heme biosynthesis.

The transformed mesenchymal stem cell, cell culture, cell line, conditioned medium or particle may be used in the regulation of processes including any one or more of the following: cell structure and motility, cell structure, cell communication, cell motility, cell adhesion, endocytosis, mitosis, exocytosis, cytokinesis, cell cycle, immunity and defense, cytokine/chemokine mediated immunity, macrophage-mediated immunity, granulocyte-mediated immunity, ligand-mediated signaling, cytokine and chemokine mediated signaling pathway, signal transduction, extracellular matrix protein-mediated signaling, growth factor homeostasis, receptor protein tyrosine kinase signaling pathway, cell adhesion-mediated signaling, cell surface receptor mediated signal transduction, JAK-STAT cascade, antioxidation and free radical removal, homeostasis, stress response, blood clotting, developmental processes, mesoderm development, skeletal development, angiogenesis, muscle development, muscle contraction, protein metabolism and modification, proteolysis, protein folding, protein complex assembly, amino acid activation, intracellular protein traffic, other protein targeting and localization, amino acid metabolism, protein biosynthesis, protein disulfide-isomerase reaction, carbohydrate metabolism, glycolysis, pentose-phosphate shunt, other polysaccharide metabolism, purine metabolism, regulation of phosphate metabolism, vitamin metabolism, amino acid biosynthesis, pre-mRNA processing, translational regulation, mRNA splicing.

The transformed mesenchymal stem cell, cell culture, cell line, conditioned medium or particle may be used in the supply of functions including any one or more of the following: signaling molecule, chemokine, growth factor, cytokine, interleukin, other cytokine, extracellular matrix, extracellular matrix structural protein, other extracellular matrix, extracellular matrix glycoprotein, protease, metalloprotease, other proteases, protease inhibitor, metalloprotease inhibitor, serine protease inhibitor, oxidoreductase, dehydrogenase, peroxidase, chaperone, chaperonin, Hsp 70 family chaperone, other chaperones, synthetase, synthase and synthetase, select calcium binding protein, aminoacyl-tRNA synthetase, lyase, isomerase, other isomerase, ATP synthase, hydratase, transaminase, other lyase, other enzyme regulator, select regulatory molecule, actin binding cytoskeletal protein, cytoskeletal protein, non-motor actin binding protein, actin and actin related protein, annexin, tubulin, cell adhesion molecule, actin binding motor protein, intermediate filament, ribonucleoprotein, ribosomal protein, translation factor, other RNA-binding protein, histone, calmodulin related protein, vesicle coat protein.

Also provided is a delivery system for delivering a conditioned medium or particle, comprising a source of conditioned medium or particle as described above together with a dispenser operable to deliver the conditioned medium or particle to a target.

We further provide for the use of such a delivery system in a method of delivering a conditioned medium or particle to a target.

There is also provided a method of obtaining a differentiated mesenchymal cell, the method comprising providing a transformed mesenchymal stem cell, cell culture or cell line as described above and differentiating the transformed mesenchymal stem cell, cell culture or cell line into an osteocyte, an adipocyte or a chondrocyte.

We further provide a differentiated mesenchymal cell obtainable by such a method.

Also provided is a pharmaceutical composition comprising a transformed mesenchymal stem cell, cell culture, cell line, conditioned medium or particle as set out above or a conditioned medium as described, together with a pharmaceutically acceptable excipient or carrier.

We provide for a method of conditioning a cell culture medium, the method comprising culturing a transformed mesenchymal stem cell, cell culture or cell line as described in a cell culture medium and optionally isolating the cell culture medium.

We further provide a method of treatment of a disease comprising obtaining a transformed mesenchymal stem cell, cell culture, cell line, conditioned medium or particle as set out above and administering the transformed mesenchymal stem cell, cell culture, cell line, conditioned medium or particle into a patient.

The disease may be selected from the diseases set out above.

### OBTAINING TRANSFORMED MESENCHYMAL STEM CELLS

Transformed mesenchymal stem cells may be obtained or derived using the methods and compositions described here from a mesenchymal stem cell, such as by genetic engineering. The genetic engineering may comprise transformation with a suitable transforming agent, such as an oncogene.

We therefore describe methods for immortalizing or transforming such cell lines by transfecting a mesenchymal stem cells with a vector comprising at least one transforming gene.

Examples of transforming genes include: (1) nuclear oncogenes such as v-myc, N-myc, T antigen and Ewing's sarcoma oncogene (Fredericksen et al. (1988) Neuron 1:439-448; Bartlett, P. et al. (1988) Proc. Natl. Acad. Sci. USA 85:3255-3259, and Snyder, E. Y. et al. (1992) Cell 68:33-51), (2) cytoplasmic oncogenes such as bcr-abl and neurofibromin (Solomon, E. et al. (1991) Science 254:1153-1160), (3) membrane oncogenes such as neu and ret (Aaronson, A. S. A. (1991) Science 254:1153-1161), (4) tumor suppressor genes such as mutant p53 and mutant Rb (retinoblastoma) (Weinberg, R. A. (1991) Science 254:1138-1146), and (5) other transforming genes such as Notch dominant negative (Coffman, C. R. et al. (1993) Cell 23:659-671). Examples of oncogenes include v-myc and the SV40 T antigen.

Transforming genes may further include telomerase gene (Tert) as well as oncogenes such as Akt, ras, EGF receptor etc.

Foreign (heterologous) nucleic acid such as encoding transforming genes may be introduced or transfected into mesenchymal stem cell. A mesenchymal stem cell which harbors foreign DNA is said to be a genetically-engineered cell. The foreign DNA may be introduced using a variety of techniques. In one embodiment, foreign DNA, including DNA encoding a transforming gene, is introduced into mesenchymal stem cell using the technique of retroviral transfection. Recombinant retroviruses harboring the gene(s) of interest are used to introduce marker genes, such as the E. coli β-galactosidase (lacZ) gene, or oncogenes. The recombinant retroviruses are produced in packaging cell lines to produce culture supernatants having a high titer of virus particles (generally 10⁵ to 10⁶ pfu/ml). The recombinant viral particles are used to infect cultures of the mesenchymal stem cell by incubating the cell cultures with medium containing the viral particles and 8 µg/ml polybrene for three hours. Following retroviral infection, the cells are rinsed and cultured in standard medium. The infected cells are then analyzed for the uptake and expression of the foreign DNA. The cells may be subjected to selective conditions which select for cells that have taken up and expressed a selectable marker gene.

In another embodiment, the foreign DNA may be introduced using the technique of calcium-phosphate-mediated transfection. A calcium-phosphate precipitate containing DNA encoding the gene(s) of interest, such a transforming gene, is prepared using the technique of Wigler et al. (1979) Proc. Natl. Acad. Sci. USA 76:1373-1376. Cultures of the mesenchymal stem cell are established in tissue culture dishes. Twenty four hours after plating the cells, the calcium phosphate precipitate containing approximately 20 µg/ml of the foreign DNA is added. The cells are incubated at room temperature for 20 minutes. Tissue culture medium containing 30 µM chloroquine is added and the cells are incubated overnight at 37° C. Following transfection, the cells are analyzed for the uptake and expression of the foreign DNA. The cells may be subjected to selection conditions which select for cells that have taken up and expressed a selectable marker gene.

As used herein, the term 'non-transformed cells' means cells which are able to grow *in vitro* without the need to immortalize the cells by introduction of a virus or portions of a viral genome containing an oncogene(s) which confers altered growth properties upon cells by virtue of the expression of viral genes within the transformed cells. These viral genes typically have been introduced into cells by means of viral infection or by means of transfection with DNA vectors containing isolated viral genes.

As used herein, the term 'genetically-engineered cell' refers to a cell into which a foreign (i.e., non-naturally occurring) nucleic acid, e.g., DNA, has been introduced. The foreign nucleic acid may be introduced by a variety of techniques, including, but not limited to, calcium-phosphate-mediated transfection, DEAE-mediated transfection, microinjection, retroviral transformation, protoplast fusion and lipofection. The genetically-engineered cell may express the foreign nucleic acid in either a transient or long-term manner. In general, transient expression occurs when foreign DNA does not stably integrate into the chromosomal DNA of the transfected cell. In contrast, long-term expression of foreign DNA occurs when the foreign DNA has been stably integrated into the chromosomal DNA of the transfected cell.

As used herein, an 'immortalized cell' or a 'transformed cell' means a cell which is capable of growing indefinitely in culture due to the introduction of an 'immortalizing gene' or 'transforming gene' which confers altered growth properties upon the cell by virtue of expression of the immortalizing or transforming gene(s) within the genetically engineered cell. Immortalizing genes and transforming genes can be introduced into cells by means of viral infection or by means of transfection with vectors containing isolated viral nucleic acid encoding one or more oncogenes. Viruses or viral oncogenes are selected which allow for the immortalization but preferably not the transformation of cells. Immortalized cells may be such that they grow indefinitely in culture but do not cause tumors when introduced into animals.

As used herein, the term 'transformed cell' refers to a cell having a transforming gene introduced into it and having the ability to grow indefinitely in culture. 'Transformation' refers to the generation of a transformed cell.

### TRANSFORMED MESENCHYMAL STEM CELL CULTURES AND CELL LINES

It will be evident that such a transformed mesenchymal stem cell that is obtained by the methods described here may be maintained as a cell or developed into a cell culture or a cell line.

Accordingly, in this document, and where appropriate, the term 'transformed mesenchymal stem cell' should be taken also to include reference to a corresponding cell culture, i.e., a transformed mesenchymal stem cell culture or a corresponding cell line, i.e., a transformed mesenchymal stem cell line. In general, the transformed mesenchymal stem cell, cell culture or cell line may be maintained in culture in the same or similar conditions and culture media as described above for derivation.

### TRANSFORMED MESENCHYMAL STEM CELL CONDITIONED MEDIUM

We further provide a medium which is conditioned by culture of the transformed mesenchymal stem cells. Such a conditioned medium is referred to in this document as a 'transformed mesenchymal stem cell conditioned medium' and is described in further detail below.

Such a conditioned medium may comprise molecules secreted, excreted, released, or otherwise produced by the transformed mesenchymal stem cells. The conditioned medium may comprise one or more molecules of the transformed mesenchymal stem cells, for example, polypeptides, nucleic acids, carbohydrates or other complex or simple molecules. The conditioned medium may also comprise one or more activities of the transformed mesenchymal stem cells. The conditioned medium may be used in place of, in addition to, or to supplement the transformed mesenchymal stem cells themselves. Thus, any purpose for which transformed mesenchymal stem cells are suitable for use in, conditioned media may similarly be used for that purpose.

Such a conditioned medium, and combinations of any of the molecules comprised therein, including in particular proteins or polypeptides, may be used to supplement the activity of, or in place of, the transformed mesenchymal stem cells, for the purpose of for example treating or preventing a disease. Thus, where it is stated that the transformed mesenchymal stem cells obtained by the methods described here may be used for a particular purpose, it will be evident that media conditioned by such transformed mesenchymal stem cells may be equally used for that purpose.

Conditioned medium may be made by any suitable method. For example, it may be made by culturing transformed mesenchymal stem cells in a medium, such as a cell culture medium, for a predetermined length of time. Any number of methods of preparing conditioned medium may be employed, including the 'Conditioned Medium Preparation Protocol' set out below.

The transformed mesenchymal stem cells may in particular comprise those produced by any of the methods described in this document. The conditioned medium will comprise polypeptides secreted by the transformed mesenchymal stem cells, as described in the Examples.

A particular example of a protocol for producing conditioned medium, which is not intended to be limiting, is as follows.

An example protocol for preparing conditioned medium from transformed mesenchymal stem cells may comprise a 'Conditioned Medium Preparation Protocol', which is as follows:

### TRANSFORMED MESENCHYMAL STEM CELL CONDITIONED. MEDIUM PREPARATION PROTOCOL

The secretion may be prepared by growing the transformed MSCs in a chemically defined serum free culture medium for three days as described above and in the Examples, and also in reference 25. 80% confluent cultures may be washed three times with PBS, and then cultured overnight in a chemically defined medium consisting of DMEM media without phenol red supplemented with 1 X Insulin-Transferrin-Selenoprotein, 10ng/ml Recombinant Human FGF2, 10ng/ml Recombinant Human EGF, 1 x glutamine-penicillin-streptomycin, and 55µM β-mercaptoethanol overnight. The cultures may then be washed three times with PBS and fresh chemically defined medium may then be added. All medium components may be obtained from Invitrogen. The cultures may be maintained in this medium for three days. This conditioned medium (CM) may then be collected, clarified by centrifugation, concentrated 50 times using tangential flow filtration with MW cut-off of 100 kDa (Satorius, Goettingen, Germany) and sterilized by filtration through a 220 nm filter.

The conditioned medium may be used in therapy as is, or after one or more treatment steps. For example, the conditioned medium may be UV treated, filter sterilised, etc. One or more purification steps may be employed. In particular, the conditioned media may be concentrated, for example by dialysis or ultrafiltration. For example, the medium may be concentrated using membrane ultrafiltration with a nominal molecular weight limit (NMWL) of for example 3K.

For the purposes described in this document, for example, treatment or prevention of disease, a dosage of conditioned medium comprising 15-750 mg protein/100 kg body weight may be administered to a patient in need of such treatment.

### TRANSFORMED MESENCHYMAL STEM CELL PARTICLE

We describe a particle which is derivable from a transformed mesenchymal stem cell (MSC). Such a particle is referred to in this document as a 'transformed mesenchymal stem cell particle'.

The transformed mesenchymal stem cell particle may be derivable from the transformed MSC by any of several means, for example by secretion, budding or dispersal from the transformed MSC. For example, the transformed mesenchymal stem cell particle may be produced, exuded, emitted or shed from the transformed MSC. Where the transformed MSC is in cell culture, the transformed mesenchymal stem cell particle may be secreted into the cell culture medium.

The transformed mesenchymal stem cell particle may in particular comprise a vesicle. The transformed mesenchymal stem cell particle may comprise an exosome. The transformed mesenchymal stem cell particle described here may comprise any one or more of the properties of the exosomes described herein.

The transformed mesenchymal stem cell particle may comprise vesicles or a flattened sphere limited by a lipid bilayer. The transformed mesenchymal stem cell particle may comprise diameters of 40-100 nm. The transformed mesenchymal stem cell particle may be formed by inward budding of the endosomal membrane. The transformed mesenchymal stem cell particle may have a density of ∼1.13 - 1.19 g/ml and may float on sucrose gradients. The transformed mesenchymal stem cell particle may be enriched in cholesterol and sphingomyelin, and lipid raft markers such as GM1, GM3, flotillin and the src protein kinase Lyn. The transformed mesenchymal stem cell particle may comprise one or more proteins present in transformed mesenchymal stem cells or transformed mesenchymal stem cell conditioned medium (transformed MSC-CM), such as a protein characteristic or specific to the transformed MSC or transformed MSC-CM. They may comprise RNA, for example miRNA.

We provide a transformed mesenchymal stem cell particle which comprises one or more genes or gene products found in transformed MSCs or medium which is conditioned by culture of transformed MSCs. The transformed mesenchymal stem cell particle may comprise molecules secreted by the transformed MSC. Such a transformed mesenchymal stem cell particle, and combinations of any of the molecules comprised therein, including in particular proteins or polypeptides, may be used to supplement the activity of, or in place of, the transformed MSCs or medium conditioned by the transformed MSCs for the purpose of for example treating or preventing a disease.

The transformed mesenchymal stem cell particle may comprise a cytosolic protein found in cytoskeleton e.g. tubulin, actin and actin-binding proteins, intracellular membrane fusions and transport e.g. annexins and rab proteins, signal transduction proteins e.g. protein kinases, 14-3-3 and heterotrimeric G proteins, metabolic enzymes e.g. peroxidases, pyruvate and lipid kinases, and enolase-1 and the family of tetraspanins e.g. CD9, CD63, CD81 and CD82. In particular, the transformed mesenchymal stem cell particle may comprise one or more tetraspanins. The transformed mesenchymal stem cell particles may comprise mRNA and/or microRNA.

The term 'particle' as used in this document may be taken to mean a discrete entity. The particle may be something that is isolatable from a transformed mesenchymal stem cell (transformed MSC) or transformed mesenchymal stem cell conditioned medium (transformed MSC-CM). The transformed mesenchymal stem cell particle may be responsible for at least an activity of the transformed MSC or transformed MSC-CM. The transformed mesenchymal stem cell particle may be responsible for, and carry out, substantially most or all of the functions of the transformed MSC or transformed MSC-CM. For example, the transformed mesenchymal stem cell particle may be a substitute (or biological substitute) for the transformed MSC or transformed MSC-CM.

The transformed mesenchymal stem cell particle may be used for any of the therapeutic purposes that the transformed MSC or transformed MSC-CM may be put to use.

The transformed mesenchymal stem cell particle may comprise at least one property of a transformed mesenchymal stem cell. The transformed mesenchymal stem cell particle may have a biological property, such as a biological activity. The transformed mesenchymal stem cell particle may have any of the biological activities of an transformed MSC. The transformed mesenchymal stem cell particle may for example have a therapeutic or restorative activity of an transformed MSC. The biological activity may comprise cardioprotection. The transformed mesenchymal stem cell particle may be capable of reducing infarct size. Reduction of infarct may be assayed in a mouse or pig model of myocardial ischemia and reperfusion injury.

The transformed mesenchymal stem cell particle may be capable of reducing oxidative stress. The reduction of oxidative stress may be assayed in an *in vitro* assay of hydrogen peroxide (H₂O₂)-induced cell death.

The transformed mesenchymal stem cell particle comprise a vesicle. The transformed mesenchymal stem cell particle may comprise an exosome.

The transformed mesenchymal stem cell particle may contain at least 70% of proteins in an mesenchymal stem cell conditioned medium (MSC-CM) or a transformed mesenchymal stem cell conditioned medium (transformed MSC-CM).

The transformed mesenchymal stem cell particle may comprise a complex of molecular weight >100 kDa. The complex of molecular weight > 100 kDa may comprise proteins of <100 kDa. The particle may comprise a complex of molecular weight > 300 kDa. The complex of molecular weight > 100 kDa may comprise proteins of < 300 kDa.

The transformed mesenchymal stem cell particle may comprise a complex of molecular weight > 1000 kDa.The particle may have a size of between 2 nm and 200 nm. The transformed mesenchymal stem cell particle may have a size of between 50ηm and 150 nm. The transformed mesenchymal stem cell particle may have a size of between between 50 nm and 100 nm.

The size of the transformed mesenchymal stem cell particle may be determined by filtration against a 0.2µM filter and concentration against a membrane with a molecular weight cut-off of 10 kDa. The size of the transformed mesenchymal stem cell particle may be determined by electron microscopy.

The transformed mesenchymal stem cell particle may comprise a hydrodynamic radius of below 100 nm. It may comprise a hydrodynamic radius of between about 30 nm and about 70 nm. It may be between about 40 nm and about 60 nm, such as between about 45 nm and about 55 nm. The transformed mesenchymal stem cell particle may comprise a hydrodynamic radius of about 50 nm. The hydrodynamic radius may be determined by laser diffraction or dynamic light scattering.

The transformed mesenchymal stem cell particle may comprise a lipid selected from the group consisting of: phospholipid, phosphatidyl serine, phosphatidyl inositol, phosphatidyl choline, shingomyelin, ceramides, glycolipid, cerebroside, steroids, cholesterol. The cholesterol-phospholipid ratio may be greater than 0.3-0.4 (mol/mol). The transformed mesenchymal stem cell particle may comprise a lipid raft.

The transformed mesenchymal stem cell particle may be insoluble in non-ionic detergent, such as Triton-X100. The transformed mesenchymal stem cell particle may be such that proteins of the molecular weights specified substantially remain in the complexes of the molecular weights specified, when the transformed mesenchymal stem cell particle is treated with a non-ionic detergent.

The transformed mesenchymal stem cell particle may be sensitive to cyclodextrin, such as 20 mM cyclodextrin. The transformed mesenchymal stem cell particle may be such that treatment with cyclodextrin causes substantial dissolution of the complexes specified.

The transformed mesenchymal stem cell particle may comprise ribonucleic acid (RNA). The particle may have an absorbance ratio of 1.9 (260:280 nm). The transformed mesenchymal stem cell particle may comprise a surface antigen selected from the group consisting of: CD9, CD109 and thy-1.

We further describe a method of producing a transformed mesenchymal stem cell particle as described above, the method comprising isolating the transformed mesenchymal stem cell particle from a transformed mesenchymal stem cell conditioned medium (MSC-CM).

The method may comprise separating the transformed mesenchymal stem cell particle from other components based on molecular weight, size, shape, composition or biological activity.

The weight may be selected from the weights set out above. The size may be selected from the sizes set out above. The composition may be selected from the compositions set out above. The biological activity may be selected from the biological activities set out above.

We further describe a method of producing a transformed mesenchymal stem cell particle as described above. The method may comprise obtaining a transformed mesenchymal stem cell conditioned medium (MSC-CM). It may comprise concentrating the transformed mesenchymal stem cell conditioned medium. The transformed mesenchymal stem cell conditioned medium may be concentrated by ultrafiltration over a >1000 kDa membrane. The method may comprise subjecting the concentrated transformed mesenchymal stem cell conditioned medium to size exclusion chromatography. A TSK Guard column SWXL, 6 x 40 mm or a TSK gel G4000 SWXL, 7.8 x 300 mm column may be employed. The method may comprise selecting UV absorbent fractions, for example, at 220 nm, that exhibit dynamic light scattering. The dynamic light scattering may be detected by a quasi-elastic light scattering (QELS) detector. The method may comprise collecting fractions which elute with a retention time of 11-13 minutes, such as 12 minutes.

We further provide a pharmaceutical composition comprising a transformed mesenchymal stem cell particle as described together with a pharmaceutically acceptable excipient, diluent or carrier.

We further provide such a transformed mesenchymal stem cell particle or such a pharmaceutical composition for use in a method of treating a disease.

We further provide for the use of such a transformed mesenchymal stem cell particle for the preparation of a pharmaceutical composition for the treatment of a disease.

We further provide for the use of such a transformed mesenchymal stem cell particle in a method of treatment of a disease in an individual.

The disease may be selected from the group consisting of: cardiac failure, bone marrow disease, skin disease, burns and degenerative diseases such as diabetes, Alzheimer's disease, Parkinson's disease and cancer.

The disease may be selected from the group consisting of: myocardial infarction, a cutaneous wound, a dermatologic disorder, a dermatological lesion, dermatitis, psoriasis, condyloma, verruca, hemangioma, keloid, skin cancer, atopic dermatitis, Behcet disease, chronic granulomatous disease, cutaneous T cell lymphoma, ulceration, a pathological condition characterised by initial injury inducing inflammation and immune dysregulation leading to chronic tissue remodeling including fibrosis and loss of function, renal ischemic injury, cystic fibrosis, sinusitis and rhinitis or an orthopaedic disease.

The transformed mesenchymal stem cell particle may be used to aid wound healing, scar reduction, bone formation, a bone graft or bone marrow transplantation in an individual.

The transformed mesenchymal stem cell particle may be used (i) in the regulation of a pathway selected from any one or more of the following: cytoskeletal regulation by Rho GTPase, cell cycle, integrin signalling pathway, Inflammation mediated by chemokine & cytokine signaling pathway, FGF signaling pathway, EGF receptor signaling pathway, angiogenesis, plasminogen activating cascade, blood coagulation, glycolysis, ubiquitin proteasome pathway, de novo purine biosynthesis, TCA cycle, phenylalanine biosynthesis, heme biosynthesis; (ii) in the regulation of processes including any one or more of the following: cell structure and motility, cell structure, cell communication, cell motility, cell adhesion, endocytosis, mitosis, exocytosis, cytokinesis, cell cycle, immunity and defense, cytokine/chemokine mediated immunity, macrophage-mediated immunity, granulocyte-mediated immunity, ligand-mediated signaling, cytokine and chemokine mediated signaling pathway, signal transduction, extracellular matrix protein-mediated signaling, growth factor homeostasis, receptor protein tyrosine kinase signaling pathway, cell adhesion-mediated signaling, cell surface receptor mediated signal transduction, JAK-STAT cascade, antioxidation and free radical removal, homeostasis, stress response, blood clotting, developmental processes, mesoderm development, skeletal development, angiogenesis, muscle development, muscle contraction, protein metabolism and modification, proteolysis, protein folding, protein complex assembly, amino acid activation, intracellular protein traffic, other protein targeting and localization, amino acid metabolism, protein biosynthesis, protein disulfide-isomerase reaction, carbohydrate metabolism, glycolysis, pentose-phosphate shunt, other polysaccharide metabolism, purine metabolism, regulation of phosphate metabolism, vitamin metabolism, amino acid biosynthesis, pre-mRNA processing, translational regulation, mRNA splicing; or (iii) in the supply of functions including any one or more of the following: signaling molecule, chemokine, growth factor, cytokine, interleukin, other cytokine, extracellular matrix, extracellular matrix structural protein, other extracellular matrix, extracellular matrix glycoprotein, protease, metalloprotease, other proteases, protease inhibitor, metalloprotease inhibitor, serine protease inhibitor, oxidoreductase, dehydrogenase, peroxidase, chaperone, chaperonin, Hsp 70 family chaperone, other chaperones, synthetase, synthase and synthetase, select calcium binding protein, aminoacyl-tRNA synthetase, lyase, isomerase, other isomerase, ATP synthase, hydratase, transaminase, other lyase, other enzyme regulator, select regulatory molecule, actin binding cytoskeletal protein, cytoskeletal protein, non-motor actin binding protein, actin and actin related protein, annexin, tubulin, cell adhesion molecule, actin binding motor protein, intermediate filament, ribonucleoprotein, ribosomal protein, translation factor, other RNA-binding protein, histone, calmodulin related protein, vesicle coat protein.

We further provide for a delivery system for delivering a transformed mesenchymal stem cell particle, comprising a source of transformed mesenchymal stem cell particle together with a dispenser operable to deliver the particle to a target.

We further provide use of such a delivery system in a method of delivering a particle to a target.

The transformed mesenchymal stem cells may mediate cardioprotective effects through secreted large complexes of ∼50-100 nm in diameter. Such complexes or particles may therefore be used for therapeutic means, including for cardioprotection, in place of the cells themselves.

The transformed mesenchymal stem cell particles, complexes or exosomes may be used for a variety of purposes, such as treatment or prevention for cardiac or heart diseases such as ischaemia, cardiac inflammation or heart failure. They may also be used for repair following perfusion injury.

Media conditioned by transformed MSCs (such as transformed mesenchymal stem cell conditioned media or transformed MSC-CM, as described below) may comprise biological activities of transformed MSC and are capable of substituting for the transformed MSCs themselves. The biological property or biological activity of an transformed MSC may therefore correspond to a biological property or activity of a transformed mesenchymal stem cell conditioned medium. Accordingly, the transformed mesenchymal stem cell particle may comprise one or more biological properties or activities of a transformed mesenchymal stem cell conditioned medium (transformed MSC-CM).

The conditioned cell culture medium such as a transformed Mesenchymal Stem Cell Conditioned Medium (transformed MSC-CM) may be obtained by culturing a transformed mesenchymal stem cell (transformed MSC), a descendent thereof or a cell line derived therefrom in a cell culture medium; and isolating the cell culture medium. The transformed mesenchymal stem cell may be produced by a process comprising obtaining a cell by dispersing a embryonic stem (ES) cell colony. The cell, or a descendent thereof, may be propagated in the absence of co-culture in a serum free medium comprising FGF2. Further details are provided elsewhere in this document.

### ISOLATION OF TRANSFORMED MESENCHYMAL STEM CELL PARTICLE

The transformed MSC particle may be produced or isolated in a number of ways. Such a method may comprise isolating the particle from a transformed mesenchymal stem cell (transformed MSC). Such a method may comprise isolating the transformed MSC particle from a transformed mesenchymal stem cell conditioned medium (transformed MSC-CM).

The transformed MSC particle may be isolated for example by being separated from non-associated components based on any property of the transformed MSC particle. For example, the transformed MSC particle may be isolated based on molecular weight, size, shape, composition or biological activity.

The conditioned medium may be filtered or concentrated or both during, prior to or subsequent to separation. For example, it may be filtered through a membrane, for example one with a size or molecular weight cut-off. It may be subject to tangential force filtration or ultrafiltration.

For example, filtration with a membrane of a suitable molecular weight or size cutoff, as described in the Assays for Molecular Weight elsewhere in this document, may be used.

The conditioned medium, optionally filtered or concentrated or both, may be subject to further separation means, such as column chromatography. For example, high performance liquid chromatography (HPLC) with various columns may be used. The columns may be size exclusion columns or binding columns.

One or more properties or biological activities of the transformed MSC particle may be used to track its activity during fractionation of the transformed mesenchymal stem cell conditioned medium (transformed MSC-CM). As an example, light scattering, refractive index, dynamic light scattering or UV-visible detectors may be used to follow the transformed MSC particles. For example, a therapeutic activity such as cardioprotective activity may be used to track the activity during fractionation.

The following paragraphs provide a specific example of how a transformed MSC mesenchymal stem cell particle such as an exosome may be obtained.

A transformed mesenchymal stem cell particle may be produced by culturing transformed mesenchymal stem cells in a medium to condition it. The medium may comprise DMEM. The DMEM may be such that it does not comprise phenol red. The medium may be supplemented with insulin, transferrin, or selenoprotein (ITS), or any combination thereof. It may comprise FGF2. It may comprise PDGF AB. The concentration of FGF2 may be about 5 ng/ml FGF2. The concentration of PDGF AB may be about 5 ng/ml. The medium may comprise glutamine-penicillin-streptomycin or b-mercaptoethanol, or any combination thereof.

The cells may be cultured for about 1, 2, 3, 4, 5, 6, 7, 8, 9, 10 days or more, for example 3 days. The conditioned medium may be obtained by separating the cells from the medium. The conditioned medium may be centrifuged, for example at 500 g. it may be concentrated by filtration through a membrane. The membrane may comprise a > 1000 kDa membrame. The conditioned medium may be concentrated about 50 times or more.

The conditioned medium may be subject to liquid chromatography such as HPLC. The conditioned medium may be separated by size exclusion. Any size exclusion matrix such as Sepharose may be used. As an example, a TSK Guard column SWXL, 6 x 40 mm or a TSK gel G4000 SWXL, 7.8 x 300 mm may be employed. The eluent buffer may comprise any physiological medium such as saline. It may comprise 20 mM phosphate buffer with 150 mM of NaCl at pH 7.2. The chromatography system may be equilibrated at a flow rate of 0.5 ml/min. The elution mode may be isocratic. UV absorbance at 220 nm may be used to track the progress of elution. Fractions may be examined for dynamic light scattering (DLS) using a quasi-elastic light scattering (QELS) detector.

Fractions which are found to exhibit dynamic light scattering may be retained. For example, a fraction which is produced by the general method as described above, and which elutes with a retention time of 11-13 minutes, such as 12 minutes, is found to exhibit dynamic light scattering. The rₕ of particles in this peak is about 55-65 nm. Such fractions comprise transformed MSC mesenchymal stem cell particles such as exosomes.

### DELIVERY OF TRANSFORMED MSC PARTICLES

The transformed MSC particles as described in this document may be delivered to the human or animal body by any suitable means.

We therefore describe a delivery system for delivering a transformed MSC particle as described in this document to a target cell, tissue, organ, animal body or human body, and methods for using the delivery system to deliver transformed MSC particles to a target.

The delivery system may comprise a source of transformed MSC particles, such as a container containing the transformed MSC particles. The delivery system may comprise a dispenser for dispensing the transformed MSC particles to a target.

Accordingly, we provide a delivery system for delivering a transformed MSC particle, comprising a source of transformed MSC particles as described in this document together with a dispenser operable to deliver the transformed MSC particles to a target.

We further provide for the use of such a delivery system in a method of delivering transformed MSC particles to a target.

Delivery systems for delivering fluid into the body are known in the art, and include injection, surgical drips, cathethers (including perfusion cathethers) such as those described in United States Patent 6,139,524, for example, drug delivery catheters such as those described in United States Patent 7,122,019.

Delivery to the lungs or nasal passages, including intranasal delivery, may be achieved using for example a nasal spray, puffer, inhaler, etc as known in the art (for example as shown in United States Design Patent D544,957.

Delivery to the kidneys may be achieved using an intra-aortic renal delivery catheter, such as that described in United States Patent 7,241,273.

It will be evident that the particular delivery should be configurable to deliver the required amount of transformed MSC particles at the appropriate interval, in order to achieve optimal treatment.

The transformed MSC particles may for example be used for the treatment or prevention of atherosclerosis. Here, perfusion of transformed MSC particles may be done intravenously to stabilize atherosclerotic plaques or reduce inflammation in the plaques. The transformed MSC particles may be used for the treatment or prevention of septic shock by intravenous perfusion.

The transformed MSC particles may be used for the treatment or prevention of heart failure. This may be achieved by chronic intracoronary or intramyocardially perfusion of transformed MSC particles to retard remodeling or retard heart failure. The transformed MSC particles may be used for the treatment or prevention of lung inflammation by intranasal delivery.

The transformed MSC particles may be used for the treatment or prevention of dermatological conditions e.g. psoriasis. Long term delivery of transformed MSC particles may be employed using transdermal microinjection needles until the condition is resolved.

It will be evident that the delivery method will depend on the particular organ to which the transformed MSC particles is to be delivered, and the skilled person will be able to determine which means to employ accordingly.

As an example, in the treatment of cardiac inflammation, the transformed MSC particles may be delivered for example to the cardiac tissue (i.e., myocardium, pericardium, or endocardium) by direct intracoronary injection through the chest wall or using standard percutaneous catheter based methods under fluoroscopic guidance for direct injection into tissue such as the myocardium or infusion of an inhibitor from a stent or catheter which is inserted into a bodily lumen.

Any variety of coronary catheter, or a perfusion catheter, may be used to administer the transformed MSC particles. Alternatively the transformed MSC particles may be coated or impregnated on a stent that is placed in a coronary vessel.

### OBTAINING MESENCHYMAL STEM CELLS (MSC)

The transformed mesenchymal stem cells described here may be isolated or produced from mesenchymal stem cells. Mesenchymal stem cells suitable for use in the production of transformed mesenchymal stem cells may be made by any method known in the art.

In particular, MSCs may be made by propagating a cell obtained by dispersing a embryonic stem (ES) cell colony, or a descendent thereof, in the absence of co-culture in a serum free medium comprising FGF2. As a further example, mesenchymal stem cells may be derived from pre-natal tissue. The pre-natal tissue, such as a foetal tissue, may be processed, such as by dissection, mincing or washing, or any combination thereof.

Each of these is described in further detail in the sections below.

### Mesenchymal Stem Cells from Embryonic Stem Cell Sources

The prior art methods of obtaining mesenchymal stem cells (MSC) or MSC-like cells from hESCs involve either transfection of a human telomerase reverse transcriptase (hTERT) gene into differentiating hESCs (Xu et al., 2004) or coculture with mouse OP9 cell line (Barberi et al., 2005). The use of exogenous genetic material and mouse cells in these derivation protocols introduces unacceptable risks of tumorigenicity or infection of xenozootic infectious agents.

The mesenchymal stem cells may be derived by the use of a clinically relevant and reproducible protocol for isolating similar or identical (such as homogenous) MSC populations from differentiating hESCs. In general, the method comprises dispersing a embryonic stem (ES) cell colony into cells. The cells are then plated out and propagated. The cells are propagated in the absence of co-culture in a serum free medium comprising fibroblast growth factor 2 (FGF2), in order to obtain mesenchymal stem cells (MSCs).

Thus, the protocol does not require serum, use of mouse cells or genetic manipulations and requires less manipulations and time, and is therefore highly scalable. Human ES cell derived MSCs (hESC-MSCs) obtained by the methods and compositions described here are remarkably similar to bone-marrow derived MSCs (BM-MSCs).

The embryonic stem cell culture may comprise a human embryonic stem cell (hESC) culture.

In a one embodiment, a method of generating mesenchymal stem cells (MSC) comprises trypsinizing and propagating hESCs without feeder support in media supplemented with FGF2 and optionally PDGF AB before sorting for CD105+CD24- cells.

The method may comprise sorting for CD105+, CD24- cells from trypsinized hESCs one week after feeder-free propagation in a media supplemented with FGF2 and optionally PDGF AB will generate to generate a hESC-MSC cell culture in which at least some, such as substantially all, or all cells are similar or identical (such as homogenous) to each other.

The MSCs produced by this method may be used to produce mesenchymal stem cell conditioned medium (MSC-CM), from which the s may be isolated.

### Disaggregating Embryonic Stem Cell Colonies

One method of producing mesenchymal stem cells may comprise dispersing or disaggregating an embryonic stem cell colony into cells.

The embryonic stem cell colony may comprise a huES9 colony (Cowan CA, Klimanskaya I, McMahon J, Atienza J, Witmyer J, et al. (2004) Derivation of embryonic stem-cell lines from human blastocysts. N Engl J Med 350: 1353-1356) or a H1 ESC colony (Thomson JA, Itskovitz-Eldor J, Shapiro SS, Waknitz MA, Swiergiel JJ, et al. (1998) Embryonic Stem Cell Lines Derived from Human Blastocysts. Science 282: 1145-1147) which are included as reference examples.

The cells in the colony may be disaggregated or dispersed to a substantial extent, i.e., at least into clumps. The colony may be disaggregated or dispersed to the extent that all the cells in the colony are single, i.e., the colony is completely disaggregated.

The disaggregation may be achieved with a dispersing agent.

The dispersing agent may be anything that is capable of detaching at least some embryonic stem cells in a colony from each other. The dispersing agent may comprise a reagent which disrupts the adhesion between cells in a colony, or between cells and a substrate, or both. The dispersing agent may comprise a protease.

The dispersing agent may comprise trypsin. The treatment with trypsin may last for example for 3 minutes or thereabouts at 37 degrees C. The cells may then be neutralised, centrifuged and resuspended in medium before plating out.

The method may comprise dispersing a confluent plate of human embryonic stem cells with trypsin and plating the cells out.

The disaggregation may comprise at least some of the following sequence of steps: aspiration, rinsing, trypsinization, incubation, dislodging, quenching, re-seeding and aliquoting. The following protocol is adapted from the Hedrick Lab, UC San Diego (http://hedricklab.ucsd.edu/Protocol/COSCell.html).

In the aspiration step, the media is aspirated or generally removed from the vessel, such as a flask. In the rinsing step, the cells are rinsed with a volume, for example 5-10 mls, of a buffered medium, which is may be free from Ca²⁺ and Mg²⁺. For example, the cells may be rinsed with calcium and magnesium free PBS. In the trypsinization step, an amount of dispersing agent in buffer is added to the vessel, and the vessel rolled to coat the growing surface with the dispersing agent solution. For example, 1 ml of trypsin in Hank's BSS may be added to a flask.

In the incubation step, the cells are left for some time at a maintained temperature. For example, the cells may be left at 37°C for a few minutes (e.g., 2 to 5 minutes). In the dislodging step, the cells may be dislodged by mechanical action, for example by scraping or by whacking the side of the vessel with a hand. The cells should come off in sheets and slide down the surface.

In the quenching step, a volume of medium is added to the flask. The medium may comprise a neutralising agent to stop the action of the dispersing agent. For example, if the dispersing agent is a protease such as trypsin, the medium may contain a protein, such as a serum protein, which will mop up the activity of the protease. In a particular example, 3 ml of serum containing cell culture medium is added to the flask to make up a total of 4 mls. The cells may be pipetted to dislodge or disperse the cells.

In the re-seeding step, the cells are re-seeded into fresh culture vessels and fresh medium added. A number of re-seedings may be made at different split ratios. For example, the cells may be reseeded at 1/15 dilution and 1/5 dilution. In a particular example, the cells may be re-seeded by adding 1 drop of cells into a 25cm² flask and 3 drops into another to re-seed the culture, and 7-8 mls media is then added to each to provide for 1/15 dilution and 1/5 dilution from for example a 75cm² flask. In the aliquoting step, the cells may be aliquoted into new dishes or whatever split ratio is desired, and media added.

In a specific embodiment, the method includes the following steps: human ES cells are first grown suspended in non-adherent manner to form embryoid bodies (EBs). 5-10 day old EBs are then trypsinized before plating as adherent cells on gelatine coated tissue culture plates.

### Maintenance as Cell Culture

The cells such as the disaggregated cells or the mesenchymal stem cells may be plated and maintained as a cell culture.

The cells may be plated onto a culture vessel or substrate such as a gelatinized plate. Crucially, the cells are grown and propagated without the presence of co-culture, e.g., in the absence of feeder cells.

The cells in the cell culture may be grown in a serum-free medium. The medium may be supplemented by one or more growth factors such as epidermal growth factor (EGF), fibroblast growth factor 2 (FGF2) and optionally platelet-derived growth factor AB (PDGF AB), at for example 5ng/ml, 10ng/ml, 15ng/ml, 20ng/ml, 25ng/ml or 30ng/ml. The cells in the cell culture may be split or subcultured when confluent. They may be split by any suitable means, such as treatment with trypsin, washing and replating. The split ratio may comprise for example 1:4.

The mesenchymal stem cells may be maintained as a cell line.

### Absence of Co-Culture

In some embodiments, our methods involve culturing cells in the absence of co-culture.

The term 'co-culture' refers to a mixture of two or more different kinds of cells that are grown together, for example, stromal feeder cells.

Thus, in typical ES cell culture, the inner surface of the culture dish is usually coated with a feeder layer of mouse embryonic skin cells that have been treated so they will not divide. The feeder layer provides an adherent surface to enable the ES cells to attach and grow. In addition, the feeder cells release nutrients into the culture medium which are required for ES cell growth. In the methods and compositions described here, the ES and MSC cells may be cultured in the absence of such co-culture.

The cells may be cultured as a monolayer or in the absence of feeder cells. The embryonic stem cells may be cultured in the absence of feeder cells to establish mesenchymal stem cells (MSC). The MSCs themselves may be cultured in the absence of feeder cells.

The dissociated or disaggregated embryonic stem cells may be plated directly onto a culture substrate. The mesenchymal stem cells may be cultured on a culture substrate. The culture substrate may comprise a tissue culture vessel, such as a Petri dish. The vessel may be pre-treated. The cells may be plated onto, and grow on, a gelatinised tissue culture plate.

An example protocol for the gelatin coating of dishes follows. A solution of 0.1% gelatin in distilled water is made and autoclaved. This may be stored at room temp. The bottom of a tissue culture dish is covered with the gelatin solution and incubated for 5-15min. Remove gelatin and plates are ready to use. Medium should be added before adding cells to prevent hypotonic lysis.

### Serum Free Media

The dissociated or disaggregated embryonic stem cells may be cultured in a medium which may comprise a serum-free medium. The mesenchymal stem cells may be cultured in a medium which may comprise a serum-free medium.

The term 'serum-free media' may comprise cell culture media which is free of serum proteins, e.g., fetal calf serum. Serum-free media are known in the art, and are described for example in US Patents 5,631,159 and 5,661,034. Serum-free media are commercially available from, for example, Gibco-BRL (Invitrogen).

The serum-free media may be protein free, in that it may lack proteins, hydrolysates, and components of unknown composition. The serum-free media may comprise chemically-defined media in which all components have a known chemical structure. Chemically-defined serum-free media is advantageous as it provides a completely defined system which eliminates variability allows for improved reproducibility and more consistent performance, and decreases possibility of contamination by adventitious agents.

The serum-free media may comprise Knockout DMEM media (Invitrogen-Gibco, Grand Island, New York).

The serum-free media may be supplemented with one or more components, such as serum replacement media, at a concentration of for example, 5%, 10%, 15%, etc. The serum-free media may comprise or be supplemented with 10% serum replacement media from Invitrogen- Gibco (Grand Island, New York).

### Growth Factor

The serum-free medium in which the dissociated or disaggregated embryonic stem cells are cultured may comprise one or more growth factors. The serum-free medium in mesenchymal stem cells are cultured may comprise one or more growth factors. A number of growth factors are known in the art, including PDGF, EGF, TGF-a, FGF, NGF, Erythropoietin, TGF-b, IGF-I and IGF-II.

The growth factor may comprise fibroblast growth factor 2 (FGF2). The medium may also contain other growth factors such as platelet-derived growth factor AB (PDGF AB). Both of these growth factors are known in the art. The method may comprise culturing cells in a medium comprising both FGF2 and PDGF AB.

Alternatively, or in addition, the medium may comprise or further comprise epidermal growth factor (EGF). Use of EGF may enhance growth of MSCs. EGF may be used at any suitable concentration, for example 5-10 ng/ml EGF. EGF may be used in place of PDGF. EGF is a protein well known in the art, and is referred to as symbol EGF, Alt. Symbols URG, Entrez 1950, HUGO 3229, OMIM 131530, RefSeq NM_001963, UniProt P01133.

Thus, we disclose the use of media comprising (i) FGF2, (ii) FGF2 and PDGF and (iii) FGF2 and EGF and other combinations.

FGF2 is a wide-spectrum mitogenic, angiogenic, and neurotrophic factor that is expressed at low levels in many tissues and cell types and reaches high concentrations in brain and pituitary. FGF2 has been implicated in a multitude of physiologic and pathologic processes, including limb development, angiogenesis, wound healing, and tumor growth. FGF2 may be obtained commercially, for example from Invitrogen-Gibco (Grand Island, New York).

Platelet Derived Growth Factor (PDGF) is a potent mitogen for a wide range of cell types including fibroblasts, smooth muscle and connective tissue. PDGF, which is composed of a dimer of two chains termed the A chain and B chain, can be present as AA or BB homodimers or as an AB heterodimer. Human PDGF-AB is a 25.5 kDa homodimer protein consisting of 13.3 kDa A chain and 12.2 B chain. PDGF AB may be obtained commercially, for example from Peprotech (Rocky Hill, New Jersey).

Epidermal growth factor or EGF is a growth factor that plays an important role in the regulation of cell growth, proliferation, and differentiation by binding to its receptor EGFR. Human EGF is a 6045-Da protein with 53 amino acid residues and three intramolecular disulfide bonds. EGF acts by binding with high affinity to epidermal growth factor receptor (EGFR) on the cell surface and stimulating the intrinsic protein-tyrosine kinase activity of the receptor. The tyrosine kinase activity, in turn, initiates a signal transduction cascade that results in a variety of biochemical changes within the cell - a rise in intracellular calcium levels, increased glycolysis and protein synthesis, and increases in the expression of certain genes including the gene for EGFR - that ultimately lead to DNA synthesis and cell proliferation. EGF may be obtained commercially from a number of manufacturers.

The growth factor(s), such as FGF2 and optionally PDGF AB, may be present in the medium at concentrations of about 100pg/ml, such as about 500pg/ml, such as about 1ng/ml, such as about 2ng/ml, such as about 3ng/ml, such as about 4ng/ml, such as about 5ng/ml. In some embodiments, the medium contains FGF2 at about 5ng/ml. The medium may also contain PDGF AB, such as at about 5ng/ml.

### Splitting Cells

Cells in culture will generally continue growing until confluence, when contact inhibition causes cessation of cell division and growth. Such cells may then be dissociated from the substrate or flask, and 'split', subcultured or passaged, by dilution into tissue culture medium and replating.

The methods and compositions described here may therefore comprise passaging, or splitting during culture. The cells in the cell culture may be split at a ratio of 1:2 or more, such as 1:3, such as 1:4, 1:5 or more. The term 'passage' designates the process consisting in taking an aliquot of a confluent culture of a cell line, in inoculating into fresh medium, and in culturing the line until confluence or saturation is obtained.

### Selection, Screening or Sorting Step

The method may further comprise a selection or sorting step, to further isolate or select for mesenchymal stem cells.

The selection or sorting step may comprise selecting mesenchymal stem cells (MSC) from the cell culture by means of one or more surface antigen markers. The use of a selection or sorting step further enhances the stringency of sorting and selection specificity for MSCs and furthermore potentially reduces possible contamination from embryonic stem cells such as hESCs and other hESC-derivatives from the starting material. This would then further reduce the risk of teratoma formation and further increase the clinical relevance of the protocol we describe.

A number of methods are known for selection or sorting based on antigen expression, and any of these may be used in the selection or sorting step described here. The selection or sorting may be achieved by means of fluorescence activated cell sorting (FACS). Thus, as known in the art, FACS involves exposing cells to a reporter, such as a labelled antibody, which binds to and labels antigens expressed by the cell. Methods of production of antibodies and labelling thereof to form reporters are known in the art, and described for example in Harlow and Lane. The cells are then passed through a FACS machine, which sorts the cells from each other based on the labelling. Alternatively or in addition, magnetic cell sorting (MACS) may be employed to sort the cells.

We have realised that while a number of candidate surface antigens known to be associated with MSCs e.g. CD105, CD73, ANPEP, ITGA4 (CD49d), PDGFRA, some of the MSC associated surface antigens e.g. CD29 and CD49e are also highly expressed in ES cells such as hESCs and their expression are verified by FACS analysis. The association of a surface antigen with MSCs may not be sufficient to qualify the antigen as a selectable marker for isolating MSCs from ES cells such as hESC. Accordingly, the selection or sorting step may employ antigens which are differentially expressed between MSCs and ES cells.

The selection or sorting step of our method may positively select for mesenchymal stem cells based on the expression of antigens. Such antigens may be identified by, for example, comparing the gene expression profiles of hESCs and hESCMSCs. In particular embodiments, the selection or sorting may specifically make use of any of the antigens shown in Table E1A and E1B below.

The selection or sorting step of our method may positively select for mesenchymal stem cells based on the expression of antigens which are identified as expressed on MSCs, but not expressed on ES cells such as hESCs.

CD73 is highly expressed on MSCs, while being not highly expressed on hESCs. Both CD73 and CD105 are highly expressed surface antigens in MSCs and are among the top 20 highly expressed surface antigens in hESC-MSCs relative to hESC, the use of either CD73 or CD105 (or both) as selectable marker for putative MSCs will be equally effective in sorting for putative MSCs generated by differentiating hESCs.

Alternatively, or in addition, the selection or sorting step may negatively select against antigens based on surface antigens that are highly expressed as surface antigen on embryonic stem cells (ES cells) such as hESCs, and not mesenchymal stem cells e.g., hESC-MSC. Selection or sorting may be based on known or previously identified hESC-specific surface antigens such as MIBP, ITGB1BP3 and PODXL, and CD24.

FACS analysis confirms the expression of CD24 on hESC but not hESC-MSCs. Therefore, CD24 may be used as a negative selection or sorting marker either on its own, or in conjunction with CD105 as a positive selectable marker for isolating putative MSCs from differentiating hESC cultures.

### Reference Examples: Mesenchymal Stem Cells from Pre-Natal Sources

The mesenchymal stem cells, from which the transformed mesenchymal stem cells may be made, may be derived from pre-natal tissue. The pre-natal tissue, such as a foetal tissue, may be processed, such as by dissection, mincing or washing, or any combination thereof.

The mesenchymal stem cell may be derived from such a pre-natal tissue based on any suitable property, such as preferential adhesion. For example, the mesenchymal stem cell may be selected based on its ability to adhere to a substrate. The substrate may for example comprise plastic. Accordingly, the mesenchymal stem cell may be derived from pre-natal tissue by allowing the mesenchymal stem cells in the pre-natal tissue mass to adhere to plastic. For this purpose, the tissue may be placed on a vessel with one or more plastic surfaces. Such a vessel may comprise a culture vessel, for example a tissue culture plate. The vessel may be gelatinised. The mesenchymal stem cells may be allowed to migrate out of the tissue mass. They may be allowed to adhere to the surface of the vessel. The rest of the pre-natal tissue may be removed, such as by washing off. Thus, the bulk of the tissue pieces may then be washed off, leaving a homogenous cell culture.

The tissue may be cultured in any suitable medium, such as Dulbecco's Modified Eagle Medium. The medium may be supplemented with any suitable supplement, such as serum replacement medium, EGF, FGF2, etc. The serum replacement medium, where present, may be included at any suitable concentration such as 10%. EGF, where present, may be included at any suitable concentration such as 5, 10, 15 or 20 ng/ml. FGF2, where present, may be included at any suitable concentration such as 5, 10, 15 or 20 ng/ml.

A specific protocol which may be used for deriving pre-natal mesenchymal stem cells may comprise the following. The cultures may be cultured in either serum-free or serum-containing culture medium. When confluent, the cultures may be passaged for example by trypsinizing and then splitting such as at 1:4 on a gelatinized tissue culture plate. Serum-free culture medium may be made up of Knockout DMEM medium supplemented with 10% serum replacement media, non-essential amino acids, 10 ng/ml FGF2, 10ng/ml Recombinant Human EGF and 55µM β-mercaptoethanol. Serum-containing culture medium may be made up of DMEM- high glucose without glutamine supplemented with penicillin-streptomycin, L-glutamine, non-essential amino acids and 10% fetal calf serum. Instead of, or in addition to EGF, PDGF may be used.

The culture may comprise or be established in the absence of co-culture, such as in the absence of feeder cells. For this purpose, the mesenchymal stem cell may be cultured without a feeder cell layer. This is described in further detail below.

The mesenchymal stem cell may be derived by optionally selecting a mesenchymal stem cell from other cells based on expression of a cell surface marker, as described in further detail below. The cell may therefore optionally be selected by detecting elevated expression of for example CD 105 (Accession Number NM_000118.1) or CD73 (Accession Number NM_002526.1), or both. The cell may be further optionally selected by detecting a reduced expression of CD24 (Accession Number NM_013230.1). Thus, the mesenchymal stem cell may be obtained by selecting for cells which are CD105+ CD24-. The mesenchymal stem cell may be selected by labelling the cell with an antibody against the appropriate surface antigen and may be selected by fluorescence activated cell sorting (FACS) or magnetic cell sorting (MACS).

### CHARACTERISTICS OF TRANSFORMED MESENCHYMAL STEM CELLS

The transformed mesenchymal stem cells obtained by the methods and compositions described here may display one or more properties or characteristics of mesenchymal stem cells.

They may satisfy any one or more of the morphologic, phenotypic and functional criteria commonly used to identify mesenchymal stem cells⁹, as known in the art. The properties or characteristics may be as defined by The International Society for Cellular Therapy. In particular, they may display one or more characteristics as set out in Dominici et al (2006).

### Morphology

The transformed mesenchymal stem cells obtained by the methods and compositions described here may exhibit one or more morphological characteristics of mesenchymal stem cells.

The transformed mesenchymal stem cells obtained by the methods described here may display a typical fingerprint whorl at confluency.

The transformed mesenchymal stem cells obtained may form an adherent monolayer with a fibroblastic phenotype. The transformed mesenchymal stem cell may be capable of adhering to plastic.

They may have an average population doubling time of between 72 to 96 hours. The optimal culture may be at 25% to 85% confluency or 15-50,000 cells per cm².

### Antigen Profile

Furthermore, the transformed mesenchymal stem cells obtained may display a surface antigen profile which is similar or identical to mesenchymal stem cells.

The transformed mesenchymal stem cells obtained by the methods described here may lack or display reduced expression of one or more pluripotency marker, such as of Oct-4, SSEA-4 and TRa1-60, for example at the polypeptide level. They may display transcript expression of one or both of OCT4 and SOX2, but at reduced levels compared to embryonic stem cells such as hES3 human ESCs. The levels of expression may be 2 times lower, 5 times lower or 10 times lower or more compared to embryonic stem cells.

The obtained transformed mesenchymal stem cells may display a 'typical' MSC-like surface antigen profile. They may for example show expression of one or more markers associated with mesenchymal stem cells. These may include expression of any one or more of the following: CD29, CD44, CD49a, CD49e, CD105, CD166, MHC I. The transformed mesenchymal stem cells may for example show reduced or absent expression of any one or more markers whose absence of expression is associated with mesenchymal stem cells. Thus, the transformed mesenchymal stem cells may display reduced or lack of expression of any one or more of HLA-DR, CD34 and CD45. The transformed mesenchymal stem cells may in particular comprise CD29+, CD44+, CD49a+ CD49e+, CD105+, CD166+, MHC I+, CD34⁻and CD45⁻ cells.

The transformed mesenchymal stem cell may be negative for mouse-specific *c-mos* repeat sequences and positive for human specific *alu* repeat sequences. It may be capable of undergoing any one or more of osteogenesis, adipogenesis or chondrogenesis, and in particular it may be capable of differentiating into any one or more of osteocytes, adipocytes or chondrocytes.

### Differentiation Potential

The transformed mesenchymal stem cells obtained may be differentiated into any mesenchymal lineage, using methods known in the art and described below. Thus, the transformed mesenchymal stem cells obtained by the methods and compositions described here may display a differentiation potential that include adipogenesis, chondrogenesis and osteogenesis ⁹.

### Proliferative Capacity

The transformed mesenchymal stem cells obtained as described can have a substantial proliferative capacity *in vitro.* In some embodiments, the transformed mesenchymal stem cells obtained may undergo at least 10 population doublings while maintaining a normal diploid karyotype. The transformed mesenchymal stem cells may be capable of undergoing at least 20-30 population doublings while maintaining a normal diploid karyotype. In some embodiments, the transformed mesenchymal stem cells display a stable gene expression and surface antigen profile throughout this time.

The transformed mesenchymal stem cells obtained may be such that they do not display any defects, such as chromosomal aberrations and/or alterations in gene expression. In some embodiments, such defects are not evident until after 10 passages, such as after 13 passages, for example after 15 passages.

### Homogeneity

The transformed mesenchymal stem cells obtained may display a high degree of uniformity. In other words, the transformed mesenchymal stem cells obtained from different sources may display one or more, such as a plurality, of uniform or distinct characteristics that are shared with each other.

They may display one or more, such as a plurality, of uniform or distinct characteristics that are shared with other mesenchymal stem cells, such as a human embryonic stem cell derived mesenchymal stem cells (hESC-MSCs), as for example described in WO2007/027157 or WO2007/027156.

The transformed mesenchymal stem cells produced by the method described here may be similar or identical (such as homogenous) in nature. That is to say, transformed mesenchymal stem cell clones isolated by the protocol may show a high degree of similarity or identity with each other, whether phenotypically or otherwise.

Similarity or identity may be gauged by a number of ways and measured by one or more characteristics. For example, the clones may be similar or identical in gene expression. For example, the transformed mesenchymal stem cells may be such that any two or more, such as all, transformed mesenchymal stem cells produced by the method exhibit substantially similar or identical gene expression profiles, that is to say, a combination of the identity of genes expressed and the level to which they are expressed.

The gene expression correlation coefficient between any two or more isolates of transformed mesenchymal stem cells obtained by the method may be greater than 0.8, such as greater than 0.85 or 0.9. The gene expression correlation coefficient between any two or more different passages, such as successive passages, of transformed mesenchymal stem cells obtained by the method may be greater than 0.8, such as greater than 0.85 or 0.9. The gene expression correlation coefficient between a transformed mesenchymal stem cell obtained by the method and hESC-MSCs, such as for example described in WO2007/027157 or WO2007/027156, may be greater than 0.8, such as greater than 0.85 or 0.9.

Homogeneity of gene expression may be measured by a number of methods. Genome-wide gene profiling may be conducted using, for example, array hybridisation of extracted RNA as described in the Examples. Total RNA may be extracted and converted into cDNA, which is hybridised to an array chip comprising a plurality of gene sequences from a relevant genome. The array may comprise NCBI Reference Sequence (RefSeq) genes, which are well characterised genes validated, annotated and curated on an ongoing basis by National Center for Biotechnology Information (NCBI) staff and collaborators.

Gene expression between samples may then be compared using analysis software. In one embodiment, the similarity or identity of gene expression may be expressed as a 'correlation coefficient'. In such measures, a high correlation coefficient between two samples indicates a high degree of similarity between the pattern of gene expression in the two samples. Conversely, a low correlation coefficient between two samples indicates a low degree of similarity between the pattern of gene expression in the two samples. Normalisation may be conducted to remove systematic variations or bias (including intensity bias, spatial bias, plate bias and background bias) prior to data analysis.

Correlation tests are known in the art and include a T-test and Pearson's test, as described in for example Hill, T. & Lewicki, P. (2006). Statistics: Methods and Applications. StatSoft, Tulsa, OK, ISBN: 1884233597 (also StatSoft, Inc. (2006). Electronic Statistics Textbook. Tulsa, OK: StatSoft. WEB: http://www.statsoft.com/textbook/stathome.html). Reference is made to Khojasteh et al., 2005, A stepwise framework for the normalization of array CGH data, BMC Bioinformatics 2005, 6:274. An Intra-class correlation coefficient (ICC) may also be conducted, as described in Khojasteh et al, *supra*.

For example, a Pearson's test may be conducted to generate a Pearson's correlation coefficient. A correlation coefficient of 1.0 indicates an identical gene expression pattern.

For such purposes, the cDNA may be hybridised to a Sentrix HumanRef-8 Expression BeadChip and scanned using a Illumina BeadStation 500x. The data may be extracted, normalised and analysed using Illumina BeadStudio (Illumina, Inc, San Diego, CA, USA). It will be clear to the reader however that any suitable chip and scanning hardware and software (which outputs a correlation measurement) may be used to assay similarity of gene expression profile.

The gene expression correlation coefficient between any two isolates as for example measured by the above means may be 0.65 or more. The gene expression correlation coefficient may be 0.70 or more, such as 0.80 or more, such as 0.85 or more, such as 0.90 or more. The coefficient may be 0.91 or more, 0.92 or more, 0.93 or more, 0.94 or more, 0.95 or more, 0.96 or more, 0.97 or more, 0.98 or more, 0.99 or more or 1.0.

In some embodiments, the method described here generates transformed mesenchymal stem cells whose gene expression correlation coefficient between any two or more isolates of transformed mesenchymal stem cells so obtained is in the same order as, or slightly less than, the correlation coefficient between technical replicates of the same RNA sample, performed a period of time apart such as 1 month apart. For example, the gene expression correlation coefficient between any two or more isolates of transformed mesenchymal stem cells may be 0.70 or more, such as 0.80 or more, such as 0.85 or more, such as 0.90 or more. The coefficient may be 0.91 or more, 0.92 or more, 0.93 or more, 0.94 or more, 0.95 or more, 0.96 or more, 0.97 or more, 0.98 or more, 0.99 or more or 1.0.

The gene expression correlation coefficients may be in such ranges for cells which have undergone the derivation, selection or sorting procedure described above. The gene expression correlation coefficient between the majority of isolates, such as all isolates, may be in such ranges.

Accordingly, we provide for a method of generating transformed mesenchymal stem cells which are substantially similar or identical (such as homogenous) with each other. The isolates may display a near identical gene expression profile.

As well as the 'internal' homogeneity described above (i.e., homogeneity between the isolates of transformed mesenchymal stem cells from the method), homogeneity may also be assessed between such isolates and other cells or cell types. In particular, comparisons may be made with mesenchymal stem cells derived by other methods, such as a human ESC-MSC culture (reference examples are described in WO2007/027157 or WO2007/027156). In some embodiments, therefore, the transformed mesenchymal stem cells obtained by the methods and compositions described here display a gene expression profile which is similar to, homogenous with, or identical with such a human ESC-MSC culture. Thus, the transformed mesenchymal stem cells obtained may show a correlation coefficient of gene expression of greater than 0.5, such as greater than 0.6, such as greater than 0.7, such as greater than 0.8, such as greater than 0.9, as with such a human ESC-MSC culture (reference examples are described in WO2007/027157 or WO2007/027156).

### Telomerase Activity

The transformed mesenchymal stem cells so derived may comprise telomerase activity. The telomerase activity may be elevated or up-regulated compared to a control cell such as a cell which is not a mesenchymal stem cell. For example, the control cell may comprise a differentiated cell, such as a differentiated cell in the mesenchymal lineage, for example, an osteocyte, adipocyte or chondrocyte.

Telomerase activity may be determined by means known in the art, for example, using TRAP activity assay (Kim et al., Science 266:2011, 1997), using a commercially available kit (TRAPeze.RTM. XK Telomerase Detection Kit, Cat. s7707; Intergen Co., Purchase N.Y.; or TeloTAGGG.TM. Telomerase PCR ELISA plus, Cat. 2,013,89; Roche Diagnostics, Indianapolis). hTERT expression can also be evaluated at the mRNA level by RT-PCR. The LightCycler TeloTAGGG.TM. hTERT quantification kit (Cat. 3,012,344; Roche Diagnostics) is available commercially for research purposes.

For example, relative telomerase activity may be measured by real time quantitative telomeric repeat amplification protocol. This qPCR-based assay quantifies product generated *in vitro* by telomerase activity present in the samples. The relative telomerase activity which is directly proportional to the amount of telomerase products may be assessed by the threshold cycle number (or Ct value) for one µg protein cell lysate. Hues9.E1 refers to a previously described hESC-MSCs line (and is included as a reference example) and HEK is a human embryonic kidney cell line. The Ct value for each fetal MSCs may be taken as a mean for multiple passages, for example, 2, 3, 4 etc passages. The passages may for example comprise three passages, such as P16, P18, and P20. For the control cells, such as Hues9.E1 as a reference example, the mean may be for two passages, such as P20 and P22. The assay may be performed multiple times, such as in triplicate, for each passage.

A specific example of a telomerase detection method is provided in the Examples.

The mesenchymal stem cells derived by the methods described here may have a Ct value, as assayed by such a method, of 25 or more, such as 26 or more or 27 or more. The Ct value may be for example 28 or more, 29 or more, 30 or more, 31 or more or 32 or more.

### Maintenance of Self-Renewal

The transformed mesenchymal stem cells may be able to maintain self-renewal.

The transformed mesenchymal stem cells and cell lines (or the differentiated cells derived from them) may be such that they do not display one or more characteristics of embryonic stem cells. Such characteristics may include expression of the *OCT4* gene, the SSEA-4 gene, the Tra1-60 gene and alkaline phosphatase activity. The mesenchymal stem cell may exhibit reduced expression of one or more characteristic markers of pluripotency. Such pluripotency markers may include *Nanog, BMP4, FGF5, Oct4, Sox-2* and *Utf1*, etc

Transformed mesenchymal stem cells made by the methods described here may be non-tumorigenic or non-teratogenic or both.

Accordingly, the transformed mesenchymal stem cells may be such that they do not substantially induce formation of teratoma when transplanted to a recipient animal. The transformed mesenchymal stem cells may be such that when implanted into an immune compromised or immunodeficient host animal do not result in tumours.

The recipient animal may comprise an immune compromised recipient animal. The immune compromised or immunodeficient host animal may comprise a SCID mouse or a Rag1 -/- mouse. The cell may be such that a recipient animal, into which a transformed mesenchymal stem cell is transformed, does not show teratoma formation for a suitable period of time such as 3 weeks, or 2 to 9 months, following implantation. The transformed mesenchymal stem cells may be such that they do not form tumours after prolonged periods of implantation, such as greater than 2 weeks, for example greater than 2 months, such as greater than 9 months.

A detailed protocol for tumourigenicity testing follows.

1×10⁶ embryonic stem cells are transplanted subcutaneously into SCID mice. At three weeks when embryonic stem cell-derived tumors are about 1 cm in diameter, transformed mesenchymal stem cells labeled with Qdot® nanocrystals (655 nm emission) using a Qtracker® Cell Labeling Kit (Quantum Dot Corp, Hayward, CA) are injected into the embryonic stem cell-derived teratoma.

Three days later, the mice are euthanized with an overdose of anesthesia and the tumors are removed. The tumors are fixed in 4% paraformaldehyde and cryosectioned at 20 µm thickness. The sections are assayed for pecam-1 immunoreactivity using rat anit-pecam1 (Pharmingen, San Diego, California) followed by FITC-conjugated rabbit anti-rat antibody (Chemicon, Temecula, California), and counterstained with DAPI. The sections are analyzed by confocal microscopy.

Transformed mesenchymal stem cells made by the methods described here are may also display one or more of the following characteristics. The transformed mesenchymal stem cells may be maintainable in cell culture for a prolonged period, such as greater than 40 generations. They may have a substantially stable karyotype as assessed by chromosome number, such when maintained in cell culture for at least 10 generations. They also may display a substantially stable gene expression pattern from generation to generation. By this we mean that the expression levels one or more, such as substantially all, of a chosen set of genes does not vary significantly between a transformed mesenchymal stem cells in one generation and transformed mesenchymal stem cells in the next generation.

The set of genes may comprise one or more, a subset, or all of, the following: cerberus (GenBank Accession nos: NM_009887, AF031896, AF035579), FABP (GenBank Accession nos: NM_007980, M65034, AY523818, AY523819), Foxa2 (GenBank Accession nos: NM_010446, X74937, L10409), Gata-1 (GenBank Accession nos: NM_008089, X15763, BC052653), Gata-4 (GenBank Accession nos: NM_008092, AF179424, U85046, M98339, AB075549), Hesx1 (GenBank Accession nos: NM_010420, X80040, U40720, AK082831), HNF4a (GenBank Accession nos: NM_008261, D29015, BC039220), c-kit (GenBank Accession nos: NM_021099, Y00864, AY536430, BC075716, AK047010, BC026713, BC052457, AK046795), PDGFRα (NM_011058, M57683, M84607, BC053036), Oct4 (GenBank Accession nos: NM_013633, X52437, M34381, BC068268), Runx1 (GenBank Accession nos: NM_009821, D26532, BC069929, AK051758), Sox17 (GenBank Accession nos: NM_011441, D49474, L29085, AK004781), Sox2 (GenBank Accession nos: NM_011443, U31967, AB108673), Brachyury (NCM_009309, X51683), TDGF1 (GenBank Accession nos: NM_011562, M87321) and Tie-2 (GenBank Accession nos: NM_013690, X67553, X71426, D13738, BC050824).

The methods described here enable the production of transformed mesenchymal stem cells as well as differentiated cells, which comprise clonal descendants of transformed mesenchymal stem cells. The term 'clonal descendant' of a cell refers to descendants of the cells which have not undergone substantially any transforming treatment or genetic alteration. Such clonal descendants have not undergone substantial genomic changes are substantially genetically identical to the parent cell, or an ancestor, such as the transformed mesenchymal stem cell. The term 'transformed mesenchymal stem cells' should also be taken to include cell lines derived from transformed mesenchymal stem cells, i.e., transformed mesenchymal stem cell lines, and *vice versa.*

### Long-Term Maintenance in Culture

The transformed mesenchymal stem cells or their descendants may be cultured for more than one generation.

For example, the cells may be cultured for more than 5, more than 10, more than 15, more than 20, more than 25, more than 50, more than 40, more than 45, more than 50, more than 100, more than 200, more than 500 or more than 800 generations. In particular, the cell lines may be maintained for 1, 2, 3, 4, 5, 6, 7, 8, 9, 10, 11, 12, 13, 14, 15, 20, 25, 30, 35, 40, 45, 50, 75, 100, 200, 500 or more generations.

Cells in culture will generally continue growing until confluence, when contact inhibition causes cessation of cell division and growth. Such cells may then be dissociated from the substrate or flask, and 'split' or passaged, by dilution into tissue culture medium and replating. The cultured cells may therefore be passaged, or split during culture. They may be split at a ratio of 1:2 or more, such as 1:3 or 1:4, 1:5 or more. The term 'passage' designates the process consisting in taking an aliquot of a confluent culture of a cell line, in inoculating into fresh medium, and in culturing the line until confluence or saturation is obtained.

The transformed mesenchymal stem cells derived according to the methods described here may however be maintained for a large number of generations. On the other hand, it has been established that 'normal' (i.e., untransformed somatic) cells derived directly from an organism are not immortal. In other words, such somatic cells have a limited life span in culture (they are mortal). They will not continue growing indefinitely, but will ultimately lose the ability to proliferate or divide after a certain number of generations. On reaching a 'crisis phase' such cells die after about 50 generations. Thus, such somatic cells may only be passaged a limited number of times.

According to the methods described here, transformed mesenchymal stem cells may be propagated for more than 50 generations. In some embodiments, the transformed mesenchymal stem cells may be propagated indefinitely as transformed mesenchymal stem cell lines. The transformed mesenchymal stem cells and transformed mesenchymal stem cells lines may be lineage restricted. In particular, they may be such that they are not capable of giving rise to all three germ layers. In some embodiments, the transformed mesenchymal stem cells lines may be non-pluripotent.

We further provide a composition comprising a plurality of cells, wherein a majority of the cells are mesenchymal stem cells. For example, at least 60% of the cells may comprise mesenchymal stem cells. In addition, we provide an isolated mesenchymal stem cell. The term cell line may be taken to refer to cells that can be maintained and grown in culture and display an immortal or indefinite life span.

### CARDIOPROTECTIVE ABILITY

The transformed mesenchymal stem cell so derived or a medium conditioned by such a cell may comprise cardioprotective ability, as described in the Examples. The cardioprotection may comprise restoration or maintenance of cardiac function during ischemia and/or reperfusion.

Cardioprotection may be assayed in any suitable model system, such as a mouse model of acute myocardial infarction (AMI). In such an assay, AMI is induced in mice by permanent ligation of the left anterior descending coronary artery as described in Salto-Tellez M, Yung Lim S, El-Oakley RM, Tang TP, ZA AL, et al. (2004) Myocardial infarction in the C57BL/6J mouse: a quantifiable and highly reproducible experimental model. Cardiovasc Pathol 13: 91-97.

100 µl of 10 X concentrated conditioned medium or non-conditioned medium (control) made as described above is then administered to the mice via an osmotic pump placed at the jugular vein over the next 72 hours. Heart function in these mice is assessed by MRI three weeks later.

Cardioprotection may also be assayed in a pig/mouse model of myocardial ischemia/reperfusion (MI/R) injury (Timmers L, Lim S-K, Arslan F, et al. Reduction of myocardial infarct size by human mesenchymal stem cell conditioned medium. Stem Cell Research. 2008;1:129-137). In this assay, injury is induced by a temporary occlusion of the left circumflex artery in pig or the LAD in mouse followed by removal of occlusion to initiate reperfusion.

The cardioprotection assays described above may also be used to test for cardioprotection especially in chronic ischemia. This and the other MI/R injury model essentially evaluate cardioprotection in different clinical indications.

The transformed mesenchymal stem cell so derived or a medium conditioned by such a cell or a particle isolated therefrom may be capable of alleviating reperfusion injury. This may be assayed using a porcine model of ischemia-reperfusion as described in WO2008/020815.

A brief protocol for assaying cardioprotective ability of mesenchymal stem cell (including transformed mesenchymal stem cell) conditioned medium follows. MI may be induced by 30 minutes occlusion of left coronary artery (LCA) by ligating the artery with a suture. Subsequent reperfusion may be initiated by releasing the suture. Five minutes before reperfusion, mice may be intravenously infused with 200 µl saline diluted conditioned medium containing 3 µg protein for Hues9.E1 (hESC-MSC, used as a reference only) CM or 150 µg protein for fetal MSC CM via the tail vein:,Control animals may be infused with 200 µl saline. After 24 hours reperfusion, infarct size (IS) as a percentage of the area at risk (AAR) may be assessed using Evans' blue dye injection and TTC staining as described previously in reference 26.

Briefly, just before excision of the heart for analysis, the LCA may be re-ligated as in the induction of ischemia, Evans blue dye may be infused into the aorta and the AAR may be defined by the area not stained by Evans' blue dye. The heart may then be excised and cross sections of the heart may be stained with TTC. Viable myocardium is stained red by TTC while non-viable myocardium is not stained. Relative infarct size may be measured as the area of non-viable myocardium not stained by TTC relative to the AAR risk defined by the area not stained by Evans' blue dye.

### Infarct Size

The transformed mesenchymal stem cell so derived or a medium conditioned by such a cell may have the ability to reduce infarct size. The infarct size may be reduced by 10% or more, 20% or more, 30% or more, 40% or more, 50% or more, 60% or more or 70% or more compared with an animal that is treated with a non-conditioned medium or saline.

### Assay for Infarct Size

Infarct size may for example be assayed using the following method.

Just prior to excision of the heart, the LCxCA (pigs) or LCA (mice) is religated at exactly the same spot as for the induction of the MI. Evans blue dye is infused through the coronary system to delineate the area at risk (AAR).

The heart is then excised, the LV is isolated and cut into 5 slices from apex to base. The slices are incubated in 1% triphenyltetrazolium chloride (TTC, Sigma-Aldrich Chemicals, Zwijndrecht, the Netherlands) in 37°C Sörensen buffer (13.6 g/L KH₂PO₄ + 17.8 g/L Na₂H PO₄ · 2H₂O, pH 7.4) for 15 minutes to discriminate infarct tissue from viable myocardium.

All slices are scanned from both sides, and in each slide, the infarct area is compared with area at risk and the total area by use of digital planimetry software (Image J). After correction for the weight of the slices, infarct size is calculated as a percentage of the AAR and of the LV.

### Oxidative Stress

The transformed mesenchymal stem cells produced by the method described here may be capable of reducing oxidative stress.

The oxidative stress may be reduced by 10% or more, 20% or more, 25% or more, 30% or more, 40% or more, 50% or more, 60% or more or 70% or more compared with an animal that is treated with a non-conditioned medium or saline.

The reduction of oxidative stress may be assayed in an *in vitro* assay of hydrogen peroxide (H₂O₂)-induced cell death.

### Assay for Oxidative Stress

The reduction of oxidative stress may for example be assayed using an *in vitro* assay of hydrogen peroxide (H₂O₂)-induced cell death. In summary, hydrogen peroxide (H₂O₂)-mediated oxidative stress is induced in human leukemic CEM cells and cell viability is monitored by Trypan blue-exclusion. Human leukemic CEM cells are incubated with conditioned medium or transformed mesenchymal stem cell (with saline as a control) and treated with 50 µM H₂O₂ to induce oxidative stress. Cell viability is assessed using Trypan Blue exclusion at 12, 24, 36 and 48 hours after H₂O₂ treatment.

The reduction of oxidative stress may further be assayed using an *in vivo* assay of DNA oxidation. *In vivo* oxidative stress may also be assayed as follows. Pigs are treated with the particle, conditioned medium or transformed mesenchymal stem cell (with saline as a control). Tissue sections of pig heart are obtained. Nuclear oxidative stress in tissue sections of treated and untreated pigs is quantified by 8-OHdG immunostaining for oxidized DNA. The tissue sections are assayed for intense nuclear staining indicative of DNA oxidation and oxidative stress.

### USES

Transformed mesenchymal stem cells and differentiated cells made according to the methods described here can be used for a variety of commercially important research, diagnostic, and therapeutic purposes. These uses are generally well known in the art, but will be described briefly here.

Mesenchymal stem cells and differentiated cell populations may be used for regenerative therapy. Mesenchymal stem cells, such as transformed mesenchymal stem cells and differentiated cells made therefrom, may be made by *ex vivo* expansion or directly administered into a patient. They may also be used for the re-population of damaged tissue following trauma.

Thus, adipocytes or fat tissues may therefrom may be used to fill up cavities or depressions during reconstructive or plastic surgery. Chondrocytes may be used for cartilage repair while osteocytes may be used for bone repair. The transformed mesenchymal stem cells made by the methods and compositions described here may be differentiated into any of these cell types and used for the purposes described.

The methods and compositions described here may be used for the production of a differentiated cell for the treatment of, or the preparation of a pharmaceutical composition for the treatment of, any one of the following: a disease treatable by regenerative therapy, cardiac failure, bone marrow disease, skin disease, burns, degenerative disease such as diabetes, Alzheimer's disease, Parkinson's disease and cancer.

Transformed mesenchymal stem cells and differentiated cells produced by the methods and compositions described here may be used for, or for the preparation of a pharmaceutical composition for, the treatment of a disease. Such disease may comprise a disease treatable by regenerative therapy, including cardiac failure, bone marrow disease, skin disease, burns, degenerative disease such as diabetes, Alzheimer's disease, Parkinson's disease, etc and cancer.

Stem cells, for example may be used as sources of mesenchymal stem cells and differentiated cells for NK or dendritic cells for immunotherapy for cancer, which mesenchymal stem cells and differentiated cells may be made by the methods and compositions described here.

It will be evident that the methods and compositions described here enable the production of transformed mesenchymal stem cells, which may of course be made to differentiate using methods known in the art. Thus, any uses of differentiated cells will equally attach to those transformed mesenchymal stem cells for which they are sources.

### DIFFERENTIATED CELLS

Differentiated cells, such as terminally differentiated cells, may be derived from the transformed mesenchymal stem cells or cell lines made according to the methods described. We therefore disclose methods for generating differentiated cells, the methods comprising generating transformed mesenchymal stem cells or cell lines as described, and deriving differentiated cells from these. The transformed mesenchymal stem cells made by the methods and compositions described here may be differentiated into any of these cell types and used for the purposes described.

Differentiated cells which may be made according to the methods described here may include any or all of the following:
i) adipocyte: the functional cell type of fat, or adipose tissue, that is found throughout the body, particularly under the skin. Adipocytes store and synthesize fat for energy, thermal regulation and cushioning against mechanical shock
ii) cardiomyocytes: the functional muscle cell type of the heart that allows it to beat continuously and rhythmically
iii) chondrocyte: the functional cell type that makes cartilage for joints, ear canals, trachea, epiglottis, larynx, the discs between vertebrae and the ends of ribs
iv) fibroblast: a connective or support cell found within most tissues of the body. Fibroblasts provide an instructive support scaffold to help the functional cell types of a specific organ perform correctly.
v) hepatocyte: the functional cell type of the liver that makes enzymes for detoxifying metabolic waste, destroying red blood cells and reclaiming their constituents, and the synthesis of proteins for the blood plasma
vi) hematopoietic cell: the functional cell type that makes blood. Hematopoietic cells are found within the bone marrow of adults. In the fetus, hematopoietic cells are found within the liver, spleen, bone marrow and support tissues surrounding the fe tus in the womb.
vii) myocyte: the functional cell type of muscles
viii) neuron: the functional cell type of the brain that is specialized in conducting impulses
ix) osteoblast: the functional cell type responsible for making bone
x) islet cell: the functional cell of the pancreas that is responsible for secreting insulin, glucogon, gastrin and somatostatin. Together, these molecules regulate a number of processes including carbohydrate and fat metabolism, blood glucose levels and acid secretions into the stomach.

### USES OF TRANSFORMED MESENCHYMAL STEM CELLS AND DIFFERENTIATED CELLS

Transformed mesenchymal stem cells and differentiated cells made according to the methods and compositions described here may be used for a variety of commercially important research, diagnostic, and therapeutic purposes.

For example, populations of differentiated cells may be used to prepare antibodies and cDNA libraries that are specific for the differentiated phenotype. General techniques used in raising, purifying and modifying antibodies, and their use in immunoassays and immunoisolation methods are described in Handbook of Experimental Immunology (Weir & Blackwell, eds.); Current Protocols in Immunology (Coligan et al., eds.); and Methods of Immunological Analysis (Masseyeff et al., eds., Weinheim: VCH Verlags GmbH). General techniques involved in preparation of mRNA and cDNA libraries are described in RNA Methodologies: A Laboratory Guide for Isolation and Characterization (R. E. Farrell, Academic Press, 1998); cDNA Library Protocols (Cowell & Austin, eds., Humana Press); and Functional Genomics (Hunt & Livesey, eds., 2000). Relatively homogeneous cell populations are particularly suited for use in drug screening and therapeutic applications.

These and other uses of transformed mesenchymal stem cells and differentiated cells are described in further detail below, and elsewhere in this document. The transformed mesenchymal stem cells and differentiated cells may in particular be used for the preparation of a pharmaceutical composition for the treatment of disease. Such disease may comprise a disease treatable by regenerative therapy, including cardiac failure, bone marrow disease, skin disease, burns, degenerative disease such as diabetes, Alzheimer's disease, Parkinson's disease, etc and cancer.

The transformed mesenchymal stem cells made by the methods and compositions described here have similar or identical properties to human embryonic stem cell derived mesenchymal stem cells (hESC-MSCs), for example as described in WO2007/027157 or WO2007/027156. Therefore, the transformed mesenchymal stem cells, and any differentiated cells made from these, may be used in any of the applications for which mesenchymal stem cells such as hESC-mesenchymal stem cells are known to be used, or in which it is possible for them to be used.

### DRUG SCREENING

Transformed mesenchymal stem cells and differentiated cells made according to the methods and compositions described here may also be used to screen for factors (such as solvents, small molecule drugs, peptides, polynucleotides, and the like) or environmental conditions (such as culture conditions or manipulation) that affect the characteristics of transformed mesenchymal stem cells or differentiated cells.

In some applications, transformed mesenchymal stem cells and differentiated cells are used to screen factors that promote maturation, or promote proliferation and maintenance of such cells in long-term culture. For example, candidate maturation factors or growth factors are tested by adding them to transformed mesenchymal stem cells or differentiated cells in different wells, and then determining any phenotypic change that results, according to desirable criteria for further culture and use of the cells.

Furthermore, gene expression profiling of transformed mesenchymal stem cells and differentiated cells may be used to identify receptors, transcription factors, and signaling molecules that are unique or highly expressed in these cells. Specific ligands, small molecule inhibitors or activators for the receptors, transcription factors and signaling molecules may be used to modulate differentiation and properties of transformed mesenchymal stem cells and differentiated cells.

Particular screening applications relate to the testing of pharmaceutical compounds in drug research. The reader is referred generally to the standard textbook 'In vitro Methods in Pharmaceutical Research', Academic Press, 1997, and U.S. Pat. No. 5,030,015), as well as the general description of drug screens elsewhere in this document. Assessment of the activity of candidate pharmaceutical compounds generally involves combining the differentiated cells with the candidate compound, determining any change in the morphology, marker phenotype, or metabolic activity of the cells that is attributable to the compound (compared with untreated cells or cells treated with an inert compound), and then correlating the effect of the compound with the observed change.

The screening may be done, for example, either because the compound is designed to have a pharmacological effect on certain cell types, or because a compound designed to have effects elsewhere may have unintended side effects. Two or more drugs can be tested in combination (by combining with the cells either simultaneously or sequentially), to detect possible drug--drug interaction effects. In some applications, compounds are screened initially for potential toxicity (Castell et al., pp. 375-410 in 'In vitro Methods in Pharmaceutical Research,' Academic Press, 1997). Cytotoxicity can be determined in the first instance by the effect on cell viability, survival, morphology, and expression or release of certain markers, receptors or enzymes. Effects of a drug on chromosomal DNA can be determined by measuring DNA synthesis or repair. [³H]thymidine or BrdU incorporation, especially at unscheduled times in the cell cycle, or above the level required for cell replication, is consistent with a drug effect. Unwanted effects can also include unusual rates of sister chromatid exchange, determined by metaphase spread. The reader is referred to A. Vickers (PP 375-410 in 'In vitro Methods in Pharmaceutical Research,' Academic Press, 1997) for further elaboration.

### TISSUE REGENERATION

Transformed mesenchymal stem cells and differentiated cells made according to the methods and compositions described here may also be used for tissue reconstitution or regeneration in a human patient in need thereof. The cells are administered in a manner that permits them to graft to the intended tissue site and reconstitute or regenerate the functionally deficient area.

For example, the methods and compositions described here may be used to modulate the differentiation of stem cells. Transformed mesenchymal stem cells and differentiated cells may be used for tissue engineering, such as for the growing of skin grafts. Modulation of stem cell differentiation may be used for the bioengineering of artificial organs or tissues, or for prosthetics, such as stents.

### CANCER

Transformed mesenchymal stem cells and differentiated cells made by the methods and compositions described here may be used for the treatment of cancer.

The terms 'cancer' and 'cancerous' refer to or describe the physiological condition in mammals that is typically characterized by unregulated cell growth. Examples of cancer include but are not limited to, carcinoma, lymphoma, blastoma, sarcoma, and leukemia.

More particular examples of such cancers include squamous cell cancer, small-cell lung cancer, non-small cell lung cancer, gastric cancer, pancreatic cancer, glial cell tumors such as glioblastoma and neurofibromatosis, cervical cancer, ovarian cancer, liver cancer, bladder cancer, hepatoma, breast cancer, colon cancer, colorectal cancer, endometrial carcinoma, salivary gland carcinoma, kidney cancer, renal cancer, prostate cancer, vulval cancer, thyroid cancer, hepatic carcinoma and various types of head and neck cancer. Further examples are solid tumor cancer including colon cancer, breast cancer, lung cancer and prostrate cancer, hematopoietic malignancies including leukemias and lymphomas, Hodgkin's disease, aplastic anemia, skin cancer and familiar adenomatous polyposis. Further examples include brain neoplasms, colorectal neoplasms, breast neoplasms, cervix neoplasms, eye neoplasms, liver neoplasms, lung neoplasms, pancreatic neoplasms, ovarian neoplasms, prostatic neoplasms, skin neoplasms, testicular neoplasms, neoplasms, bone neoplasms, trophoblastic neoplasms, fallopian tube neoplasms, rectal neoplasms, colonic neoplasms, kidney neoplasms, stomach neoplasms, and parathyroid neoplasms. Breast cancer, prostate cancer, pancreatic cancer, colorectal cancer, lung cancer, malignant melanoma, leukaemia, lympyhoma, ovarian cancer, cervical cancer and biliary tract carcinoma are also included.

The transformed mesenchymal stem cells and differentiated cells made according to the methods and compositions described here may also be used in combination with anticancer agents such as endostatin and angiostatin or cytotoxic agents or chemotherapeutic agent. For example, drugs such as such as adriamycin, daunomycin, cis-platinum, etoposide, taxol, taxotere and alkaloids, such as vincristine, and antimetabolites such as methotrexate. The term 'cytotoxic agent' as used herein refers to a substance that inhibits or prevents the function of cells and/or causes destruction of cells. The term is intended to include radioactive isotopes (e.g. I, Y, Pr), chemotherapeutic agents, and toxins such as enzymatically active toxins of bacterial, fungal, plant or animal origin, or fragments thereof.

Also, the term includes oncogene product/tyrosine kinase inhibitors, such as the bicyclic ansamycins disclosed in WO 94/22867; 1,2-bis(arylamino) benzoic acid derivatives disclosed in EP 600832; 6,7-diamino-phthalazin-1-one derivatives disclosed in EP 600831; 4,5-bis(arylamino)-phthalimide derivatives as disclosed in EP 516598; or peptides which inhibit binding of a tyrosine kinase to a SH2-containing substrate protein (see WO 94/07913, for example). A 'chemotherapeutic agent' is a chemical compound useful in the treatment of cancer. Examples of chemotherapeutic agents include Adriamycin, Doxorubicin, 5-Fluorouracil (5-FU), Cytosine arabinoside (Ara-C), Cyclophosphamide, Thiotepa, Busulfan, Cytoxin, Taxol, Methotrexate, Cisplatin, Melphalan, Vinblastine, Bleomycin, Etoposide, Ifosfamide, Mitomycin C, Mitoxantrone, Vincristine, VP-16, Vinorelbine, Carboplatin, Teniposide, Daunomycin, Carminomycin, Aminopterin, Dactinomycin, Mitomycins, Nicotinamide, Esperamicins (see U.S. Pat. No. 4,675,187), Melphalan and other related nitrogen mustards, and endocrine therapies (such as diethylstilbestrol (DES), Tamoxifen, LHRH antagonizing drugs, progestins, anti-progestins etc).

### DISEASES TREATABLE

The proteome of transformed mesenchymal stem cells obtained by the methods described here may be analysed.

The transformed mesenchymal stem cells thus obtained my have an expression profile which is similar to that of mesenchymal stem cells, such as hESC-MSCs described in WO2007/027157 or WO2007/027156. Thus, transformed mesenchymal stem cells derived by our methods may have significant biological similarities to their counterparts, e.g., in their ability to secrete paracrine factors.

Accordingly, the transformed mesenchymal stem cells described here may be used for any purpose for which hESC-MSCs such as those described in WO2007/027157 or WO2007/027156 are suitable.

The transformed mesenchymal stem cells may be used to treat diseases of metabolism, defense response, and tissue differentiation including vascularization, hematopoiesis and skeletal development, or whose repair or treatment involves any one or more of these biological processes. Similarly, the proteins expressed, such as secreted, by the transformed mesenchymal stem cells, singly or in combination, such as in the form of a conditioned medium, may be used to supplement the activity of, or in place of, the transformed mesenchymal stem cells, for the purpose of for example treating or preventing such diseases.

The transformed mesenchymal stem cells may be used to activate signalling pathways in cardiovascular biology, bone development and hematopoiesis such as Jak-STAT, MAPK, Toll-like receptor, TGF-beta signalling and mTOR signaling pathways. Thus, the transformed mesenchymal stem cells, proteins expressed by them, etc, may be used to prevent or treat a disease in which any of these signalling pathways is involved, or whose aetiology involves one or more defects in any one or more of these signalling pathways.

Accordingly, transformed mesenchymal stem cells may be used to treat cardiac failure, bone marrow disease, skin disease, burns and degenerative diseases such as diabetes, Alzheimer's disease, Parkinson's disease and cancer.

Transformed mesenchymal stem cells may also be used to treat myocardial infarction, a cutaneous wound, a dermatologic disorder, a dermatological lesion, dermatitis, psoriasis, condyloma, verruca, hemangioma, keloid, skin cancer, atopic dermatitis, Behcet disease, chronic granulomatous disease, cutaneous T cell lymphoma, ulceration, a pathological condition characterised by initial injury inducing inflammation and immune dysregulation leading to chronic tissue remodeling including fibrosis and loss of function, renal ischemic injury, cystic fibrosis, sinusitis and rhinitis or an orthopaedic disease.

They may be used to aid wound healing, scar reduction, bone formation, a bone graft or bone marrow transplantation in an individual.

We provide for the use of transformed mesenchymal stem cells obtained herein or medium conditioned by such transformed mesenchymal stem cells in the regulation of pathways including any one or more of the following: cytoskeletal regulation by Rho GTPase, cell cycle, integrin signalling pathway, Inflammation mediated by chemokine & cytokine signaling pathway, FGF signaling pathway, EGF receptor signaling pathway, angiogenesis, plasminogen activating cascade, blood coagulation, glycolysis, ubiquitin proteasome pathway, de novo purine biosynthesis, TCA cycle, phenylalanine biosynthesis, heme biosynthesis.

We provide also for the use of transformed mesenchymal stem cells obtained herein or medium conditioned by such transformed mesenchymal stem cells in the regulation of processes including any one or more of the following: cell structure and motility, cell structure, cell communication, cell motility, cell adhesion, endocytosis, mitosis, exocytosis, cytokinesis, cell cycle, immunity and defense, cytokine/chemokine mediated immunity, macrophage-mediated immunity, granulocyte-mediated immunity, ligand-mediated signaling, cytokine and chemokine mediated signaling pathway, signal transduction, extracellular matrix protein-mediated signaling, growth factor homeostasis, receptor protein tyrosine kinase signaling pathway, cell adhesion-mediated signaling, cell surface receptor mediated signal transduction, JAK-STAT cascade, antioxidation and free radical removal, homeostasis, stress response, blood clotting, developmental processes, mesoderm development, skeletal development, angiogenesis, muscle development, muscle contraction, protein metabolism and modification, proteolysis, protein folding, protein complex assembly, amino acid activation, intracellular protein traffic, other protein targeting and localization, amino acid metabolism, protein biosynthesis, protein disulfide-isomerase reaction, carbohydrate metabolism, glycolysis, pentose-phosphate shunt, other polysaccharide metabolism, purine metabolism, regulation of phosphate metabolism, vitamin metabolism, amino acid biosynthesis, pre-mRNA processing, translational regulation, mRNA splicing.

We further provide for the use of transformed mesenchymal stem cells obtained herein or medium conditioned by such transformed mesenchymal stem cells in the supply of functions including any one or more of the following: signaling molecule, chemokine, growth factor, cytokine, interleukin, other cytokine, extracellular matrix, extracellular matrix structural protein, other extracellular matrix, extracellular matrix glycoprotein, protease, metalloprotease, other proteases, protease inhibitor, metalloprotease inhibitor, serine protease inhibitor, oxidoreductase, dehydrogenase, peroxidase, chaperone, chaperonin, Hsp 70 family chaperone, other chaperones, synthetase, synthase and synthetase, select calcium binding protein, aminoacyl-tRNA synthetase, lyase, isomerase, other isomerase, ATP synthase, hydratase, transaminase, other lyase, other enzyme regulator, select regulatory molecule, actin binding cytoskeletal protein, cytoskeletal protein, non-motor actin binding protein, actin and actin related protein, annexin, tubulin, cell adhesion molecule, actin binding motor protein, intermediate filament, ribonucleoprotein, ribosomal protein, translation factor, other RNA-binding protein, histone, calmodulin related protein, vesicle coat protein.

Furthermore, the transformed mesenchymal stem cells obtained herein or medium conditioned by such transformed mesenchymal stem cells may be used to treat diseases which these functions may have a role in, or whose repair or treatment involves any one or more of these biological processes. Similarly, the proteins expressed by the transformed mesenchymal stem cells, singly or in combination, such as in the form of a conditioned medium, may be used to supplement the activity of, or in place of, the transformed mesenchymal stem cells, for the purpose of for example treating or preventing such diseases.

The gene products expressed by the transformed mesenchymal stem cells obtained herein may be used to activate important signalling pathways in cardiovascular biology, bone development and hematopoiesis such as Jak-STAT, MAPK, Toll-like receptor, TGF-beta signalling and mTOR signaling pathways. Accordingly, the hESC-MSCs, proteins expressed by them, etc, may be used to prevent or treat a disease in which any of these signalling pathways is involved, or whose aetiology involves one or more defects in any one or more of these signalling pathways.

Accordingly, such a conditioned medium may be used to treat cardiac failure, bone marrow disease, skin disease, burns and degenerative diseases such as diabetes, Alzheimer's disease, Parkinson's disease and cancer.

Such a conditioned medium may also be used to treat myocardial infarction, a cutaneous wound, a dermatologic disorder, a dermatological lesion, dermatitis, psoriasis, condyloma, verruca, hemangioma, keloid, skin cancer, atopic dermatitis, Behcet disease, chronic granulomatous disease, cutaneous T cell lymphoma, ulceration, a pathological condition characterised by initial injury inducing inflammation and immune dysregulation leading to chronic tissue remodeling including fibrosis and loss of function, renal ischemic injury, cystic fibrosis, sinusitis and rhinitis or an orthopaedic disease.

The conditioned medium may be used to aid wound healing, scar reduction, bone formation, a bone graft or bone marrow transplantation in an individual.

In particular, the conditioned medium may be used to regulate the processes involved in vascularisation, hematology (specifically immune processes) or musculoskeletal development, etc.

Such a composition may be used for any purpose the conditioned medium may be used. Unless the context dictates otherwise, the term 'conditioned medium' should be taken to include not only cell culture medium exposed to MSCs as well as such a composition comprising one or more, such as substantially all, the polypeptides which are present in the conditioned medium.

Furthermore, any one or more proteins secreted from the transformed mesenchymal stem cells described here, including in the form of conditioned media, may be used for the same purposes as the hESC-MSCs as described in WO2007/027157 or WO2007/027156.

The transformed mesenchymal stem cells may also be used as sources for any of the proteins secreted or expressed by them. We therefore provide for a method of producing a polypeptide comprising obtaining a transformed mesenchymal stem cell as described, culturing the transformed mesenchymal stem cell and isolating the polypeptide from the transformed mesenchymal stem cell, such as from a medium in which the transformed mesenchymal stem cell is growing.

### Dermatologic Disorders

Media conditioned by the transformed mesenchymal stem cells obtained by the methods described in this document may be used for the treatment of dermatologic disorders.

Therefore the topical application of such conditioned medium on cutaneous wounds or dermatological lesions or disorders such as dermatitis or psoriasis improves healing and reduces scarring. It should also maintain homeostasis. The conditioned medium may be delivered in liposome-based emulsion, gel or cream formulations and part of standard wound dressing. The conditioned medium may also be used as a supplement in cosmetic skincare product to promote skin repair and healing.

A suitable animal model to test the efficacy of such conditioned medium on cutaneous disorders is a mouse model of dermatitis. Epicutaneous sensitization of mice is performed as described earlier [A72, A73]. Briefly, 50µg of Blo t 5 in 100µl of PBS or PBS alone are applied to 1cm² gauze and patched to the skin with a transparent dressing (Smith Nephew). This procedure is repeated twice over a period of 50 days. CM and NCM are then applied to 1cm2 gauze and patched to the skin as described above for varying period of time.

For histological examination of skin inflammation, specimens are obtained from the patched skins and fixed in 10% buffered neutral formalin immediately.

### Asthma and Allergy

Media conditioned by the transformed mesenchymal stem cells obtained by the methods described in this document may be used for the treatment of asthma and allergy.

Asthma is a complex disease with an equally complex etiology caused by a poorly characterized set of genetic and environmental factors. The resulting pathology is immune dysregulation leading to chronic inflammation of the airways and subepithelial fibrosis characterized by increase in smooth muscle mass and increased deposition of extracellular matrix proteins and subsequently, reduced lung function.

Current therapies include modulating several factors or signaling pathways e.g. the ECM, integrins, and mesenchymal cell function [A74], toll-like receptors [A75], growth factors such as TGF-β and EGF [A76, A77] and the IL6 pathway [A78]. The CM by hESC-MSCs has been predicted to have biological effects on these targeted area and we predict that the CM helps restore immune regulation in asthmatic lungs and promote tissue repair and minimize scarring of lung tissues.

To investigate the effects of such conditioned medium (CM) and the non-conditioned medium (NCM) on chronic airway inflammation and airway, epicutaneous sensitization of mice are performed as described above [A72, A73] After 50 days, the patched mice are anesthetized and receive intranasal challenge with 50 µg of Blo t 5 for three consecutive days. Twenty-four hours after the last dose, airway hyperresponsivensess (AHR) is measured using invasive BUXCO[A79]. The mice are anesthetized and given conditioned medium or non-conditioned medium intranasally for three consecutive days. Twenty-four hours after the last dose, airway hyperresponsivensess (AHR) is measured. BAL fluid is collected after another twenty-four hours. Following bronchoalveolar lavage collection, lungs are fixed with 10% neutralized formalin.

To investigate the the effects of CM and the NCM on acute airway inflammation and airway.

A Blot t 5 specific Th2 cell line which secretes high level of IL-4, IL-5, IL-13 and with undetectable level of IFN-γ, is used to establish a mouse allergy model. Briefly, sensitization of naive mice is done by adoptive transfer of 2.5 x 10⁶ Blo t 5 specific Th2 cells intravenously in each mouse. These mice are anesthetized and intranasal (IN) challenged with 50 µg of Blo t 5 for three consecutive days. Twenty-four hours after the last IN challenge, airway hyperresponsivensess (AHR) is measured using invasive BUXCO [A79]. The mice are then anesthetized and given CM or NCM intranasally for three consecutive days. BAL fluid is collected at forty-eight hours after the last Blo t 5 challenge. Following bronchoalveolar lavage collection, lungs are fixed with 10% neutralized formalin for histopathological analysis.

In clinical practice, the use of CM to treat lung disease may be administered effectively using standard aerosol therapy [A80-86].

### Other Diseases

Media conditioned by the transformed mesenchymal stem cells obtained by the methods described in this document may be used for the treatment of other diseases.

In general, we predict that such conditioned medium is useful in restoring homeotstasis and promoting tissue repair in pathological conditions where the initial injury induced inflammation and immune dysregulation leads to chronic tissue remodeling that includes fibrosis and loss of function. Other diseases include renal ischemic injury, cystic fibrosis, sinusitis and rhinitis.

### Orthopedics

Media conditioned by the transformed mesenchymal stem cells obtained by the methods described in this document may be used for the treatment of orthopedic disorders.

Current therapeutic strategies for repair of musculoskeletal tissue often include the use of a biomaterial (ceramics or polymers) not only to provide mechanical support but also as a scaffold to promote cell migration, cell adhesion, proliferation and differentiation to initiate vascularization and ultimately new bone formation [A87-90]. Based on the computation analysis of such conditioned medium, incorporation of CM into the scaffold design may enhance cell migration, proliferation, adhesion, skeletal differentiation and vascularization of the scaffold.

To test the effect of CM on bone regeneration in defects that would otherwise have led to atrophic nonunions, New Zealand white rabbits receive a 15-mm critical size defect on one radius [A91], which is filled with a suitable matrix such as a collagen sponge or hydrogel coated with either CM or NCM. Radiographs are obtained every 3 weeks. After 6 or 12 weeks, animals are killed. New bone is measured by microCT scans and vascularity is measured using anti-CD31 staining of endothelial cells in the implant. There should be increased vascularity at the least initially and also increased new bone formation.

To test the effects of CM on cartilage repair, a rabbit model of osteochondral injury [A92] is used. CM is coated on a suitable scaffold such as collagen or hydrogel and implanted into 3-mm osteochondral knee defects [A93]. For clinical applications, CM may be used by incorporating the CM into existing scaffolds or bone grafts [A94].

### Bone Marrow Transplantation

MSCs have been shown to enhance bone marrow transplantation [A95] and ameliorate graft versus host disease. Transplantation of MSCs has been shown to improve the outcome of allogeneic transplantation by promoting hematopoietic engraftment [A96] and limiting GVHD [A97, A98]. It is postulated that MSCs mediate these effects through the enhancement of the hematopoieitic stem cell niche [A99] and the induction of tolerance and reduce GVHD, rejection of allogeneic tissue transplant and modulation of inflammation [A98], possiblly through the secretion of soluble factors [A100].

Potential clinical applications of such conditioned medium by MSCs: expansion of hematopoietic stem cell population in vitro by supplementing culture media with CM or *in vivo* by infusing CM with hematopoietic stem cells during transplantation, ameliorate GVHD by intravenous infusion of CM or induction of immune tolerance to transplanted cells or tissues by intravenous infusion of CM as part of the pre and post-transplant therapy.

### Heart Disease

The transformed mesenchymal stem cells described here may be used for treatment or prevention of heart disease.

Heart disease is an umbrella term for a variety for different diseases affecting the heart. As of 2007, it is the leading cause of death in the United States, England, Canada and Wales, killing one person every 34 seconds in the United States alone. Heart disease includes any of the following.

### Coronary Heart Disease

Coronary artery disease is a disease of the artery caused by the accumulation of atheromatous plaques within the walls of the arteries that supply the myocardium. Angina pectoris (chest pain) and myocardial infarction (heart attack) are symptoms of and conditions caused by coronary heart disease. Over 459,000 Americans die of coronary heart disease every year. In the United Kingdom, 101,000 deaths annually are due to coronary heart disease.

### Cardiomyopathy

Cardiomyopathy is the deterioration of the function of the myocardium (i.e., the actual heart muscle) for any reason. People with cardiomyopathy are often at risk of arrhythmia and/or sudden cardiac death. Extrinsic cardiomyopathies - cardiomyopathies where the primary pathology is outside the myocardium itself comprise the majority of cardiomyopathies. By far the most common cause of a cardiomyopathy is ischemia.

The World Health Organization includes as specific cardiomyopathies: Alcoholic cardiomyopathy, coronary artery disease, congenital heart disease, nutritional diseases affecting the heart, ischemic (or ischaemic) cardiomyopathy, hypertensive cardiomyopathy, valvular cardiomyopathy, inflammatory cardiomyopathy.

Also included are:
Cardiomyopathy secondary to a systemic metabolic disease
Intrinsic cardiomyopathies (weakness in the muscle of the heart that is not due to an identifiable external cause)
Dilated cardiomyopathy (DCM, the most common form, and one of the leading indications for heart transplantation. In DCM the heart (especially the left ventricle) is enlarged and the pumping function is diminished)
Hypertrophic cardiomyopathy (HCM or HOCM, a genetic disorder caused by various mutations in genes encoding sarcomeric proteins. In HCM the heart muscle is thickened, which can obstruct blood flow and prevent the heart from functioning properly),
Arrhythmogenic right ventricular cardiomyopathy (ARVC, which arises from an electrical disturbance of the heart in which heart muscle is replaced by fibrous scar tissue. The right ventricle is generally most affected)
Restrictive cardiomyopathy (RCM, which is the least common cardiomyopathy. The walls of the ventricles are stiff, but may not be thickened, and resist the normal filling of the heart with blood).
Noncompaction Cardiomyopathy - the left ventricle wall has failed to properly grow from birth and such has a spongy appearance when viewed during an echocardiogram.

### Cardiovascular Disease

Cardiovascular disease is any of a number of specific diseases that affect the heart itself and/or the blood vessel system, especially the veins and arteries leading to and from the heart. Research on disease dimorphism suggests that women who suffer with cardiovascular disease usually suffer from forms that affect the blood vessels while men usually suffer from forms that affect the heart muscle itself. Known or associated causes of cardiovascular disease include diabetes mellitus, hypertension, hyperhomocysteinemia and hypercholesterolemia.

Types of cardiovascular disease include atherosclerosis

### Ischaemic Heart Disease

Ischaemic heart disease is disease of the heart itself, characterized by reduced blood supply to the organs. This occurs when the arteries that supply the oxygen and the nutrients gets stopped and the heart will not get enough of the oxygen and the nutrients and will eventually stop beating.

### Heart Failure

Heart failure, also called congestive heart failure (or CHF), and congestive cardiac failure (CCF), is a condition that can result from any structural or functional cardiac disorder that impairs the ability of the heart to fill with or pump a sufficient amount of blood throughout the body. Cor pulmonale is a failure of the right side of the heart.

### Hypertensive Heart Disease

Hypertensive heart disease is heart disease caused by high blood pressure, especially localised high blood pressure. Conditions that can be caused by hypertensive heart disease include: left ventricular hypertrophy, coronary heart disease, (Congestive) heart failure, hypertensive cardiomyopathy, cardiac arrhythmias, inflammatory heart disease, etc.

Inflammatory heart disease involves inflammation of the heart muscle and/or the tissue surrounding it. Endocarditis comprises inflammation of the inner layer of the heart, the endocardium. The most common structures involved are the heart valves. Inflammatory cardiomegaly. Myocarditis comprises inflammation of the myocardium, the muscular part of the heart.

### Valvular Heart Disease

Valvular heart disease is disease process that affects one or more valves of the heart. The valves in the right side of the heart are the tricuspid valve and the pulmonic valve. The valves in the left side of the heart are the mitral valve and the aortic valve. Included are aortic valve stenosis, mitral valve prolapse and valvular cardiomyopathy.

[The above text is adapted from Heart disease. (2009, February 3). In Wikipedia, The Free Encyclopedia. Retrieved 06:33, February 20, 2009, from http://en.wikipedia.org/w/index.php?title=Heart disease&oldid=268290924]

### PRE-/MATURE MIRNA RATIO

We also demonstrate in the Examples that the ratio of precursor to mature miRNA changes depending on the state of a cell. Accordingly, such a ratio, which we term the 'pre-/mature miRNA ratio' may be used as a marker to monitor the state of a cell.

The Examples show, using quantitative RT-PCR (qRT-PCR) analysis, that exosomes secreted from mesenchymal stem cells (MSCs) derived from human embryonic stem cells (hESC) contain miRNAs. Such miRNAs are present predominantly in the precursor (pre)-miRNA form with no detectable level of primary (pri) miRNA. Thus, for example, the ratio of pre- to mature miRNA for hsa-let-7b (miRBase Accession Number: MI0000063) and hsa-let-7g (miRBase Accession Number: MI0000433) in the exosome relative to that in the cells was 169 and 1078 times higher respectively.

We show in the Examples that MSCs transformed with a c-myc gene do not senesce and continue to proliferate and maintain a high level of telomerase activity. We demonstrate using real-time RT-PCR analysis that, relative to the secretion by the parental MSCs, the ratio of pre- to mature miRNA in the myc-transformed MSC secretion for hsa-let-7b miRNA (miRBase Accession Number: MI0000063) is increased by 2.8 times. On the other hand, the ratio of pre- to mature miRNA for hsa-let-7g miRNA is unchanged as a result of transformation.

hsa-let-7b is described in detail elsewhere in this document.

Thus, transformation of MSC with an oncogene causes the cells to display an oncogenic state; in such an oncogenic state, the ratio of precursor to mature hsa-let-7b miRNA (miRBase Accession Number: MI0000063) is increased, relative to the ratio of precursor to mature hsa-let-7b miRNA in an untransformed cell. Accordingly, the ratio of precursor to mature hsa-let-7b miRNA miRNA may be used to monitor the oncogenic state of a cell.

The ratio of precursor to mature miRNA for other miRNA species is also observed to be changed as a result of the transformation. Accordingly, the pre- to mature miRNA ratio of these other miRNA species may be used, alternatively, or in addition to, the pre- to mature miRNA ratio of hsa-let-7b miRNA, as a marker for oncogenic transformation.

We find that the ratio of precursor to mature miRNA for certain miRNA species is changed as a result of a change of a cellular state, e.g., a change of a physiological state or a change of a pathological state, or a transition between a physiological state to a pathological state.

Accordingly, cellular changes of state may be monitored by monitoring the pre- to mature miRNA ratio of these miRNAs.

We describe a method of monitoring the state of a cell, the method comprising establishing, for a selected microRNA (miRNA) species secreted by the cell, a ratio of: (a) a precursor form of the miRNA species (pre-miRNA); to (b) a mature form of the miRNA species (mature miRNA); in which the pre- to mature miRNA ratio so established is indicative of the state of the cell.

The cell may be comprised in a cell mass, tissue, organ or organism. The pre- to mature miRNA ratio may be indicative of the state of the respective cell mass, tissue, organ or organism.

The miRNA species may be comprised in exosomes secreted by the cell. The method may comprise obtaining such exosomes and obtaining precursor and mature forms of the miRNA species therefrom.

The pre- to mature miRNA ratio may be established by hybridisation to an array comprising nucleic acid sequences capable of binding to and distinguishing between precursor and mature forms of the miRNA species.

The pre- to mature miRNA ratio may be established by real time polymerase chain reaction (RT-PCR).

The method may comprise establishing a profile comprising a plurality of pre- to mature miRNA ratios for a plurality of selected miRNA species, each indicative of the state of the cell.

The method may comprise establishing a profile comprising a plurality of pre- to mature miRNA ratios for a plurality of selected miRNA species, the profile being established by hybridisation to an array comprising a plurality of probes capable of binding to and distinguishing between precursor and mature forms of the plurality of the selected miRNA species.

The exosomes may be obtained from a sample of an organism, which organism comprises the cell. The sample may comprise a blood sample, a serum sample, a saliva sample or a urine sample.

The state of the cell may comprise a physiological state, such as a cell cycle state, a differentiation state, a development state or a metabolic state.

The state of the cell may comprise a pathological state, such as a disease state, a human disease state, a diabetic state, an immune disorder state, a neurodegenerative disorder state, an oncogenic state, a cancerous state or a tumour state.

We further describe a method as set out above for detecting a change in state of a cell, the method comprising detecting a change in a pre- to mature miRNA ratio of the cell (or a profile comprising such a ratio), in which such a change indicates a change in state of the cell.

We further describe a method as set out above for establishing that a cell is in a particular state, the method comprising comparing a pre- to mature miRNA ratio of the cell (or a profile comprising such a ratio) with a pre- to mature miRNA ratio (or a profile comprising such a ratio) of a cell known to be in that particular state.

We further describe a method of monitoring the state of an organism, the method comprising obtaining a sample from or of that organism, performing a method as set out above on the sample, and establishing the state of the organism from the pre- to mature miRNA ratio thereby obtained.

We further describe a method of detecting a disease in an organism, the method comprising obtaining a sample from or of that organism, performing a method as set out above on the sample, and comparing the pre- to mature miRNA ratio thereby obtained with a pre- to mature miRNA ratio of a sample known to be of or from a diseased organism.

We further describe a method of treatment or prevention of a disease in an organism, the method comprising detecting a disease in an organism according to Claim 14, and administering a treatment for that disease to the organism.

The cell may comprise a mesenchymal stem cell (MSC). The states of the cell may comprise an untransformed state and a transformed state, such as a c-myc transformed state. The microRNA may comprise hsa-let-7b microRNA. The ratio may be 2.8 higher in a transformed state compared to a normal state. Any combination of the above may be possible.

We further describe an array comprising a plurality of nucleic acid sequences capable of binding to and distinguishing between precursor and mature forms of a plurality of miRNA species.

### HSA-LET-7B

hsa-let-7b has miRBASE accession number MI0000063. Pre- hsa-let-7b has the following sequence:
>hsa-let-7b MI0000063 
CGGGGUGAGGUAGUAGGUUGUGUGGUUUCAGGGCAGUGAUGUUGCCCCUCGGAAGAUAACUAU
ACAACCUACUGCCUUCCCUG
Pre- hsa-let-7b has the following structure:

    u                          ucagggcagugaug
cgggg gagguaguagguugugugguu              u
||||| |||||||||||||||||||||
guccc uuccgucauccaacauaucaa              u
    -                          uagaaggcuccccg

Mature hsa-let-7b has the miRBase accession number MIMAT0000063 and the following sequence:
>hsa-let-7b MIMAT0000063
   UGAGGUAGUAGGUUGUGUGGUU

hsa-let-7b is described in detail in the following publications: "Identification of novel genes coding for small expressed RNAs" Lagos-Quintana M, Rauhut R, Lendeckel W, Tuschl T. Science. 294:853-858(2001); "Reduced accumulation of specific microRNAs in colorectal neoplasia" Michael MZ, O' Connor SM, van Holst Pellekaan NG, Young GP, James RJ. Mol Cancer Res. 1:882-891(2003); "Altered expression profiles of microRNAs during TPA-induced differentiation of HL-60 cells" Kasashima K, Nakamura Y, Kozu T. Biochem Biophys Res Commun. 322:403-410(2004); "A mammalian microRNA expression atlas based on small RNA library sequencing" Landgraf P, Rusu M, Sheridan R, Sewer A, Iovino N, Aravin A, Pfeffer S, Rice A, Kamphorst AO, Landthaler M, Lin C, Socci ND, Hermida L, Fulci V, Chiaretti S, Foa R, Schliwka J, Fuchs U, Novosel A, Muller RU, Schermer B, Bissels U, Inman J, Phan Q, Chien M. Cell. 129:1401-1414(2007) and "Patterns of known and novel small RNAs in human cervical cancer" Lui WO, Pourmand N, Patterson BK, Fire A. Cancer Res. 67:6031-6043(2007).

### CHANGE OF CELLULAR STATE

According to the methods and compositions described here, the pre- to mature miRNA ratio may be used to monitor various cellular changes of states.

Such cellular changes of state may comprise changes of physiological states, such as a cell cycle state. Accordingly, monitoring the pre- to mature miRNA ratio of an miRNA species may be used to monitor the G1 state, an M state, a G2 state or a S state of a cell.

The physiological state may comprise a differentiation state; in other words, the pre- to mature miRNA ratio of an miRNA species may be used to determine whether a cell is differentiated or un-differentiated. It may be used for example to detect the pluripotency of a cell. It may be used to detect whether a cell is a stem cell, or whether a cell is a differentiating cell or if the cell is terminally differentiated, for example.

The physiological state may comprise a developmental state or developmental stage. Thus, the pre- to mature miRNA ratio of an miRNA species may be used to determine if a cell is of or from an embryonic state or stage, foetal state or stage, a neonatal state or stage, an infant state or stage, a juvenile state or stage, a toddler state or stage, an adolescent state or stage, an adult state or stage etc.

### MONITORING OF PATHOLOGICAL STATES

The cellular state may comprise a pathological state, such as a disease state. Thus, the pre- to mature miRNA ratio of an miRNA species may be used to monitor a pathological state of a cell, or a change of a normal state to a pathological state.

The pre- to mature miRNA ratio may be used to detect whether the cell is in a normal (undiseased) state or a diseased state. It may be used to monitor the progression of a cell from a normal, undiseased state to a diseased state. It may be used to monitor the stage of disease of the cell. The disease may comprise any of the variety of diseases an organism suffers or may suffer.

In general, the disease state may comprise any one or more of hypertrophic cardiomyopathy, bacterial endocarditis, agyria, amyotrophic lateral sclerosis, dizziness, tetralogy of fallot, myocarditis, alcoholism, anemia, brachial plexus, neuropathies, hemorrhoids, congenital heart defects, alopecia areata, sickle cell anemia, mitral valve prolapse, autonomic nervous system diseases, abnormalities, alzheimer disease, angina pectoris, rectal diseases or arrhythmogenic right.

The disease state may comprise any one or more of ventricular dysplasia, acne rosacea, amblyopia, ankylosing spondylitis, atrial fibrillation, cardiac tamponade, acquired immunodeficiency syndrome, amyloidosis, anorexia, anxiety, autism, brain neoplasms, central nervous system diseases, color vision defects, arteriosclerosis, back pain, breast diseases, central nervous system infections, colorectal neoplasms, arthritis, behcet's syndrome, breast neoplasms, cerebral palsy, common cold, asthma, bipolar disorder, burns or cervix neoplasms.

The disease state may comprise any one or more of communication disorders, atherosclerosis, blindness, candidiasis, charcot-marie disease, crohn disease, attention deficit disorder, brain injuries, cataract, ulcerative colitis, cumulative trauma disorders, cystic fibrosis, developmental disabilities, eating disorders, erysipelas, fibromyalgia, decubitus ulcer, diabetes, emphysema, escherichia coli infections, folliculitis, deglutition disorders, diabetic foot or encephalitis.

The disease state may comprise any one or more of esophageal diseases, food hypersensitivity, dementia, down syndrome, japanese encephalitis, eye neoplasms, food poisoning, dengue, dyslexia, endometriosis, fabry's disease, gastroenteritis, depression, dystonia, epilepsy, chronic fatigue syndrome, gastroesophageal reflux, gaucher's disease, hematologic diseases, hirschsprung disease, hydrocephalus, hyperthyroidism, gingivitis, hemophilia, histiocytosis, hyperhidrosis, hypoglycemia, glaucoma, hepatitis and hiv infections.

The disease state may comprise any one or more of hyperoxaluria, hypothyroidism, glycogen storage disease, hepatolenticular degeneration, hodgkin disease, hypersensitivity, immunologic deficiency syndromes, headache, hernia, holt-oram syndrome, hypertension, impotence, congestive heart failure, herpes genitalis, huntington's disease, pulmonary hypertension, incontinence, infertility, leukemia, systemic lupus erythematosus, maduromycosis, mental retardation, inflammation, liver neoplasms, lyme disease, malaria or inborn errors of metabolism.

The disease state state may comprise any one or more of inflammatory bowel diseases, long qt syndrome, lymphangiomyomatosis, measles, migraine, influenza, low back pain, lymphedema, melanoma, mouth abnormalities, latex allergy, obstructive lung diseases, lymphoma, meningitis, mucopolysaccharidoses or leprosy.

The disease state may comprise any one or more of lung neoplasms, macular degeneration, menopause, multiple sclerosis, muscular dystrophy, myofascial pain syndromes, osteoarthritis, pancreatic neoplasms, peptic ulcer, myasthenia gravis, nausea, osteoporosis, panic disorder, persian gulf syndrome, myeloma, acoustic neuroma, otitis media, paraplegia, phenylketonuria, myeloproliferative disorders, nystagmus, ovarian neoplasms or parkinson disease.

The disease state may comprise any one or more of pheochromocytoma, myocardial diseases, opportunistic infections, pain, pars planitis, phobic disorders, myocardial infarction, hereditary optic atrophy, pancreatic diseases, pediculosis, plague, poison ivy dermatitis, prion diseases, reflex sympathetic dystrophy, schizophrenia, shyness, poliomyelitis, prostatic diseases, respiratory tract diseases, scleroderma, sjogren's syndrome or polymyalgia rheumatica.

The disease state may comprise any one or more of prostatic neoplasms, restless legs, scoliosis, skin diseases, postpoliomyelitis syndrome, psoriasis, retinal diseases, scurvy, skin neoplasms, precancerous conditions, rabies, retinoblastoma, sex disorders, sleep disorders, pregnancy, rare diseases, sarcoidosis, sexually transmitted diseases, spasmodic torticollis or spinal cord injuries.

The disease state may comprise any one or more of stuttering, testicular neoplasms, trichotillomania, urinary tract, infections, spinal dystaphism, substance-related disorders, thalassemia, trigeminal neuralgia, urogenital diseases, spinocerebellar degeneration, sudden infant death, thrombosis, tuberculosis, vascular diseases, strabismus, suicide, tinnitus, tuberous sclerosis, virus diseases, post-traumatic stress disorders, syringomyelia, tourette syndrome, turner's syndrome or vision disorders.

The disease state may comprise any one or more of psychological stress, temporomandibular joint dysfunction syndrome, trachoma, urinary incontinence, vomiting, von willebrand's disease, renal osteodystrophy, bacterial infections, digestive system, neoplasms, bone neoplasms, vulvar diseases, ectopic pregnancy, tick-borne diseases, marfan syndrome, aging, williams syndrome, angiogenesis factor, urticaria, sepsis, malabsorption syndromes or wounds and injuries.

The disease state may comprise any one or more of cerebrovascular accident, multiple chemical sensitivity, dizziness, hydronephrosis, yellow fever, neurogenic arthropathy, hepatocellular carcinoma, pleomorphic adenoma, vater's ampulla, meckel's diverticulum, keratoconus skin, warts, sick building syndrome, urologic diseases, ischemic optic neuropathy, common bile duct calculi, otorhinolaryngologic diseases, superior vena cava syndrome, sinusitis, radius fractures, osteitis deformans, trophoblastic neoplasms, chondrosarcoma or reading and carotid stenosis.

The disease state may comprise any one or more of varicose veins, creutzfeldt-jakob syndrome, gallbladder diseases, replacement of joint, vitiligo, nose diseases, environmental illness, megacolon, pneumonia, vestibular diseases, cryptococcosis, herpes zoster, fallopian tube neoplasms, infection, arrhythmia, glucose intolerance, neuroendocrine tumors, scabies.

The disease state may comprise any one or more of alcoholic hepatitis, parasitic diseases, salpingitis, cryptococcal meningitis, intracranial aneurysm, grief, calculi, pigmented nevus, rectal neoplasms, mycoses, hemangioma, colonic neoplasms, hypervitaminosis a, nephrocalcinosis, kidney neoplasms, vitamins, carcinoid tumor, celiac disease, pituitary diseases, brain death, biliary tract diseases or prostatitis.

The disease state may comprise any one or more of iatrogenic disease, gastrointestinal hemorrhage, adenocarcinoma, toxic megacolon, amputees, seborrheic keratosis, osteomyelitis, barrett esophagus, hemorrhage, stomach neoplasms, chickenpox, cholecystitis or chondroma.

The disease state may comprise any one or more of bacterial infections and mycoses, parathyroid neoplasms, spermatic cord torsion, adenoma, lichen planus, anal gland neoplasms, lipoma, tinea pedis, alcoholic liver diseases, neurofibromatoses, lymphatic diseases, elder abuse, eczema, diverticulitis, carcinoma, pancreatitis, amebiasis, pregnancy complications, pyelonephritis, infectious mononucleosis or aneurysm.

In particular, the disease state may comprise a disease such as diabetes, immune disorders, neurodegenerative disorders or cancers or tumours. Thus, the pre- to mature miRNA ratio may be used to monitor the cellular state of a cell of an organism which is suffering, or is prone to suffering, any of the diseases listed above.

It will be appreciated that the cell may be comprised in a tissue, tissue mass, organ or organism. Where this is the case, the precursor to mature miRNA ratio may be used to determine, in addition to the cellular state, the state of the tissue, tissue mass, organ or organism in which the cell is comprised.

### EXOSOMES

Exosomes are secreted microparticles of 30-100 ηm in diameter that were first discovered to be released by reticulocytes (1A). They are essentially phospholipid vesicle containing both protein and RNA.

Exosomes are now known to be secreted by many cell types including those of haematological origin (B cells, dendritic cells, mast cells, T cells and platelets) and of nonhaematological origin (intestinal epithelial cells, Schwann cells, neuronal cells, tumor cells, tumor cell lines (these include murine microglial cells, mesothelioma cells, melanoma cells, ovarian carcinoma cells), and sperm (reviewed (2A)). However, their functions remain poorly understood. Recent studies have indicated that they may play a role in health and diseases such as tumor metastasis, in antigen presentation and in transmitting infectious agents (3A, 4A).

The biogenesis of exosomes proceeds through multiple highly regulated cellular processes that includes the formation of intraluminal vesicles in endosomal multivesicular bodies (MVBs) through the well characterized ESCRT (endosomal sorting complex required for transport)-mediated lateral segregation of the cargo and inward budding of the endosomal membrane (5A).

As such highly regulated cellular processes are generally sensitive to changes in the physiological or pathological state of the cell, it is conceivable that biogenesis of exosome will also be affected in tandem, and that these effects would be manifested in the quantity or composition of the exosomes.

It is generally presumed that at least a fraction of exosomes secreted by cells in our body are released into the circulation. Indeed, exosomes in easily accessible bodily fluids such as blood and urine have been found to originate from many cell types (2A) and it has been shown that circulating exosomal and tumor-derived microRNA profiles in tumor patients were similar (6A) Together, these characteristics make circulating exosomes ideal targets for identifying biomarkers (7A).

As exosomes carry both protein and RNA load, exosomal proteins and RNAs in bodily fluids such as urine and blood are both being evaluated as biomarkers for disease (7A). The proteomics of urinary exosomes had been profiled for potential biomarkers (8A-11A). Exosomal Fetuin-A has been identified as a novel urinary biomarker for detecting acute kidney injury (12A).

### BIOLOGICAL SAMPLE

The methods and compositions described here involve the ratio of precursor to mature miRNA of secreted by a cell in order to monitor its state. Conveniently, the ratio may be determined by taking a biological sample comprising secretions of the cell.

Where the cell is comprised in an organism, the sample may comprise any number of things, including, but not limited to, bodily fluids (including, but not limited to, blood, nasopharyngeal secretions, urine, serum, lymph, saliva, anal and vaginal secretions, perspiration and semen, of virtually any organism.

### PREPARATION OF EXOSOMES

Exosomes may be prepared using a number of methods known to the person skilled in the art. In general, the medium containing cellular secretions may be spun in a centrifuge at 1000 rpm. The supernatant is then concentrated against a ultrofiltration membrane with a 1000 kDa molecular weight cut-off.

Alternatively or in addition, US Patent number 6,899,863 describes a method of preparing membrane vesicles such as exosomes using column chromatography.

Exosomes may also be prepared using the technique described in Sze et al (17A).

This protocol may be briefly summarised as follow: to harvest MSC secretion, 80% of the confluent HuES9.E1 cultures (used as a reference only) are washed with phoshate-buffered saline (PBS), transferred to a chemically defined, serum-free culture medium for an overnight incubation, washed with PBS and cultured in fresh, chemically defined, serum-free culture medium for 3 days.

The conditioned medium which contains MSC secretion is collected, clarified by centrifugation, concentrated 50 times using 100-kDa MW cutoff ultrafiltration membranes (Satorius) and sterilized by filtration through a 220-nm filter.

A further detailed description of methods for isolating and preparing exosomes is set out elsewhere in this document.

### PREPARATION OF miRNAs

miRNAs may be prepared from exosomes collected for example from cellular secretions by any of a number of means known to the person skilled in the art. An example protocol for preparing miRNAs follows:

RNA is isolated from conditioned medium by adding three volumes of Trizol LS (Invitrogen) to one volume of conditioned medium and completing the extraction according to the manufacturer's protocol.

Total RNA and small RNAs are purified using Trizol (Invitrogen) and mirVana miRNA Isolation Kit (AppliedBiosystems), respectively.

RNA is quantitated using Quant-iTTM RiboGreen RNA Assay Kit (Invitrogen) and resolved on 1.5% agarose gel con-taining 8% glyoxal or 15% Novex Tris-borate-EDTA (TBE)-urea gels (Invitrogen).

The separated RNA in the gels are visualized by ethidium bromide (EB) staining.

Alternatively, or in addition, conditioned medium is pre-incubated with equal volume of PBS, cell lysis buffer (Biovision), 40 mM cyclodextrin (Sigma-Aldrich) or 4000 U/ml phospholipase A2 (Sigma-Aldrich) at 37°C for 30 min followed by addition of one-tenth volume of a 1-mg/ml RNase A (Roche). These mixtures are then incubated for another 5 min at 37°C. The RNAs are extracted with Trizol LS and separated on the TBE-urea gels followed by EB staining.

### QUANTITATION OF PRECURSOR MIRNA AND MATURE MIRNA

The Examples describe in detail a protocol for preparing and isolating miRNA that may be used with the methods and compositions described here. Micro RNA for use in the methods and compositions described here may also be prepared using a number of methods known to the person skilled in the art.

For example, micro RNAs may be detected and quantitated using sequencing, Northern hybridisation, a modified Invader® assay, real-time PCR, looped-primer RT-PCR and array hybridisation, etc. These techniques are described in more detail below.

Sequencing of microRNAs in a sample may be done in order to identify and quantitatively decode the entire population of microRNAs in a sample. Such a service is provided by for example LC Sciences (www.lcsciences.com).

Northern hybridization has been used to detect and quantitate miRNAs, as described in Lim, L. et al. 2003. Genes & Development 17:991-1008). Alternatively, RNAse protection may be used for this purpose. Probes for RNase protection and Northern hybridisation may be made through conventional means known to the person skilled in the art. A kit such as the mirVana™ miRNA Probe Construction KitAM1550 (Catalogue number AM1550, Ambion, Applied Biosystems, Austin, Texas, USA) may be used for this purpose.

A modified Invader® assay, as described by Allawi, H. T. et al. 2004. RNA 10:1153-1161 may also be used to detect and quantitate miRNAs. The modified Invader assay is a quantitative, sensitive, and rapid miRNA assay which may be used successfully in the analysis of miRNAs, using as little as 50-100 ng of total cellular RNA or as few as 1,000 lysed cells. This assay is capable of discriminating between miRNAs differing by a single nucleotide and between precursor and mature miRNAs. The Invader miRNA assay, which can be performed in unfractionated detergent lysates, uses fluorescence detection in microtiter plates and requires only 2-3 h incubation time, allowing for parallel analysis of multiple samples in high-throughput screening analyses.

Detection and quantitation of precursor miRNA and mature miRNA may also be achieved through various methods known in the art such as described in US Patent No. 7,601,495 (Chen). Furthermore, the Examples describe in detail a protocol for detecting and quantitating mature and precursor miRNA that may be used with the methods and compositions described here. The method of US Patent No. 7,601,495 (Chen) is suitable for mature miRNA.

A number of kits are also available commercially for detecting and quantitating precursor and mature miRNA and may be used. An example of such a kit is the mirPremier™ microRNA Isolation Kit (Catalogue numbers SNC10 and SNC50, Sigma-Aldrich, St. Louis, Missouri, USA). Another kit that may be used for isolating and preparing miRNA is the RNAqueous®-Micro Kit (Catalogue No AM 1931, Ambion, Applied Biosystems, Austin, Texas, USA).

For example, miRNA detection and quantitation may be performed using the miRCURY LNA™ Universal RT microRNA PCR (Exiqon). This comprises a ready to use panel containing pre-aliquoted microRNA PCR primer sets in 384-well PCR plates. cDNA is added and PCR master mix are added to the wells and real-time PCR profiling of up to 730 microRNAs may be achieved. Either the microRNA Ready-to-Use PCR, Human panel I+II, V1.M (Catalogue number 203601, Exiqon A/S, Vedbaek, Denmark) or the microRNA Ready-to-Use PCR, Human panel I+II, V1.R (Catalogue number 203602, Exiqon A/S, Vedbaek, Denmark) may be used, depending on the detection equipment.

As an alternative, the mirVana™ qRT-PCR miRNA Detection Kit (Catalogue No AM1558, Ambion, Applied Biosystems, Austin, Texas, USA) may be used to detect and quantitate miRNA.

### LOOPED-PRIMER RT-PCR

As an example, a real-time quantitation method termed looped-primer RT-PCR may be used for accurate and sensitive detection of mature miRNAs. This assay is capable of discriminating between two miRNAs that differ by as little as a single nucleotide and between mature miRNAs and their precursors.

In brief, miRNA is purified from cultured cells using mirVana miRNA Isolation Kit (Catalogue number AM1560 or AM1561, Ambion, Applied Biosystems, Austin, Texas, USA).

A real time polymerase chain reaction is then carried out. The assay includes two steps, reverse transcription and polymerase chain reaction. The reverse transcription step comprises stem-loop reverse transcription, in which stem-loop RT primers are annealed to miRNA targets and extended in the presence of reverse transcriptase. RT reactions containing RNA samples, looped-primers, 1X buffer, reverse transcriptase, and RNase inhibitor are incubated for 30 min each, at 16°C and at 42°C.

In the polymerase chain reaction step, real-time PCR is carried out. microRNA-specific forward primer, TaqMan® probe, and reverse primer are used for PCR reactions. Real-time PCR is performed on an Applied Biosystems 7900HT Real-Time PCR System. For data analysis, the copy number per cell is estimated based on the standard curve of synthetic lin-4 miRNA. Quantitation of miRNAs is estimated based on measured CT values.

Northern analysis may also be carried out. Solution hybridization-based Northern analysis may be carried out using mirVanaTM miRNA Detection Kit (Catalogue number AM1552, Ambion, Applied Biosystems, Austin, Texas, USA).

A diagram illustrating this method is shown as Figure 13.

### MICROARRAY ANALYSIS

miRNA detection and quantitation may also be performed by microarray hybridisation. An example protocol which may be used follows:

The RNAs were labeled with Cy5 or Cy3 and hybridized with the preloaded probes.

The data in each experiment are analyzed using standard data analysis that includes the determination of detectable signals, calculation of signal intensities and calculation of differential ratios. First, the background is subtracted. Background is determined using a regression-based background mapping method. The regression is performed on 5-25% of the lowest intensity data points excluding blank spots.

Raw data matrices are then subtracted by the background matrix.

With background subtraction, Cy3/Cy5 channel normalization is carried out using a LOWESS (locally weighted regression) method on the background-subtracted data.

The normalization removes system-related variations, such as sample amount variations, different labeling dyes and signal gain differences of scanners so that biological variations can be faithfully revealed.

A transcript is listed as detectable if it meets at least two conditions: signal intensity higher than 3 (background SD) and spot CV < 0.5. CV is calculated by (SD)/(signal intensity).

When repeating probes are found on an array, a transcript is listed as detectable only if the signals from at least 50% of the repeating probes are above detection level.

The microarray hybridisation service may be performed by a commercial provider, such as LC Sciences (www.lcsciences.com). This service is a genome-wide microRNA (miRNA) expression profiling service using p.Paraflo® technology and proprietary probe design, which enables highly sensitive and specific direct detection of miRNAs. Standard arrays for mature miRNA of all species available in the latest version of the Sanger miRBase database (Release 14.0) are used.

### MICRO RNAs

The micro RNA (miRNA) may be selected from the group consisting of: hsa-miR-424 (miRBase Accession Number: MI0001446), hsa-miR-424* (miRBase Accession Number: MI0001446), hsa-miR-425 (miRBase Accession Number: MI0001448), hsa-miR-425* (miRBase Accession Number: MI0001448), hsa-miR-454 (miRBase Accession Number: MI0003820), hsa-miR-455-3p (miRBase Accession Number: MI0003513), hsa-miR-483-5p (miRBase Accession Number: MI0002467), hsa-miR-484 (miRBase Accession Number: MI0002468), hsa-miR-491-5p (miRBase Accession Number: MI0003126), hsa-miR-503 (miRBase Accession Number: MI0003188), hsa-miR-505* (miRBase Accession Number: MI0003190), hsa-miR-532-5p (miRBase Accession Number: MI0003205), hsa-miR-574-15-3p (miRBase Accession Number: MI0003581), hsa-miR-584 (miRBase Accession Number: MI0003591), hsa-miR-612 (miRBase Accession Number: MI0003625), hsa-miR-625 (miRBase Accession Number: MI0003639), hsa-miR-629 (miRBase Accession Number: MI0003643), hsa-miR-708 (miRBase Accession Number: MI0005543), hsa-miR-744 (miRBase Accession Number: MI0005559), hsa-mir-766 (miRBase Accession Number: MI0003836), hsa-miR-768-3p (miRBase Accession Number: MI0005117), hsa-miR-768-5p (miRBase Accession Number: MI0005117), hsa-miR-769-5p (miRBase Accession Number: MI0003834), hsa-miR-877 (miRBase Accession Number: MI0005561), hsa-miR-92, hsa-mir-92a-1 (miRBase Accession Number: MI0000093), hsa-mir-92a-2 (miRBase Accession Number: MI0000094), hsa-miR-92b (miRBase Accession Number: MI0003560), hsa-miR-93 (miRBase Accession Number: MI0000095), hsa-miR-98 (miRBase Accession Number: MI0000100), hsa-miR-99a (miRBase Accession Number: MI0000101), hsa-miR-99b (miRBase Accession Number: MI0000746), hsa-let-7a, hsa-let-7a-1 (miRBase Accession Number: MI0000060), hsa-let-7a-2 (miRBase Accession Number: MI0000061), hsa-let-7a-3 (miRBase Accession Number: MI0000062), hsa-miR-151-3p (miRBase Accession Number: MI0000809), hsa-miR-23a (miRBase Accession Number: MI0000079), hsa-let-7b (miRBase Accession Number: MI0000063), hsa-miR-151-5p (miRBase Accession Number: MI0000809), hsa-miR-23a* (miRBase Accession Number: MI0000079), hsa-let-7b* (miRBase Accession Number: MI0000063), hsa-miR-152 (miRBase Accession Number: MI0000462), hsa-mir-23b (miRBase Accession Number: MI0000439), hsa-let-7c (miRBase Accession Number: MI0000064), hsa-miR-155 (miRBase Accession Number: MI0000681), hsa-miR-24, hsa-mir-24-1 (miRBase Accession Number: MI0000080), hsa-let-7d (miRBase Accession Number: MI0000065), hsa-miR-15a (miRBase Accession Number: MI0000069), hsa-miR-24-2* (miRBase Accession Number: MI0000081), hsa- 20, let-7d*, hsa-let-7d (miRBase Accession Number: MI0000065), hsa-miR-15b (miRBase Accession Number: MI0000438), hsa-miR-25 (miRBase Accession Number: MI0000082), hsa-let-7e (miRBase Accession Number: MI0000066), hsa-miR-16, hsa-mir-16-1 (miRBase Accession Number: MI0000070), hsa-mir-16-2 (miRBase Accession Number: MI0000115), hsa-miR-26a, hsamir-26a-1 (miRBase Accession Number: MI0000083), hsa-mir-26a-2 (miRBase Accession Number: MI0000750), hsa-let-7f, hsa-let-7f1 (miRBase Accession Number: MI0000067), hsa-let-7f-2 (miRBase Accession Number: MI0000068), hsa-miR-17 (miRBase Accession Number: MI0000071), hsa-miR-26b (miRBase Accession Number: MI0000084), hsa-let-7g (miRBase Accession Number: MI0000433), hsa-miR-181a, hsa-mir-181a-2 (miRBase Accession Number: MI0000269), hsa-miR-27a (miRBase Accession Number: MI0000085), hsa-let-7i (miRBase Accession Number: MI0000434), hsa-miR-181a*, hsa-mir-181a-1 (miRBase Accession Number: MI0000289), has-miR-27b (miRBase Accession Number: MI0000440), hsa-miR-100 (miRBase Accession Number: MI0000102), hsa-miR-181a-2* (miRBase Accession Number: MI0000269), hsa-miR-27b* (miRBase Accession Number: MI0000440), hsa-miR-103, hsa-mir-103-2 (miRBase Accession Number: MI0000108), hsamir-103-1 (miRBase Accession Number: MI0000109), hsa-mir-103-1-as 3 (miRBase Accession Number: MI0007261), hsa-mir-103-2-as (miRBase Accession Number: MI0007261), hsa-miR-181 b, hsa-mir-181b-1 (miRBase Accession Number: MI0000270), hsamir-181b-2 (miRBase Accession Number: MI0000683), hsa-miR-28-3p (miRBase Accession Number: MI0000086), hsa-miR-106a (miRBase Accession Number: MI0000113), hsa-miR-181c (miRBase Accession Number: MI0000271), hsa-miR-28-5p (miRBase Accession Number: MI0000086), hsa-miR-572 (miRBase Accession Number: MI0003579), hsa-miR-106b (miRBase Accession Number: MI0000734), hsa-miR-181d (miRBase Accession Number: MI0003139), hsa-miR-296-5p (miRBase Accession Number: MI0000747), hsa-miR-107 (miRBase Accession Number: MI0000114), hsa-miR-185 (miRBase Accession Number: MI0000482), hsa-miR-29a (miRBase Accession Number: MI0000087), hsa-miR-574-5p (miRBase Accession Number: MI0003581), hsa- 25miR-10a, hsa-miR-186 (miRBase Accession Number: MI0000483), hsa-miR-29c (miRBase Accession Number: MI0000735), hsa-miR-575 (miRBase Accession Number: MI0003582), hsa-miR-122 (miRBase Accession Number: MI0000442), hsa-miR-187* (miRBase Accession Number: MI0000274), hsa-miR-30a (miRBase Accession Number: MI0000088), hsa-miR-1224-Sp (miRBase Accession Number: MI0003764), hsa-miR-18a (miRBase Accession Number: MI0000072), hsa-miR-30a* (miRBase Accession Number: MI0000088), hsa-miR-1228 (miRBase Accession Number: MI0006318), hsa-miR-18b (miRBase Accession Number: MI0001518), hsa-miR-30b (miRBase Accession Number: MI0000441), hsa-miR-1234 (miRBase Accession Number: MI0006324), hsa-miR-191 (miRBase Accession Number: MI0000465), hsa-miR-30c, hsa-mir-30c-2 (miRBase Accession Number: MI0000254), hsa-mir-30c-1 (miRBase Accession Number: MI0000736), hsa-miR-1237 (miRBase Accession Number: MI0006327), hsa-miR-191* (miRBase Accession Number: MI0000465), hsa-miR-30d (miRBase Accession Number: MI0000255), hsa-miR-638 (miRBase Accession Number: MI0003653), hsa-miR-1238 (miRBase Accession Number: MI0006328), hsa-miR-192 (miRBase Accession Number: MI0000234), hsa-miR-30e (miRBase Accession Number: MI0000749), hsa-miR-663 (miRBase Accession Number: MI0003672), hsa-miR-124, hsa-mir-124-1 (miRBase Accession Number: MI0000443), hsa-mir-124-2 (miRBase Accession Number: MI0000444), hsa-mir-124-3 (miRBase Accession Number: MI0000445), hsa-mir-124b (miRBase Accession Number: MI0000260), hsa-miR-193a-5p (miRBase Accession Number: MI0000487), hsa-miR-30e* (miRBase Accession Number: MI0000749), hsa-miR-671-5p (miRBase Accession Number: MI0003760), hsa-miR-125a-3p (miRBase Accession Number: MI0000469), hsa-miR-195 (miRBase Accession Number: MI0000489), hsa-miR-31 (miRBase Accession Number: MI0000089), hsa-miR-30, hsa-mir-30c-2 (miRBase Accession Number: MI0000254), hsa-mir-30c-1 (miRBase Accession Number: MI0000736), hsa-miR-125a-5p (miRBase Accession Number: MI0000469), hsa-miR-197 (miRBase Accession Number: MI0000239), hsa-miR-31* (miRBase Accession Number: MI0000089), hsa-miR-125b, hsa-mir-125b-1 (miRBase Accession Number: MI0000446), hsa-mir-125b-2 (miRBase Accession Number: MI0000470), hsa-miR-198 (miRBase Accession Number: MI0000240), hsa-miR-320, hsa-mir-320a (miRBase Accession Number: MI0000542), hsa-mir-320b-1 (miRBase Accession Number: MI0003776), hsa-mir-320c-1 (miRBase Accession Number: MI0003778), hsa-mir-320b-2 (miRBase Accession Number: MI0003839), hsa-mir-320d-1 (miRBase Accession Number: MI0008190), hsa-mir-320c-2 (miRBase Accession Number: MI0008191), hsa-mir-320d-2 (miRBase Accession Number: MI0008192), hsa-mir-320e (miRBase Accession Number: MI0014234), hsa-miR-765 (miRBase Accession Number: MI0005116), hsa-miR-126 (miRBase Accession Number: MI0000471), hsa-miR-199a-3p, hsa-miR-199a-3p (miRBase Accession Number: MI0000242), hsa-mir-199a-2 (miRBase Accession Number: MI0000281), hsa-miR-324-5p (miRBase Accession Number: MI0000813), hsa-miR-128, hsa-mir-128-1 (miRBase Accession Number: MI0000447), hsa-mir-128-2 (miRBase Accession Number: MI0000727), hsa-miR-199a875p, hsa-miR-328 (miRBase Accession Number: MI0000804), hsa-miR-130a (miRBase Accession Number: MI0000448), hsa-miR-199b-5p (miRBase Accession Number: MI0000282), hsa-miR-330-3p (miRBase Accession Number: MI0000803), hsa-miR-130b (miRBase Accession Number: MI0000748), hsa-miR-19b, hsa-mir-19b-1 (miRBase Accession Number: MI0000074), hsa-mir-19b-2 (miRBase Accession Number: MI0000075), hsa-miR-331-3p (miRBase Accession Number: MI0000812), hsa-miR-132 (miRBase Accession Number: MI0000449), hsa-miR-20a (miRBase Accession Number: MI0000076), hsa-miR-335 (miRBase Accession Number: MI0000816), hsa-miR-137 (miRBase Accession Number: MI0000454), hsa-miR-20b (miRBase Accession Number: MI0001519), hsa-miR-342-3p (miRBase Accession Number: MI0000805), hsa-miR-923 (miRBase Accession Number: MI0005715), hsa-miR-140-3p (miRBase Accession Number: MI0000456), hsa-miR-21 (miRBase Accession Number: MI0000077), hsa-miR-345 (miRBase Accession Number: MI0000825), hsa-miR-143 (miRBase Accession Number: MI0000459), hsa-miR-210 (miRBase Accession Number: MI0000286), hsa-miR-34a (miRBase Accession Number: MI0000268), hsa-miR-145 (miRBase Accession Number: MI0000461), hsa-miR-212 (miRBase Accession Number: MI0000288), hsa-miR-34a* (miRBase Accession Number: MI0000268), hsa-miR-145* (miRBase Accession Number: MI0000461), hsa-miR-214 (miRBase Accession Number: MI0000290), hsa-miR-361-5p (miRBase Accession Number: MI0000760), hsa-miR-933 (miRBase Accession Number: MI0005755), hsa-miR-146a (miRBase Accession Number: MI0000477), hsa-miR-22 (miRBase Accession Number: MI0000078), hsa-miR-362-3p, 5, hsa-miR-940 (miRBase Accession Number: MI0005762), hsa-miR-146b-5p (miRBase Accession Number: MI0003129), hsa-miR-22* (miRBase Accession Number: MI0000078), hsa-miR-362-5p (miRBase Accession Number: MI0000762), hsa-miR-148b (miRBase Accession Number: MI0000811), hsa-miR-221 (miRBase Accession Number: MI0000298), hsa-miR-365, hsa-mir-365-1 (miRBase Accession Number: MI0000767), hsa-mir-365-2 (miRBase Accession Number: MI0000769), hsa-miR-149 (miRBase Accession Number: MI0000478), hsa-miR-221* (miRBase Accession Number: MI0000298), hsa-miR-374b (miRBase Accession Number: MI0005566), hsa-miR-149* (miRBase Accession Number: MI0000478), hsa-miR-222 (miRBase Accession Number: MI0000299), hsa-miR-421 (miRBase Accession Number: MI0003685), hsa-miR-150* (miRBase Accession Number: MI0000479), hsa-miR-222* and hsa-miR-423-5p (miRBase Accession Number: MI0001445).

The miRBase database is a searchable database of published miRNA sequences and annotation and may be accessed at http://www.mirbase.org. miRBase is described in the following articles miRBase: microRNA sequences, targets and gene nomenclature. Griffiths-Jones S, Grocock RJ, van Dongen S, Bateman A, Enright AJ. NAR, 2006, 34, Database Issue, D140-D144; The microRNA Registry. Griffiths-Jones S. NAR, 2004, 32, Database Issue, D109-D111. The following publication provides guidelines on miRNA annotation: A uniform system for microRNA annotation. Ambros V, Bartel B, Bartel DP, Burge CB, Carrington JC, Chen X, Dreyfuss G, Eddy SR, Griffiths-Jones S, Marshall M, Matzke M, Ruvkun G, Tuschl T. RNA, 2003, 9(3), 277-279

### MICROARRAYS

We describe sets comprising nucleic acid sequences capable of specifically binding to, and distinguishing between, precursor and mature forms of each of the miRNAs listed above.

The sets of nucleic acids sequences may conveniently be arranged in an ordered manner. They may be attached to a substrate. For example, a microarray comprising such sets of nucleic acids may be provided.

The microarray may comprise a set of primers or probes capable of hybridising to abnd detecting precursor forms of the microRNA, as well as a set of primers or probes capable of hybridising to and detecting mature forms of the microRNA.

The construction of such a microarray may make use of the list of miRNA transcripts listed in Sanger miRBase Release 10.1.

### PRE- TO MATURE MIRNA RATIO

The pre- to mature miRNA ratio may be simply calculated as a ratio of the quantity of the precursor form of the miRNA species against the mature form of the miRNA species.

Alternatively, or in addition, the ratio of precursor form to mature form of an miRNA species known not to be changed as a result of the change in cellular state may be used as an internal control.

Thus, instead of, or in addition to, monitoring the change in the pre- to mature miRNA ratio of an miRNA species, the ratio of a first ratio against a second ratio may be used for monitoring purposes. Here, the first ratio is the ratio of precursor form to mature form of an miRNA species known to be changed as a result of the change in cellular state and the second ratio being the ratio of precursor form to mature form of an miRNA species known *not* to be changed as a result of the change in cellular state.

### PRE- TO MATURE MIRNA RATIO PROFILE

Alternatively or in addition to determining the ratio of precursor to mature miRNA of a single miRNA species, a profile comprising a plurality of pre- to mature miRNA ratios for a plurality of selected miRNA species, each indicative of the state of the cell may also be established. Changes to such a profile may be monitored as a means to monitor the state of a cell. The profile may be established by any means known in the art, such as by hybridisation to an array comprising a plurality of probes capable of binding to and distinguishing between precursor and mature forms of the plurality of the selected miRNA species.

### OTHER USES

We describe a method for detecting a change in state of a cell. The method comprises detecting a change in a pre- to mature miRNA ratio of the cell (or a profile comprising such a ratio), in which such a change indicates a change in state of the cell.

We describe a method for establishing that a cell is in a particular state. The method comprises comparing a pre- to mature miRNA ratio of the cell (or a profile comprising such a ratio) with a pre- to mature miRNA ratio (or a profile comprising such a ratio) of a cell known to be in that particular state.

We describe a method of monitoring the state of an organism. The method comprises obtaining a sample from or of that organism, establishing the pre- to mature mi-RNA ratio of an miRNA species in the sample, and establishing the state of the organism from the pre- to mature miRNA ratio thereby obtained.

We describe a method of detecting a disease in an organism. The method comprises obtaining a sample from or of that organism, establishing the pre- to mature mi-RNA ratio of an miRNA species in the sample and comparing the pre- to mature miRNA ratio thereby obtained with a pre- to mature miRNA ratio of a sample known to be of or from a diseased organism.

We describe a method of treatment or prevention of a disease in an organism. The method comprises detecting a disease in an organism as described above and administering a treatment for that disease to the organism.

### EXAMPLES

Examples 1 to 15 describe the oncogenic transformation of mesenchymal stem cells. Examples 16 to 25 describe the use of a pre-/mature miRNA ratio as a marker to monitor the state of a cell.

### Example 1 (incorporated herein as a reference example). Materials and Methods - Oncogenic transformation of huES9.E1 MSC

The previously described human ESC-derived huES9.E1 MSCs was infected at passage 21 or p16 with lentivirus carrying either a c-myc gene or a GFP gene to generate two types of transfected cells, cmyc-MSC and GFP-MSC, respectively. The c-myc cDNA was amplified from pMXs-hc-MYC [32] using primers PTDmyc (5' GAA TTC GAA TGC CCC TCA ACG TTA GC 3') and PTDmyca (5' CTC GAG CGC ACA AGA GTT CCG TAG C 3'). The amplified fragment was inserted into pDrive vector and sequenced. The c-myc fragment was digested and cloned as a Xho1(filled)/EcoR1 fragment into a Sma1/EcoR1 digested pLVX-puro vector with compatible ends (Clontech, www.clontech.com). Lentiviral particles were produced using Lenti-X HT Packaging System (Clontech, www.clontech.com) according to the manufacturer's protocol. Viral titer was determined using by a Lenti-X^{™} qRT-PCR titration kit (Clontech, www.clontech.com). The HuES9.E1 hESC-MSCs [24] that were infected at passage 21 were plated at 10⁶ cells per 10 cm dish and infected with viruses at a MOI=5 in the presence of 4 µg/ml polybrene for overnight. The next day, culture medium was replaced with fresh medium. Selection began 48 hours after infection by replacing the culture medium with one containing puromycin (2 µg/ml). After three days of selection, cells were expanded as per human ESC-derived huES9.E1 MSCs and these cells were pooled to generate the E1-myc 21.1 line. For the HuES9.E1 hESC-MSCs [24] that were infected at passage 16, cells were plated on three wells of a six-well plate. Viral infection and subsequent drug selection for each well were performed as described above. Clonal lines from each of the three independently infected cell cultures were derived by limiting dilution. When the cloned cells were expanded to 10⁷ cells (or a confluent 15 cm culture dish), the cells were designated p1. Three clonal lines were generated and named E1-myc 16.1, E1-myc 16.2 and E1-myc 16.3 lines, respectively. Integration of the c-myc or GFP transgene was confirmed by amplifying genomic DNA using specific primers for exon2 and exon3 of c-myc respectively: 5'-GCCCCTGGTGCTCCATGAGGAGACACC'-3' and 5'-ACATTCTCCTCGGTGTCCGAGG-3' using the following PCR conditions: one cycle of 94, 2min; 32 cycles of 94°C, 15s; 60 °C, 30s; 72 °C, 90s and one cycle of 72 °C, 5min. The PCR products were resolved on a 1% agarose gel.

Differentiation of the myc-MSCs to adipocytes, chondrocytes and osteocytes was performed using adipogenic, chondrogenic and osteogenic hMSC Differentiation BulletKits, respectively (Lonza, Walkersville, MD) as per manufacturer' instructions. Karyotyping by G-banding was performed by the Cytogenetics Laboratory, KKH.

### Example 2. Materials and Methods - Telomerase activity

Relative telomerase activity was measured by SYBR® Green real time quantitative telomeric repeat amplification protocol assay using a modified method as described by Wege H. et al [33]. Briefly, 3 million cells were harvested and cell lysate was prepared using a commercially available mammalian cell extraction kit (Cat K269-500-1, BioVision, www.biovision.com). The composition of the reagents for the PCR amplification was 1 µg of protein cell lysate, 10 µl of 2 X SYBR Green Super Mix (Cat 170-8880, BioRad, Singapore) with 0.1µg of TS primer (5'-AATCCGTCGAGCAGACTT-3'), 0.1 µg of ACX primer (5'-GCGCGG[CTTACC]3CTAACC-3') and 10mM EGTA in a total volume of 25µl. The reaction was first incubated at 25°C for 20 min to allow the telomerase in the cell lysate to elongate the TS primers followed by 2 min incubation at 95°C to inactivate telomerase activity and denature the primers. The telomerase product was amplified by PCR for 40 cycles of 95°C, 30s; 60°C, 90s. The relative telomerase activity was assessed against that of HEK293 cells using the threshold cycle number (or Ct value) for 1 µg protein cell lysate.

### Example 3. Materials and Methods - Rate of cell cycling

To assess cell cycle rate, 2×10⁷ cells were pre-labeled with 2ml of 10µM CFDA (Molecular Probe, Eugene, Or) in PBS at 37°C for 15 minutes, cultured for 24 hours and then replated at 5×104 cells per well in 6-well coated with gelatin. At 0, 24, 48, and 72 hours, cells from duplicate wells were harvested, and fixed in 2% paraformaldehyde, and analyzed on a FACSplus (Becton Dickinson; San Jose, CA). The number of cell cycles per 24 hours was calculated assuming that each halving of cellular fluorescence represented one cell division. Therefore, the number of cell cycles per 24 hours (n) was calculated as n=lg(F-Fn)/lg2 where F is initial average cellular fluorescence and Fn is the average cellular fluorescence after 24 hours. The number of cell cycles was then plotted against time to derive the average time per cell cycle.

### Example 4. Materials and Methods - Surface antigen analysis

Expression of cell surface antigens on Hus9E1 and E1myc MSCs was analyzed using flow cytometry. The cells were tryspinized for 5 minutes, centrifuged, resuspended in culture media and incubated in a bacterial culture dish for 1 hour in a 37 °C, 5% CO₂ incubator. The cells were then collected, centrifuged, washed in 2% FBS. 2.5×10⁵ cells were then incubated with each of the following conjugated monoclonal antibodies: CD29-PE, CD44-FITC, CD49a-PE, CD49e-PE, CD105-FITC, CD166-PE, CD73-FITC, CD34-FITC, CD45-FITC, HLADR-PE, and MHC1-PE (PharMingen, San Diego, CA) for 60 min on ice. After incubation, cells were washed and resuspended in 2% FBS. Nonspecific fluorescence was determined by incubation of similar cell aliquots with isotype-matched mouse monoclonal antibodies (PharMingen, San Diego, CA). Data were analyzed by collecting 20,000 events on a BD FACSCalibur™ Flow Cytometer (BD Biosciences, San Jose, CA) instrument using CELLQuest software.

### Example 5. Materials and Methods - Analysis of RNA by qRT-PCR

Relative myc transcript level in the cells was determined using qRT-PCR. 20ηg RNA from GFP-MSC (p17) and E1myc-MSC (p24, p27, p29) was converted to cDNA using a High-Capacity cDNA Reverse Transcription Kit (ABI, USA). The cDNA was then amplified by one cycle of 94, 10 min; 40 cycles of 94°C, 15s; 60°C, 60s and one cycle of 95°C, 15s, 60°C 60s, 95°C, 15s with primer sets specific for either myc or actin transcript on the StepOnePlus Real-Time PCR system (Applied Biosystems, www.appliedbiosystems.com.sg). The myc-specific primer set is 5'-CCCGCCCCTGTCCCCTAGCCG-3' and 5'-AGAAGGGTGTGACCGCAACGTAG3'.

### Example 6 (incorporated herein as a reference example). Materials and Methods - Illumina gene chip analysis

Total RNA was prepared in technical triplicates from different passages of myc-MSCs. RNA was prepared from E1-myc 16.3 cells at p4, p7, and p8, and from the parental HuES9-E1 MSCs at p15 and p16 using Illumina® TotalPrep RNA Amplification Kit. 500µg RNA was converted to biotinylated cRNA using the Illumina RNA Amplification Kit (Ambion, Inc., Austin, TX) according to the manufacturer's directions. 750ng of the biotinylated cRNA were hybridized to the Sentrix HumanRef-8 Expression BeadChip Version 3 (Illumina, Inc., San Diego, CA), washing and scanning were performed according to the Illumina BeadStation 500x manual. The data were analyzed using Genespring GX 10. Quantile normalization was performed by a shift to 75^{th} percentile, and the normalized data were baseline transformed to the median of all samples.

### Example 7. Materials and Methods - Western blot hybridization

Proteins were separated on 4-12% SDS-polyacrylamide gels, electroblotted onto a nitrocellulose membrane, blocked, incubated with the primary antibodies against human CD9, ACTIN (Cruz Biotechnology, Santa Cruz, CA, USA), and MYC (Abcam, Cambridge, MA). The blot was then incubated with a horseradish peroxidase-coupled anti-mouse IgG (Santa Cruz Biotechnology, Santa Cruz, CA) followed by HRP-enhanced chemiluminescent substrate (Thermo Fisher Scientific Inc, Waltham, MA) and then exposed to an X-ray film.

### Example 8. Materials and Methods - HPLC purification of microparticles

The instrument setup consisted of a liquid chromatography system with a binary pump, an auto injector, a thermostated column oven and a UV-visible detector operated by the Class VP software from Shimadzu Corporation (Kyoto, Japan). The Chromatography columns used were TSK Guard column SWXL, 6 x 40 mm and TSK gel G4000 SWXL, 7.8 x 300 mm from Tosoh Corporation (Tokyo, Japan).The following detectors, Dawn 8 (light scattering), Optilab (refractive index) and QELS (dynamic light scattering) were connected in series following the UV-visible detector. The last three detectors were from Wyatt Technology Corporation (California, USA) and were operated by the ASTRA software. The components of the sample were separated by size exclusion i.e. the larger molecules will elute before the smaller molecules. The eluent buffer used was 20 mM phosphate buffer with 150 mM of NaCl at pH 7.2. This buffer was filtered through a pore size of 0.1 µm and degassed for 15 minutes before use. The chromatography system was equilibrated at a flow rate of 0.5 ml/min until the signal in Dawn 8 stabilized at around 0.3 detector voltage units. The UV-visible detector was set at 220 ηm and the column was oven equilibrated to 25°C. The elution mode was isocratic and the run time was 40 minutes. The volume of sample injected ranged from 50 to 100 µl. The hydrodynamic radius, Rh was computed by the QELS and Dawn 8 detectors. The highest count rate (Hz) at the peak apex was taken as the Rh. Peaks of the separated components visualized at 220 ηm were collected as fractions for further characterization studies.

### Example 9. Materials and Methods - Testing secretion for cardioprotection

The secretion was prepared by growing the transformed MSCs in a chemically defined serum free culture medium for three days as previously described [34]. Briefly, cells at p12 were first expanded in serum-containing culture medium as described above. At p 15, 80% confluent cell cultures were washed three times with PBS and then incubated in a chemically defined medium consisting of DMEM without phenol red (Invitrogen Corporation, Carlsbad, CA) and supplemented with insulin, transferrin, and selenoprotein (ITS) (Invitrogen Corporation, Carlsbad, CA), 5 ng/ml FGF2 (Invitrogen Corporation, Carlsbad, CA), 5 ng/ml PDGF AB (Peprotech, Rocky Hill, NJ), glutamine-penicillin-streptomycin, and β-mercaptoethanol overnight. The cell culture was then washed with PBS and replaced with fresh chemically defined medium for another three days to produce the conditioned medium. This CM was collected and clarified by centrifugation at 500 x g. The clarified CM concentrated 50 times by reducing its volume by a factor of 50 using a tangential flow filtration system with membrane MW cut-off of 100 kDa (Satorius, Goettingen, Germany). The use of a membrane MW cut-off of 100 kDa allow molecules with MW of less 100 kDa to pass through the filter resulting a preferential loss of molecules less than 100 kDa. The concentrated CM was then sterilized by filtration through a 220 nm filter.

The CM was tested in a mouse model of ischemia and reperfusion injury. MI was induced by 30 minutes left coronary artery (LCA) occlusion and subsequent reperfusion. Five minutes before reperfusion, mice were intravenously infused with 200 µl saline solution of 0.3µg exosome protein purified from culture medium conditioned by myc-MSCs. Control animals were infused with 200 µl saline. After 24 hours reperfusion, infarct size (IS) as a percentage of the area at risk (AAR) was assessed using Evans' blue dye injection and TTC staining as described previously [27].

### Example 10. Materials and Methods - Statistical analysis

Two-way ANOVA with post-hoc Dunnett was used to test the difference in infarct size between groups. Correlation coefficient of each pairs of array was assessed using Pearson correlation test.

### Example 11 (incorporated herein as a reference example). Results - Transforming huES9.E1 MSC cultures

HuES9.E1 MSCs at passage 21 were infected with either GFP- or myc-containing lentivirus. The infected cultures were placed under the puromycin selection for three days. Surviving cells were pooled. PCR amplification of genomic DNA demonstrated that the myc transgene was successfully integrated in the genome (Figure 1A). Unlike the myc-transfected cells which was pooled to form the E1-myc 21.1 line, the GFP-transfected cells progressed into senescence with decreasing rate of proliferation and acquiring a much flattened, spreading morphology (Figure 1B) and could not be propagated beyond p26. The myc-transformed cells expressed a 100 fold increase in c-myc transcript level relative to the GFP-transfected cells (GFP-MSCs) (Figure 1C) and higher telomerase activity (Figure 1D). To generate independently cloned lines, three HuES9.E1 MSC cultures at p16 were independently infected and placed under puromycin drug selection. The surviving cell cultures were cloned by limiting dilution to generate three lines, E1-myc 16.1, E1-myc 16.2 and E1-myc 16.3 lines, respectively. The lines were karyotyped by G-banding. The cell morphology of all three cell lines was similar to that of E1-myc 21.1 line. Only E1-myc 16.3 line had the parental karyotype of 46 XX with a pericentric inversion of chromosomal 9 between p11 and q13 in 20/20 metaphases [24] and was therefore used in all the subsequent experiments (Figure 1E). In contrast to their parental cells, the myc-transformed cells proliferated faster with a population doubling time of 13 hours versus a population doubling time of 19 hours in untransformed MSCs. The average cell cycle time was decreased from 19 hours to 11 hours using CFDA cell labelling as previously described [35] (Figure 1F). The transformed cells effectively bypassed senescence and continued to maintain their proliferative rates for at least another 20 passages. The transformed cells were smaller and rounder in shape with prominent nuclei. These cells also lost contact inhibition resulting in the formation of cell clusters (Figure 1C). Consistent with increased proliferation, the cells had higher levels of telomerase activity than GFP-transfected or non transfected cells (Figure 1E).

### Example 12. Results - Assessment of myc-MSCs

The myc-MSC culture were assessed according to the ISCT minimal criteria for the definition of human MSCs [31]. As observed earlier (Figure 1C), the culture did not adhere to plastic culture dishes as well as their untransformed MSCs especially at confluency when the cells started to form clusters instead of adhering to the plastic dish as a monolayer. The surface antigen profile of the myc-transformed cells was quite similar to that of their parental cells except in their negative expression of MHC I. The cells were CD29⁺, CD44⁺, CD49a⁺ CD49e⁺, CD73⁺ CD105⁺, CD166⁺, MHC I , HLA-DR , CD34 and CD45 (Figure 2). The *in vitro* differentiation potential of both polyclonal E1-myc 21.1 and monoclonal E1-myc 16.3 cell lines was next examined (Figure 3). Both cell lines differentiated readily into chondrocytes and osteocytes (Figure 3A, Figure 3B) but not adipocytes. The induction of adipogenesis in MSCs required 4 cycles of 6 days' treatment protocol consisting of 3 days' exposure to induction medium and 3 days' exposure to maintenance medium. We observed that exposure to the induction medium induced death in the myc-transformed cells but not the untransformed parental cells (Figure 3C). These observations suggested that myc-transformed cells cannot undergo adipogenic differentiation. Together, these observations demonstrated that unlike a previous report where myc transformation was observed not to alter the fundamental characteristics of MSCs [29], we observed here that c-myc transformation affected a defining property of MSCs i.e. the potential to undergo adipogenesis

### Example 13 (incorporated herein as a reference example). Results - Gene expression profile

Genome-wide gene expression profiling of myc-transformed MSCs and their parental MSCs by microarray hybridisation was performed to assess the relatedness between the cell types. Microarray hybridization were performed in duplicate on Sentrix Human Ref-8 Expression BeadChip version 3 (Illumina, Inc., San Diego, CA) using RNA from E1-myc 16.3 MSCs at p4, p7, and p8, and from the parental HuES9-E1 MSCs at p15 and p16. The gene expression profile among different passages of E1-myc 16.3 MSCs or among different passages of the parental HuES9-E1 MSCs was highly similar with a correlation value greater than 0.98. The corrrelation coefficient, r² between E1-myc 16.1 MSCs and parental HuES9-E1 MSCs was also relatively high at 0.92 (Figure 4A). A total of 161 genes was upregulated and 226 genes downregulated at least 2 fold in E1-myc 16.1 MSCs suggesting that there were changes in gene expression after myc transformation. These differentially expressed genes were functionally clustered by PANTHER (Protein ANalysis THrough Evolutionary Relationships) [36,37] in which the observed frequency of genes for each biological process in each gene set was compared with the reference frequency which, in this case is the frequency of genes for that biological process in the NCBI database. There were 11 over-represented biological processes for the 161 upregulated genes namely, metabolic process, nucleobase, nucleoside, nucleotide and nucleic acid metabolic process, primary metabolic process, amino acid transport, sulfur metabolic process, organelle organization, mitochondrion organization, peroxisomal transport, cellular amino acid and derivative metabolic process, polyphosphate catabolic process, and protein metabolic process. There were 4 under-represented processes: vesicle-mediated transport, exocytosis, cell surface receptor linked signal transduction, and immune system process (Figure 4B). In the 226 downregulated genes, there were 37 over- and one under-represented biological processes (Figure 4C).

For the up-regulated genes, many of the associated over-represented processes were generally important for increasing cell mass or anabolic activity for cell division and were consistent with the observed increased cell proliferation activity. The under-represented processes, namely vesicle-mediated transport, exocytosis suggested that exosome production might not be affected. For the down-regulated genes, the 37 over-represented processes could be broadly classified into processes that are associated with adhesion, differentiation, communication, immune response, cell death and metabolism. These processes were also consistent with some of our observations of the myc-transduced MSCs, namely reduced adherence to plastic, loss of adipogenic differentiation potential and loss of MHC I expression.

### Example 14. Results - Cardioprotective activity of secretion

The loss of adipogenic potential in myc-transformed MSC indicated that other aspects of the characteristics of ESC-derived MSCs such as the production of therapeutic exosomes might also be compromised by the transformation. We had previously demonstrated that exosomes secreted by ESC-derived MSCs was protective in a mouse model of myocardial ischemia and reperfusion injury [27]. To test if this aspect was compromised, the transformed cells were grown in a chemically defined medium, the conditioned culture medium (CM) harvested and exosomes were purified as previously described [34,38]. Despite increased c-myc transcript and protein levels in the transformed, c-myc protein was not detectable in the CM and purified exosomes (Figure 5A). The HPLC protein profile of the CM was similar to that of CM from untransformed MSCs [38] (Figure 5B) with the fastest eluting fraction having a retention time of about 12 minutes. Dynamic light scattering analysis of this peak revealed the presence of particles that were within a hydrodynamic radius range of 50-65 ηm. In a typical run, we routinely purified about 1.5 mg of exosomes per liter of conditioned medium. HPLC-purified exosomes from either E1-myc 21.1 or E1-myc 16.3 was administered to the mouse model of myocardial ischemia-reperfusion injury at a dosage of 0.3 or 0.4 µg per mouse respectively (Figure 5C). The area at risk (AAR) as a percentage of left ventricular (LV) area in E1-myc 21.1 exosome, E1-myc 16.3 exosome or the saline-treated control group was similar at 39.1 ±3.4%, 41.7±4.7% and 40.8 ±11.8%, respectively. The relative infarct size (IS/AAR) in mice treated with E1-myc 21.1 exosome or E1-myc 16.3 exosome was 23.4 ± 8.2%, and 22.6 ± 4.5%, respectively and their relative infarct sizes were significantly lower than the relative infarct size of 38.5 ±5.6% in saline-treated mice (p<0.001 and p<0.002, respectively).

### Example 15. Discussion

This report describes the transformation of human ESC-derived MSCs by over-expression of a c-myc gene. This transformation enabled the cells to bypass senescence, increase telomerase activity and enhance proliferation. Generally, genome-wide gene expression between the transformed cells versus their parental cells was conserved with a correlation coefficient of 0.92 The transformed cells also have the characteristic surface antigen profile: CD29⁺, CD44⁺, CD49a⁺ CD49e⁺, CD105⁺, CD166⁺, MHC I⁻, HLA-DR⁻, CD34⁻ and CD45⁻. Although the transformed cells fulfilled most of fundamental requisites in ISCT minimal criteria for the definition of human MSCs [31], they nevertheless have an altered MSC phenotype. They exhibited reduced adherence to plastic and failed to undergo adipogenesis which ironically was reported to be most robust among the three fundamental MSC differentiation potentials in the human ESC-derived MSCs [24]. Therefore, in contrast to a previous report [29] that observed no fundamental changes in MSC properties after myc transformation, we observed some fundamental changes in myc-transformed cells such that the cells no longer fulfilled the ISCT minimal criteria for the definition of human MSCs [31] and are technically not MSCs. Despite the loss of a defining MSC property, the myc-transformed cells continued to secrete exosomes that could reduce infarct size in a mouse model of ischemia/reperfusion injury. However, we noted that the amount of exosomes secreted by each cell was reduced after transformation but this could be adequately compensated for by the higher proliferative rate or by plating at a higher cell density of cells for the harvest of exosomes. Therefore c-myc transformation represents a practical strategy in ensuring an infinite supply of cells for the production of exosomes either as therapeutic agents or delivery vehicles. In addition, the increased proliferative rate reduces the time for cell production and thereby reduces production costs.

### Example 16. Materials and Methods: Construction and Production of c-mcyc and GFP Lentiviral Vectors

The c-myc cDNA sequence was amplified from pMXs-hc-MYC (15A) using primers containing either XhoI or EcoRI restriction site.

The amplified fragment was then cut with XhoI or EcoRI and cloned into a XhoI-EcoRI restricted lentiviral vector, pLVX-puro (Clontech, www.clontech.com).

The recombined vector was then sequenced to confirm the recombined myc sequence. Lentiviral particles were produced using Lenti-X HT Packaging System (Clontech, www.clontech.com) according to the manufacturer's protocol.

Viral titer was determined using by a Lenti-X^{™} qRT-PCR titration kit (Clontech, www.clontech.com).

### Example 17 (incorporated herein as a reference example). Materials and Methods: Transformation of MSC

HuES9.E1 hESC-MSCs (14A) at passage 22 were infected with lentivirus containing either a c-myc or GFP transgene.

The cells were plated at 10⁶ cells per 10 cm dish. The next day, the cells infected with viruses at a MOI=5 in the presence of 4 µg/ml polybrene overnight.

The culture medium was replaced the next day and selection with puromycin (2 µg/ml) began 48 hours after infection.

Insertion of the c-myc or GFP transgene was confirmed by amplifiying genomic DNA using specific primers for exon2 and 3 respectively: 5'-GCCCCTGGTGCTCCATGAGGAGACACC'-3' and 5'-ACATTCTCCTCGGTGTCCGAGG-3' under PCR conditions were one cycle of 94, 2min; 32 cycles of 94°C, 15s; 60 °C, 30s; 72 °C, 90s and one cycle of 72 °C, 5min.

The PCR products were resolved on a 1% agarose gel.

### Example 18. Materials and Methods: Telomerase Activity

Relative telomerase activity was measured by SYBR® Green real time quantitative telomeric repeat amplification protocol assay using a modified method as described by Wege H. et al (16A).

Briefly, 3 million cells were harvested and cell lysate was prepared using a commercially available mammalian cell extraction kit (Cat K269-500-1, BioVision, www.biovision.com).

The composition of the reagents for the PCR amplification was 1 µg of protein cell lysate, 10 µl of 2 X SYBR Green Super Mix (Cat 170-8880, BioRad, Singapore) with 0.1µg of TS primer (5'-AATCCGTCGAGCAGACTT-3'), 0.1 1 µg of ACX primer (5'-GCGCGG[CTTACC]3CTAACC-3') and 10mM EGTA in a total volume of 25µl.

The reaction was first incubated at 25°C for 20 min to allow the telomerase in the cell lysate to elongate the TS primers followed by 2 min incubation at 95°C to inactivate telomerase activity and denature the primers.

The telomerase product was amplified by PCR for 40 cycles of 95°C, 30s; 60°C, 90s. The relative telomerase activity was assessed against that of HEK cells using the threshold cycle number (or Ct value) for 1 µg protein cell lysate.

### Example 19 (incorporated herein as a reference example). Materials and Methods: Preparation of Conditioned Medium Containing Exosomes

Conditioned medium (CM) was prepared from HuES9.E1 MSC, (c-myc)MSC and (GFP)MSC as described previously (17A).

Briefly, 80% confluent HuES9.E1 cultures were washed with PBS, transferred to a chemically defined, serum-free culture medium for an overnight incubation, washed with PBS and cultured in fresh chemically defined, serum-free culture medium for 3 days.

The CM which contained MSC secretion was collected, clarified by centrifugation, concentrated 50 times using 100-kDa MW cutoff ultrafiltration membranes (Sartorius, www.sartorius.com), and sterilized by filtration through a 0.2µm filter.

### Example 20. Materials and Methods: Isolation of RNA

RNA was isolated from CM by adding three volumes of Trizol LS (Invitrogen, www.invitrogen.com) to one volume of CM and completing the extraction according to the manufacturer's protocol.

Total RNA and small RNAs from MSC were purified using Trizol (Invitrogen, www.invitrogen.com) and *mir*Vana™ miRNA Isolation Kit (Applied Biosystems, www.appliedbiosystems.com.sg), respectively.

RNA was quantitated using Quant-iTTM RiboGreen^{®} RNA Assay Kit (Invitrogen, www.invitrogen.com) and resolved on 1.5% agarose gel containing 8% glyoxal or 15% Novex® TBE-Urea Gels (Invitrogen, www.invitrogen.com).

The separated RNA in the gels was visualized by ethidium bromide (EB) staining. For some experiments, CM was pre-incubated with equal volume of PBS, cell lysis buffer (BioVision, www.biovision.com), 40 mM cyclodextrin (Sigma-Aldrich) or 4,000 U/ml phospholipase A2 (Sigma-Aldrich, www.sigmaaldrich.com) at 37°C for 30 minutes followed by addition of ¹/₁₀^{th} volume of a 1mg/ml RNase A (Roche Applied Science, www.roche-applied-science.com).

These mixtures were then incubated for another 5 minutes at 37°C. The RNAs were extracted with Trizol LS and separated on the TBE-urea gels followed by EB staining.

### Example 21. Materials and Methods: Analysis of RNA by qRT-PCR

Relative myc transcript level in the cells was determined using qRT-PCR. 20ηg RNA from GFP-MSC (p17) and cmyc-MSC (p24, p27, p29) was converted to cDNA using a High-Capacity cDNA Reverse Transcription Kit (ABI, USA).

The cDNA was then amplified by one cycle of 94, 10 min; 40 cycles of 94°C, 15s; 60°C, 60s and one cycle of 95°C, 15s, 60°C 60s, 95°C, 15s with primer sets specific for either myc or actin transcript on the StepOnePlus Real-Time PCR system (Applied Biosystems, www.appliedbiosystems.com.sg).

The myc-specific primer set is 5'-CCCGCCCCTGTCCCCTAGCCG-3' and 5'-AGAAGGGTGTGACCGCAACGTAG3'.

To detect mature miRNA in MSC or CM, 10 ηg small RNA were converted into cDNA and resuspended in 15 µl using TaqMan microRNA Reverse Transcription Kit (Applied Biosystems, www.appliedbiosystems.com.sg) according to manufacturer's protocol.

PCR reaction for each RNA sample was performed in triplicate, with 1 µl cDNA and 10 µl of TaqMan 2X Universal PCR Master Mix (Applied Biosystems, www.appliedbiosystems.com.sg) in 20-µl reaction volume in a StepOne Plus Realtime PCR system (Applied Biosystems, www.appliedbiosystems.com.sg).

The thermal cycling parameters were one cycle of 95°C, 10 minutes followed by 40 cycles of 95°C, 15 seconds and then one cycle of 60°C, 60 seconds.

The primers used were TaqMan primers: hsa-let-7b (Cat AB Assay ID_000378, AppliedBiosystems) and hsa-let-7g (Applied Biosystems, www.appliedbiosystems.com.sg).

For qRT-PCR detection of pre-miRNAs, the primers were designed using Primer Express ® Software Version 3.0 (AppliedBiosystems) as previously described (18A).

For conversion to cDNA, 10 ηg of the RNA sample was denatured with 10 pmol of the lower primer in a 12 µl reaction volume at 80°C for 5 minutes.

The RNA and lower primer were then annealed at 60°C for 5 minutes and then cooled to room temperature.

5X reaction buffer, dNTPs, DTT, RNase inhibitor, and the Thermoscript reverse transcriptase (Invitrogen, www.invitrogen.com) were added as recommended by the manufacturer. The reaction mix was incubated for 45 minutes at 60°C followed by 5 minutes incubation at 85°C.

PCR was then performed using ¹/₁₀^{th} of the reaction mix containing cDNA with a SYBR green PCR Master. Mix (AppliedBiosystems) in 20 µl reaction volume.

The PCR parameters were one cycle of 15 seconds at 95°C and 40 cycles of 1 minute at 60°C. PCR reactions were performed in triplicate.

Primer sequences for hsa-let-7b pre-miRNA (Accession number MI0000063, http://microrna.sanger.ac.uk) were 5'TGAGGTAGTAGGTTGTGTGGT3' and 5' GGAAGGCAGTAGGTTGTATAG3'

For hsa-let-7g pre-miRNA (Accession number MI0000137, http://microrna.sanger.ac.uk) the primer sequences were 5'GTAGTAGTTTGTACAGTTTGAGGGT3' and 5'GGCAGTGGCCTGTACAGT3'.

### Example 22. Materials and Methods: Statistical Analysis

Statistical significance was analyzed using Student's t-test.

### Example 23 (incorporated herein as a reference example). Results: Transformation of MSC by c-myc Transgenesis

hESC-MSCs could be extensively propagated in culture up to passage 25 at a 1:4 split before they start undergoing senescence.

HuES9.E1 hESC-MSCs p22 were transfected with either a c-myc gene or a GFP gene via a lentiviral vector (Figure 6). As expected, c-myc transfected MSCs (myc-MSCs) expressed higher level of c-myc mRNA than GFP-transfected MSCs (GFP-MSCs) (Figure 7).

At p23 or one passage after transfection, GFP-MSC culture exhibited senescence characteristics: larger and more flattened cells with few proliferating or dividing cells. In contrast, myc-MSC culture exhibited oncogenic characteristics: loss of contact inhibition and numerous small proliferating cells.

Instead of growing as an adherent monolayer, myc-MSCs formed aggregates with multi-cellular layers (Figure 8).

Unlike the GFP-MSCs or the parental MSCs, telomerase activity in myc-MSCs was significantly higher (Figure 9).

### Example 24. Results: Ratio of Pre- to Mature microRNA is Altered on Transformation

We have previously reported that relative to the cellular miRNA population, secreted miRNA population had a high ratio of pre- to mature miRNA for at least two miRNAs, hsalet-7b and hsa-let-7g miRNAs.

To determine if transformation affected this ratio for hsa-let-7b and hsa-let-7g miRNAs, secreted RNAs were prepared from culture medium conditioned by myc-MSCs and myc-MSCs, respectively.

Quantitative RT-PCR was performed.

C-myc transformation increased the pre- to mature miRNA ratio for hsa-let-7b and not for hsa-let-7g miRNAs (Figure 10).

The ratio of hsa-let-7b pre- to mature miRNA was increased from 2.5 to 7.1, representing a 2.8 fold increase.

To determine if this change in ratio was due to a change in pre-, mature or both miRNA levels in the secretion or cells, these levels were determined by qRT-PCR.

### Example 25. Discussion

We have previously demonstrated that hESC-MSCs secrete RNA-containing exosomes (Chen et al., in press).

Using culture medium conditioned by these cells, we observed that this CM contained RNA encapsulated in cholesterol-rich phospholipid vesicles and contained mainly small RNAs with no detectable RNAs greater than 500 nt.

The exosomal small RNAs include miRNAs, some ribosomal RNA degradative intermediates and most possibly, other small non-coding RNAs but not the more abundant intact mRNAs or ribosomal RNAs present in cells.

MSC exosomal miRNAs were predominantly in the precursor instead of mature form such that the pre- to mature miRNA ratio for some of the exosomal RNAs such as hsa-let-7b and hsa-let-7g miRNA significantly exceeded that in cellular RNA.

Here, we postulated that the pre- to mature miRNA ratio was likely to be modulated by the physiological or pathological state of the cells.

Here we demonstrated that the pre- to mature miRNA ratio for secreted hsa-let-7b but not hsa-let-7g miRNA was increased upon transformation with c-myc oncogene.

This selective alteration in ratio of pre- to mature miRNA in secreted miRNA can be adapted to extend the current practice of identifying unique circulating microRNA (miRNAs) profiles as predictive and prognostic markers for cancer (13A) (6A).

The use of pre- to mature miRNA ratio to assess secreted or circulating miRNAs will introduce a quantitative component to the present qualitative profiling of circulating miRNAs, minimize variations inherent in biological samples and sample preparations, and generally increase the robustness of biomarkers based on pre- to mature miRNA ratio.

### REFERENCES

1. Le Blanc K, Pittenger M (2005) Mesenchymal stem cells: progress toward promise. Cytotherapy 7: 36-45.
2. Minguell JJ, Erices A (2006) Mesenchymal stem cells and the treatment of cardiac disease. Exp Biol Med (Maywood) 231: 39-49.
3. Schuleri KH, Boyle AJ, Hare JM (2007) Mesenchymal stem cells for cardiac regenerative therapy. Handb Exp Pharmacol: 195-218.
4. Abdel-Latif A, Bolli R, Tleyjeh IM, Montori VM, Perin EC, et al. (2007) Adult bone marrow-derived cells for cardiac repair: a systematic review and meta-analysis. Arch Intern Med 167: 989-997.
5. Mazhari R, Hare JM (2007) Advances in cell-based therapy for structural heart disease. Prog Cardiovasc Dis 49: 387-395.
6. Ohnishi S, Nagaya N (2007) Prepare cells to repair the heart: mesenchymal stem cells for the treatment of heart failure. Am J Nephrol 27: 301-307.
7. Behfar A, Terzic A (2007) Optimizing adult mesenchymal stem cells for heart repair. J Mol Cell Cardiol 42: 283-284.
8. Atsma DE, Fibbe WE, Rabelink TJ (2007) Opportunities and challenges for mesenchymal stem cell-mediated heart repair. Curr Opin Lipidol 18: 645-649.
9. Gnecchi M, He H, Liang OD, Melo LG, Morello F, et al. (2005) Paracrine action accounts for marked protection of ischemic heart by Akt-modified mesenchymal stem cells. Nat Med 11: 367-368.
10. Caplan AI, Dennis JE (2006) Mesenchymal stem cells as trophic mediators. J Cell Biochem 98: 1076-1084.
11. Kinnaird T, Stabile E, Burnett MS, Shou M, Lee CW, et al. (2004) Local delivery of marrow-derived stromal cells augments collateral perfusion through paracrine mechanisms. Circulation 109: 1543-1549.
12. Leedham SJ, Brittan M, McDonald SA, Wright NA (2005) Intestinal stem cells. J Cell Mol Med 9: 11-24.
13. Togel F, Hu Z, Weiss K, Isaac J, Lange C, et al. (2005) Administered mesenchymal stem cells protect against ischemic acute renal failure through differentiation-independent mechanisms. Am J Physiol Renal Physiol 289: F31-42.
14. Patschan D, Plotkin M, Goligorsky MS (2006) Therapeutic use of stem and endothelial progenitor cells in acute renal injury: ca ira. Curr Opin Pharmacol 6: 176-183.
15. Miyahara Y, Nagaya N, Kataoka M, Yanagawa B, Tanaka K, et al. (2006) Monolayered mesenchymal stem cells repair scarred myocardium after myocardial infarction. Nat Med 12: 459-465.
16. Gnecchi M, He H, Noiseux N, Liang OD, Zhang L, et al. (2006) Evidence supporting paracrine hypothesis for Akt-modified mesenchymal stem cell-mediated cardiac protection and functional improvement. Faseb J 20: 661-669.
17. Mayer H, Bertram H, Lindenmaier W, Korff T, Weber H, et al. (2005) Vascular endothelial growth factor (VEGF-A) expression in human mesenchymal stem cells: autocrine and paracrine role on osteoblastic and endothelial differentiation. J Cell Biochem 95: 827-839.
18. Nakagami H, Maeda K, Morishita R, Iguchi S, Nishikawa T, et al. (2005) Novel autologous cell therapy in ischemic limb disease through growth factor secretion by cultured adipose tissue-derived stromal cells. Arterioscler Thromb Vasc Biol 25: 2542-2547.
19. Van Overstraeten-Schlogel N, Beguin Y, Gothot A (2006) Role of stromal-derived factor-1 in the hematopoietic-supporting activity of human mesenchymal stem cells. Eur J Haematol 76: 488-493.
20. Cheng L, Qasba P, Vanguri P, Thiede MA (2000) Human mesenchymal stem cells support megakaryocyte and pro-platelet formation from CD34(+) hematopoietic progenitor cells. J Cell Physiol 184: 58-69.
21. Caplan AI, Dennis JE (2006) Mesenchymal stem cells as trophic mediators. J Cell Biochem.
22. Liu CH, Hwang SM (2005) Cytokine interactions in mesenchymal stem cells from cord blood. Cytokine 32: 270-279.
23. Pittenger MF, Martin BJ (2004) Mesenchymal stem cells and their potential as cardiac therapeutics. Circ Res 95: 9-20.
24. Lian Q, Lye E, Suan Yeo K, Khia Way Tan E, Salto-Tellez M, et al. (2007) Derivation of Clinically Compliant MSCs from CD105+, CD24- Differentiated Human ESCs. Stem Cells 25: 425-436.
25. Lai RC, Arslan F, Tan SS, Tan B, Choo A, et al. Derivation and characterization of human fetal MSCs: An alternative cell source for large-scale production of cardioprotective microparticles. J Mol Cell Cardiol.
26. Lai RC, Arslan F, Lee MM, Sze SK, Choo A, et al. (2010) Exosome secreted by MSC reduces myocardial ischemia/reperfusion injury. Stem Cell Res.
27. Timmers L, Lim S-K, Arslan F, Armstrong JS, Hoefler IE, et al. (2008) Reduction of myocardial infarct size by human mesenchymal stem cell conditioned medium. Stem Cell Research 1: 129-137.
28. Chen TS, Lai RC, Lee MM, Choo AB, Lee CN, et al. (2010) Mesenchymal stem cell secretes microparticles enriched in pre-microRNAs. Nucleic Acids Res 38: 215-224.
29. Sun D, Zhuang X, Xiang X, Liu Y, Zhang S, et al. (2010) A Novel Nanoparticle Drug Delivery System: The Anti-inflammatory Activity of Curcumin Is Enhanced When Encapsulated in Exosomes. Mol Ther 18: 1606-1614.
30. Nagai A, Kim WK, Lee HJ, Jeong HS, Kim KS, et al. (2007) Multilineage Potential of Stable Human Mesenchymal Stem Cell Line Derived from Fetal Marrow. PLoS ONE 2: e1272.
31. Dominici M, Le Blanc K, Mueller I, Slaper-Cortenbach I, Marini F, et al. (2006) Minimal criteria for defining multipotent mesenchymal stromal cells. The International Society for Cellular Therapy position statement. Cytotherapy 8: 315-317.
32. Kitamura T, Koshino Y, Shibata F, Oki T, Nakajima H, et al. (2003) Retrovirus-mediated gene transfer and expression cloning: powerful tools in functional genomics. Exp Hematol 31: 1007-1014.
33. Wege H, Chui MS, Le HT, Tran JM, Zern MA (2003) SYBR Green real-time telomeric repeat amplification protocol for the rapid quantification of telomerase activity. Nucleic Acids Res 31: E3-3.
34. Sze SK, de Kleijn DP, Lai RC, Khia Way Tan E, Zhao H, et al. (2007) Elucidating the secretion proteome of human embryonic stem cell-derived mesenchymal stem cells. Mol Cell Proteomics 6: 1680-1689.
35. Que J, Lian Q, El Oakley RM, Lim B, Lim SK (2007) PI3 K/Akt/mTOR-mediated translational control regulates proliferation and differentiation of lineage-restricted RoSH stem cell lines. J Mol Signal 2: 9.
36. Thomas PD, Campbell MJ, Kejariwal A, Mi H, Karlak B, et al. (2003) PANTHER: a library of protein families and subfamilies indexed by function. Genome Res 13: 2129-2141.
37. Thomas PD, Kejariwal A, Guo N, Mi H, Campbell MJ, et al. (2006) Applications for protein sequence-function evolution data: mRNA/protein expression analysis and coding SNP scoring tools. Nucleic Acids Res 34: W645-650.
38. Lai RC, Arslan F, Lee MM, Sze NS, Choo A, et al. (2010) Exosome secreted by MSC reduces myocardial ischemia/reperfusion injury. Stem Cell Res 4: 214-222.
1A. Pan, B.T. and Johnstone, R.M. (1983) Fate of the transferrin receptor during maturation of sheep reticulocytes in vitro: selective externalization of the receptor. Cell, 33, 967-978.
2A. Simpson, R.J., Lim, J.W., Moritz, R.L. and Mathivanan, S. (2009) Exosomes: proteomic insights and diagnostic potential. Expert Rev Proteomics, 6, 267-283.
3A. Lakkaraju, A. and Rodriguez-Boulan, E. (2008) Itinerant exosomes: emerging roles in cell and tissue polarity. Trends Cell Biol, 18, 199-209.
4A. Zoller, M. (2009) Tetraspanins: push and pull in suppressing and promoting metastasis. Nat Rev Cancer, 9, 40-55.
5A. Cocucci, E., Racchetti, G. and Meldolesi, J. (2009) Shedding microvesicles: artefacts no more. Trends Cell Biol, 19, 43-51.
6A. Rabinowits, G., Gercel-Taylor, C., Day, J.M., Taylor, D.D. and Kloecker, G.H. (2009) Exosomal microRNA: a diagnostic marker for lung cancer. Clin Lung Cancer, 10, 42-46.
7A. Al-Nedawi, K., Meehan, B. and Rak, J. (2009) Microvesicles: messengers and mediators of tumor progression. Cell Cycle, 8, 2014-2018.
8A. Pisitkun, T., Shen, R.F. and Knepper, M.A. (2004) Identification and proteomic profiling of exosomes in human urine. Proc Natl Acad Sci U S A, 101, 13368-13373.
9A. Hoorn, E.J., Pisitkun, T., Zietse, R., Gross, P., Frokiaer, J., Wang, N.S., Gonzales, P.A., Star, R.A. and Knepper, M.A. (2005) Prospects for urinary proteomics: exosomes as a source of urinary biomarkers. Nephrology (Carlton), 10, 283-290.
10A. Adachi, J., Kumar, C., Zhang, Y., Olsen, J.V. and Mann, M. (2006) The human urinary proteome contains more than 1500 proteins, including a large proportion of membrane proteins. Genome Biol, 7, R80.
11A. Pisitkun, T., Johnstone, R. and Knepper, M.A. (2006) Discovery of urinary biomarkers. Mol Cell Proteomics, 5, 1760-1771.
12A. Zhou, H., Pisitkun, T., Aponte, A., Yuen, P.S., Hoffert, J.D., Yasuda, H., Hu, X., Chawla, L., Shen, R.F., Knepper, M.A. et al. (2006) Exosomal Fetuin-A identified by proteomics: a novel urinary biomarker for detecting acute kidney injury. Kidney Int, 70, 1847-1857.
13A. Taylor, D.D. and Gercel-Taylor, C. (2008) MicroRNA signatures of tumor-derived exosomes as diagnostic biomarkers of ovarian cancer. Gynecol Oncol, 110, 13-21.
14A. Lian, Q., Lye, E., Suan Yeo, K., Khia Way Tan, E., Salto-Tellez, M., Liu, T.M., Palanisamy, N., El Oakley, R.M., Lee, E.H., Lim, B. et al. (2007) Derivation of Clinically Compliant MSCs from CD105+, CD24- Differentiated Human ESCs. Stem Cells, 25, 425-436.
15A. Kitamura, T., Koshino, Y., Shibata, F., Oki, T., Nakajima, H., Nosaka, T. and Kumagai, H. (2003) Retrovirus-mediated gene transfer and expression cloning: powerful tools in functional genomics. Exp Hematol, 31, 1007-1014.
16A. Wege, H., Chui, M.S., Le, H.T., Tran, J.M. and Zern, M.A. (2003) SYBR Green real-time telomeric repeat amplification protocol for the rapid quantification of telomerase activity. Nucleic Acids Res, 31, E3-3.
17A. Sze, S.K., de Kleijn, D.P., Lai, R.C., Khia Way Tan, E., Zhao, H., Yeo, K.S., Low, T.Y., Lian, Q., Lee, C.N., Mitchell, W. et al. (2007) Elucidating the secretion proteome of human embryonic stem cell-derived mesenchymal stem cells. Mol Cell Proteomics, 6, 1680-1689.
18A. Schmittgen, T.D., Jiang, J., Liu, Q. and Yang, L. (2004) A high-throughput method to monitor the expression of microRNA precursors. Nucleic Acids Res, 32, e43.

## Claims

1. A method of monitoring the transformation state of a mesenchymal stem cell (MSC), the method comprising establishing, for hsa-let-7b microRNA secreted by the mesenchymal stem cell, a ratio of: (a) a precursor form of hsalet-7b microRNA (pre-miRNA); to (b) a mature form of hsa-let-7b microRNA (mature miRNA); in which the pre- to mature miRNA ratio so established is indicative of the transformation state of the mesenchymal stem cell.

2. A method according to Claim 1, in which the hsa-let-7b microRNA is comprised in exosomes secreted by the mesenchymal stem cell.

3. A method according to Claim 2, in which and the method comprises obtaining such exosomes and obtaining precursor and mature forms of the hsa-let-7b microRNA therefrom.

4. A method according to Claim 1, 2 or 3, which comprises establishing a profile comprising a plurality of pre- to mature miRNA ratios for a plurality of selected miRNA species, each indicative of the state of the cell.

5. A method according to any preceding claim, in which the pre- to mature miRNA ratio is established by hybridisation to an array comprising nucleic acid sequences capable of binding to and distinguishing between precursor and mature forms of the miRNA species.

6. A method according to any of Claims 1 to 4, in which the pre- to mature miRNA ratio is established by real time polymerase chain reaction (RT-PCR).

7. A method according to any preceding claim, in which the pre- to mature miRNA ratio is 2.8 higher in a transformed state compared to a normal state.

8. A method according to any preceding claim, in which the mesenchymal stem cell is in an untransformed state or a c-myc transformed state.

9. An array comprising a plurality of probes capable of binding to and distinguishing between precursor and mature forms of a plurality of selected miRNA species, in which the plurality of selected miRNA species includes hsa-let-7b microRNA.

10. A method comprising the steps of:
(a) providing a mesenchymal stem cell (MSC);
(b) introducing an oncogene into the mesenchymal stem cell to thereby transform it; and
(c) establishing a pre- to mature miRNA ratio of hsa-let-7b microRNA secreted by the mesenchymal stem cell by a method according to any preceding claim so as to detect such transformation;
in which the transformed mesenchymal stem cell does not secrete a gene product of the oncogene into a medium in which it is grown.

11. A method according to Claim 10, in which: (a) the oncogene comprises c-myc; (b) the mesenchymal stem cell (MSC) comprises an established cell line; or (c) the mesenchymal stem cell (MSC) is derived from umbilical cord.

12. A method according to Claim 10 or 11, in which transformed mesenchymal stem cell: (a) is capable of bypassing senescence; (b) has an increased proliferation rate; (c) has a decreased population doubling time preferably 24 hours; or (d) has an increased telomerase activity; as compared to a mesenchymal stem cell that has not been transformed.

13. A method of providing a conditioned medium, the method comprising the steps set out in Claim 10, 11 or 12, followed by culturing the transformed mesenchymal stem cell, or a descendent thereof, in a cell culture medium to condition it, and separating the conditioned from the transformed mesenchymal stem cell.

14. A method of providing an exosome, the method comprising: (a) obtaining a mesenchymal stem cell conditioned medium (MSC-CM) by a method according to Claim 13; (b) concentrating the mesenchymal stem cell conditioned medium, preferably by ultrafiltration over a >1000 kDa membrane; (c) subjecting the concentrated mesenchymal stem cell conditioned medium to size exclusion chromatography, preferably using a a TSK Guard column SWXL, 6 x 40 mm or a TSK gel G4000 SWXL, 7.8 x 300 mm column; and (d) selecting UV absorbant fractions, preferably, at 220 nm, that exhibit dynamic light scattering, preferably detected by a quasi-elastic light scattering (QELS); in which step (d) preferably comprises collecting fractions which elute with a retention time of 11-13 minutes, preferably 12 minutes.

15. A method of preparing a pharmaceutical composition, the method comprising obtaining a mesenchymal stem cell conditioned medium (MSC-CM) by a method according to Claim 13 or an exosome by a method according to Claim 14 and admixing the MSC-CM or exosome, as the case may be, with a pharmaceutically acceptable carrier or diluent.

16. A method according to any of claims 1-8 and 10-15, in which transformed mesenchymal stem cell, the conditioned medium, exosome or pharmaceutical composition comprises at least one biological property of a mesenchymal stem cell, for example cardioprotection, or in which the conditioned medium, exosome or pharmaceutical composition is capable of reducing infarct size for example as assayed in a mouse or pig model of myocardial ischemia and reperfusion injury, or which is capable of reducing oxidative stress for example as assayed in an *in vitro* assay of hydrogen peroxide (H₂O₂)-induced cell death.

## Patentansprüche

1. Verfahren zur Überwachung des Transformationszustands einer mesenchymalen Stammzelle (MSC), umfassend, für hsa-let-7b-microRNA, die von der mesenchymalen Stammzelle sezerniert wird, das Bestimmen eines Verhältnisses von (a) einer Vorläuferform der hsa-let-7b-microRNA (pre-miRNA); zu (b) einer reifen Form der hsa-let-7b-microRNA (reife miRNA); wobei das derart bestimmte Verhältnis von pre-miRNA zu reifer miRNA indikativ für den Transformationszustand der mesenchymalen Stammzelle ist.

2. Verfahren nach Anspruch 1, wobei die hsa-let-7b-microRNA in Exosomen umfasst ist, die von der mesenchymalen Stammzelle sezerniert werden.

3. Verfahren nach Anspruch 2, wobei das Verfahren den Erhalt derartiger Exosome sowie den Erhalt von Vorläuferformen und reifen Formen der hsa-let-7b-microRNA aus den Exosomen umfasst.

4. Verfahren nach Anspruch 1, 2 oder 3, umfassend das Erstellen eines Profils, das eine Vielzahl an Verhältnissen von pre-miRNA zu reifer miRNA für eine Vielzahl an ausgewählten miRNA-Spezies umfasst, wobei jedes Verhältnis indikativ für den Zustand der Zelle ist.

5. Verfahren nach einem der vorhergehenden Ansprüche, wobei das Verhältnis von pre-miRNA zu reifer miRNA mittels der Hybridisierung an einen Array bestimmt wird, der Nukleinsäuresequenzen umfasst, die zu einer Bindung an Vorläuferformen und reife Formen sowie zu einer Unterscheidung zwischen Vorläuferformen und reifen Formen der microRNA-Spezies in der Lage sind.

6. Verfahren nach einem der Ansprüche 1 bis 4, wobei das Bestimmen des Verhältnisses von pre-miRNA zu reifer miRNA mittels einer Echtzeit-Polymerasekettenreaktion (RT-PCR) erfolgt.

7. Verfahren nach einem der vorhergehenden Ansprüche, wobei das Verhältnis von pre-miRNA zu reifer miRNA in einem transformierten Zustand um 2,8 höher ist als in einem normalen Zustand.

8. Verfahren nach einem der vorhergehenden Ansprüche, wobei die mesenchymale Stammzelle in einem untransformierten Zustand oder in einem durch c-myc transformierten Zustand vorliegt.

9. Array, der eine Vielzahl an Sonden umfasst, die zu einer Bindung an Vorläuferformen und reife Formen sowie zu einer Unterscheidung zwischen Vorläuferformen und reifen Formen einer Vielzahl an ausgewählten microRNA-Spezies in der Lage sind, wobei die Vielzahl an ausgewählten miRNA-Spezies hsa-let-7b-microRNA einschließt.

10. Verfahren, umfassend die folgenden Schritte:
(a) das Bereitstellen einer mesenchymalen Stammzelle (MSC);
(b) das Einbringen eines Onkogens in die mesenchymale Stammzelle, um diese dadurch zu transformieren; und
(c) das Bestimmen eines Verhältnisses von pre-miRNA zu reifer miRNA für durch die mesenchymale Stammzelle sezernierte hsa-let-7b-microRNA mittels eines Verfahrens nach einem der vorhergehenden Ansprüche, um eine derartige Transformation zu detektieren;
wobei die transformierte mesenchymale Stammzelle kein Genprodukt des Onkogens in ein Medium sezerniert, in dem sie kultiviert wird.

11. Verfahren nach Anspruch 10, wobei (a) das Onkogen c-myc umfasst; (b) die mesenchymale Stammzelle (MSC) eine etablierte Zelllinie umfasst; oder (c) die mesenchymale Stammzelle (MSC) aus der Nabelschnur abgeleitet ist.

12. Verfahren nach Anspruch 10 oder 11, wobei die transformierte mesenchymale Stammzelle im Vergleich zu einer mesenchymalen Stammzelle, die nicht transformiert worden ist, (a) zur Umgehung der Seneszenz in der Lage ist; (b) eine erhöhte Proliferationsrate aufweist; (c) eine reduzierte Populationsverdopplungszeit aufweist, bevorzugt 24 Stunden; oder (d) eine erhöhte Telomeraseaktivität aufweist.

13. Verfahren zum Bereitstellen eines konditionierten Mediums, umfassend die in Anspruch 10, 11 oder 12 genannten Schritte, gefolgt von dem Kultivieren der transformierten mesenchymalen Stammzelle oder eines Nachkommens von dieser in einem Zellkultur-medium, um die Zelle oder den Nachkommen der Zelle zu konditionieren, sowie dem Trennen der konditionierten von der transformierten mesenchymalen Stammzelle.

14. Verfahren zum Bereitstellen eines Exosoms, umfassend (a) den Erhalt eines durch mesenchymale Stammzellen konditionierten Mediums (MSC-CM) mittels eines Verfahrens nach Anspruch 13; (b) das Aufkonzentrieren des durch mesenchymale Stammzellen konditionierten Mediums, bevorzugt mittels Ultrafiltration durch eine Membran mit > 1.000 kDa; (c) das Durchführen einer Größenausschlusschromatographie an dem aufkonzentrierten, durch mesenchymale Stammzellen konditionierten Medium, bevorzugt unter Verwendung einer Säule TSK Guard Column SWXL mit 6 × 40 mm oder einer Säule TSK Gel G4000 SWXL mit 7,8 × 300 mm; und (d) das Auswählen von UV-absorbierenden Fraktionen, bevorzugt bei 220 nm, die eine dynamische Lichtstreuung aufweisen, die bevorzugt mittels QELS (*quasi-elastic light scattering*) detektiert wird; wobei Schritt (d) bevorzugt das Sammeln der Fraktionen umfasst, die mit einer Retentionszeit von 11 bis 13 Minuten, bevorzugt von 12 Minuten, eluieren.

15. Verfahren zur Herstellung einer pharmazeutischen Zusammensetzung, umfassend den Erhalt eines durch mesenchymale Stammzellen konditionierten Mediums (MSC-CM) mittels eines Verfahrens nach Anspruch 13 oder den Erhalt eines Exosoms mittels eines Verfahrens nach Anspruch 14 sowie das Mischen des MSC-CM bzw. des Exosoms mit einem pharmazeutisch verträglichen Träger oder Diluenten.

16. Verfahren nach einem der Ansprüche 1 bis 8 und 10 bis 15, wobei die transformierte mesenchymale Stammzelle, das konditionierte Medium, das Exosom oder die pharmazeutische Zusammensetzung mindestens eine biologische Eigenschaft einer mesenchymalen Stammzelle umfasst, wie z. B. eine kardioprotektive Eigenschaft, oder wobei das konditionierte Medium, das Exosom oder die pharmazeutische Zusammensetzung zur Reduktion des Ausmaßes eines Infarkts, wie z. B. untersucht in einem murinen oder porcinen Modell der Myokardischämie und der Reperfusionsverletzung, oder zur Reduktion von oxidativem Stress, wie z. B. untersucht in einem *in vitro* Assay des durch Wasserstoffperoxid (H₂O₂) induzierten Zelltods, in der Lage ist.

## Revendications

1. Procédé de surveillance de l'état de transformation d'une cellule souche mésenchymateuse (MSC), le procédé comprenant l'établissement, pour un microARN hsa-let-7b sécrété par la cellule souche mésenchymateuse, d'un rapport de : (a) une forme précurseur de microARN hsalet-7b (pré-ARNmi) ; à (b) une forme mature de microARN hsa-let-7b (ARNmi mature) ; dans lequel le rapport pré-ARNmi à ARNmi mature ainsi établi est indicatif de l'état de transformation de la cellule souche mésenchymateuse.

2. Procédé selon la revendication 1, dans lequel le microARN hsa-let-7b est compris dans des exosomes sécrétés par la cellule souche mésenchymateuse.

3. Procédé selon la revendication 2, dans lequel le procédé comprend l'obtention de tels exosomes et l'obtention de formes précurseur et mature du microARN hsalet-7b à partir de ceux-ci.

4. Procédé selon l'une des revendications 1, 2 ou 3, qui comprend l'établissement d'un profil comprenant une pluralité de rapports pré-ARNmi à ARNmi mature pour une pluralité d'espèces d'ARNmi choisies, chacune indicative de l'état de la cellule.

5. Procédé selon l'une quelconque des revendications précédentes, dans lequel le rapport pré-ARNmi à ARNmi mature est établi par hybridation à un réseau comprenant des séquences d'acide nucléique capables de se lier aux, et de distiguer entre, les formes précurseur et mature des espèces d'ARNmi.

6. Procédé selon l'une quelconque des revendications 1 à 4, dans lequel le rapport pré-ARNmi à ARNmi mature est établi par réaction en chaîne de la polymérase en temps réel (RT-PCR).

7. Procédé selon l'une quelconque des revendications précédentes, dans lequel le rapport pré-ARNmi à ARNmi mature est 2,8 fois plus élevé dans un état transformé par comparaison avec un état normal.

8. Procédé selon l'une quelconque des revendications précédentes, dans lequel la cellule souche mésenchymateuse est dans un état non transformé ou un état transformé par c-myc.

9. Réseau comprenant une pluralité de sondes capables de se lier aux, et de distinguer entre, des formes précurseur et mature d'une pluralité d'espèces d'ARNmi choisies, dans lequel la pluralité d'espèces d'ARNmi choisies comprend le microARN hsa-let-7b.

10. Procédé comprenant les étapes consistant à :
(a) se procurer une cellule souche mésenchymateuse (MSC) ;
(b) introduire un oncogène dans la cellule souche mésenchymateuse pour la transformer de cette façon ; et
(c) établir un rapport pré-ARNmi à ARNmi mature de microARN hsa-let-7b sécrété par la cellule souche mésenchymateuse par un procédé selon l'une quelconque des revendications précédentes de façon à détecter une telle transformation ;
dans lequel la cellule souche mésenchymateuse transformée ne sécrète pas de produit génique de l'oncogène dans un milieu dans lequel elle est en croissance.

11. Procédé selon la revendication 10, dans lequel : (a) l'oncogène comprend c-myc ; (b) la cellule souche mésenchymateuse (MSC) comprend une lignée cellulaire établie ; ou (c) la cellule souche mésenchymateuse (MSC) est issue de cordon ombilical.

12. Procédé selon l'une des revendications 10 ou 11, dans lequel la cellule souche mésenchymateuse transformée : (a) est capable de contourner la sénescence ; (b) a une vitesse de prolifération accrue ; (c) a un temps de doublement de la population diminué de préférence de 24 heures ; ou (d) a une activité télomérase accrue ; par comparaison avec une cellule souche mésenchymateuse qui n'a pas été transformée.

13. Procédé de fourniture d'un milieu conditionné, le procédé comprenant les étapes indiquées dans l'une des revendications 10, 11 ou 12, en faisant suivre par une culture de la cellule souche mésenchymateuse transformée, ou d'une descendante de celle-ci, dans un milieu de culture cellulaire pour la conditionner, et une séparation de la cellule souche mésenchymateuse conditionnée de la cellule souche mésenchymateuse transformée.

14. Procédé de fourniture d'un exosome, le procédé comprenant : (a) obtenir un milieu conditionné de cellules souches mésenchymateuses (MSC-CM) par un procédé selon la revendication 13 ; (b) concentrer le milieu conditionné provenant de cellules souches mésenchymateuses, de préférence par ultrafiltration sur une membrane de > 1000 kDa ; (c) soumettre le milieu conditionné de cellules souches mésenchymateuses concentré à une chromatographie d'exclusion stérique, de préférence à l'aide d'une colonne TSK Guard SWXL, 6 x 40 mm, ou d'une colonne TSK gel G4000 SWXL, 7,8 x 300 mm ; et (d) choisir des fractions absorbant les UV, de préférence, à 220 nm, qui présentent une diffusion dynamique de la lumière, de préférence détectée par diffusion quasi-élastique de la lumière (QELS) ; dans lequel l'étape (d) comprend de préférence la collecte de fractions qui s'éluent avec un temps de rétention de 11-13 minutes, de préférence de 12 minutes.

15. Procédé de préparation d'une composition pharmaceutique, le procédé comprenant l'obtention d'un milieu conditionné de cellules souches mésenchymateuses (MSC-CM) par un procédé selon la revendication 13 ou d'un exosome par un procédé selon la revendication 14 et le mélange du MSC-CM ou de l'exosome, selon le cas, avec un support ou diluant pharmaceutiquement acceptable.

16. Procédé selon l'une quelconque des revendications 1-8 et 10-15, dans lequel la cellule souche mésenchymateuse transformée, le milieu conditionné, l'exosome ou la composition pharmaceutique comprend au moins une propriété biologique d'une cellule souche mésenchymateuse, par exemple la cardioprotection, ou dans lequel le milieu conditionné, l'exosome ou la composition pharmaceutique est capable de réduire la taille d'un infarctus par exemple tel qu'évalué dans un modèle de souris ou de cochon d'ischémie myocardique et de lésion de reperfusion, ou qui est capable de réduire le stress oxydant par exemple tel qu'évalué dans un essai *in vitro* de mort cellulaire induite par le peroxyde d'hydrogène (H₂O₂).
